# EUROPEAN PATENT APPLICATION

(11) **EP 2 716 686 A1**
(43) Date of publication of application: **09.04.2014**
(21) Application number: 12793193.9
(22) Date of filing: 22.05.2012
(51) Int. Cl.: C08G 77/48, A61K 8/892, A61K 47/34, A61Q 1/00, A61Q 1/12, A61Q 5/00, A61Q 15/00, A61Q 17/04, A61Q 19/00, C08K 5/00, C08L 83/14

(54) **NOVEL ORGANOPOLYSILOXANE ELASTOMER AND USE THEREFOR**

(30) Priority: 30.05.2011 JP 2011121097
(71) Applicant: Dow Corning Toray Co., Ltd., Tokyo 100-0004 (JP)
(72) Inventor: TAMURA Seiki, Ichihara-shi Chiba 299-0108 (JP); SOUDA Tatsuo, Ichihara-shi Chiba 299-0108 (JP); HASEGAWA Chiichiro, Ichihara-shi Chiba 299-0108 (JP); KOJIMA Kazuhiko, Ichihara-shi Chiba 299-0108 (JP); HORI Seiji, Ichihara-shi Chiba 299-0108 (JP)
(74) Representative: Ede, Eric
(86) International application number: PCT/JP2012/063089
(87) International publication number: WO 2012/165235

(57) **Abstract**

The present invention relates to an organopolysiloxane elastomer that: has a sugar alcohol-modified group bonded to a silicon atom, and has a three-dimensional cross-linked network structure comprising carbon-silicon bonds in the cross-linking portions. This organopolysiloxane elastomer exhibits, for example, compatibility with various oils, superior thickening properties, gelling properties, and emulsification properties, as well as imparting an excellent sensation, in particular, a thick velvety smoothness and being pleasant to use.

## Description

### TECHNICAL FIELD

This application claims priority rights based on Japanese Patent Application No. 2011-121097 filed May 30, 2011 in Japan, the content of which is incorporated herein by reference. The present invention relates to a novel organopolysiloxane elastomer having a crosslinked structure and uses thereof. The applications and background art of the novel organopolysiloxane elastomer of the present invention are common to the corresponding disclosed content of another patent application claiming priority rights based on Japanese Patent Application No. 2011-121097 filed on the same day as this application (related to a modified organopolysiloxane elastomer having a siloxane dendron structure and a mono-diglycerin-modified organopolysiloxane elastomer).

### BACKGROUND ART

As an organopolysiloxane elastomer having a hydrophilic group, a silicone polymer which is swellable with respect to silicone oil and a paste-like silicone composition which is produced using the silicone polymer and can stably and uniformly disperse water have been reported (Patent Documents 1 and 2). This is a material provided with enhanced adaptability with respect to formulations containing water in addition to stable viscosity-improving characteristics for silicone oils by introducing a polyoxyalkylene group into the gel skeleton of a silicone gel composition (Patent Document 3) or powder, paste-like, or grease-like silicone composition (Patent Document 4) reported prior to these patents.

However, although the compositions proposed in Patent Documents 1 and 2 have excellent characteristics for emulsifying/dispersing water in silicone oils, the compositions cannot stably emulsify/disperse water in a system containing non-silicone oils such as a hydrocarbon oil or an ester oil, leading to the problem that the viscosity-improving effects are also limited. Further, there is the drawback that when the resulting emulsion is stored for a long period of time, the pH decreases and the emulsion develops an abnormal odor.

On the other hand, a polyoxyalkylene group-containing organopolysiloxane elastomer having a structure differing from that of the materials described above and a silicone paste containing this elastomer and a solvent have also been proposed (Patent Documents 5 and 6). These technologies are characterized in that decamethylcyclopentasiloxane, which also has excellent compatibility with non-silicone oil agents as a solvent, is used, and the effect of this substance enables the stable emulsification/dispersion of water in silicone/non-silicone mixed oils.

However, when this organopolysiloxane elastomer uses a chain silicone oil or non-silicone oil other than a cyclic dimethylpolysiloxane such as decamethylcyclopentasiloxane as a solvent, there is a problem in that it becomes difficult to stably emulsify/disperse water in a silicone/non-silicone mixed oil or a non-silicone oil.

Incidentally, known technologies which aim to demonstrate stable emulsification performance with a formulation containing organic oils by improving the compatibility of the organopolysiloxane elastomer itself with non-silicone oils include a technology using a composition containing a blend of a crosslinked elastomeric silicone polyether and a crosslinked elastomeric silicone containing an alkyl group having from 3 to 40 carbon atoms (Patent Document 7) and a technology using an elastomer silicone terpolymer composition containing a polyoxyalkylene group and a higher alkyl group having at least 10 carbon atoms (Patent Document 8).

Although these technologies make it possible to stably emulsify/disperse water in not only silicone oils but also oil phases containing large amounts of organic oils, a large amount of the elastomer is required in order to achieve stable emulsification with the technology of Patent Document 7, which is problematic in that it is economically disadvantageous. In addition, with the technology of Patent Document 8, the feel of the emulsion when applied is heavy, which leads to the problem that the emulsion tends to have an oily feel or a texture with strong stickiness.

In addition, a technology which uses a silicone substance containing a polymer network structure formed by the polymerization of a polyfunctional organosilicone compound containing an alkenyl group and a hydride group in the same molecule (Patent Document 9), a technology which uses a polyether siloxane elastomer compatible with polar solvents (Patent Document 10), a technology which uses a crosslinked silicone polymer mesh-like composition expanded by alkyltrisiloxane (Patent Document 11), and the like have also been reported.

However, with the technology of Patent Document 9, even if an attempt is made to introduce allyl polyether into the molecule, a self-polymerization reaction of the polyfunctional organosilicone occurs preferentially due to the problem of compatibility and the difference between the reactivities of the C=C bonding sites, which causes the substance to gelify quickly. Therefore, the polyether group serving as a hydrophilic portion cannot be introduced evenly, which leads to the problem that fluctuations in emulsification performance tend to occur. The technologies of Patent Documents 10 and 11 have problems in that the stability of W/O emulsions containing organic oils is insufficient. In recent years, the use of a silicone polymer ether elastomer gel which demonstrates improved compatibility with various organic components and a stable viscosity-improving effect as a result of the introduction of a polyoxypropylene group has also been proposed (Patent Documents 12 and 13), but the function of this material as a W/O emulsifier is small.

Further, several technologies related to emulsions or emulsion-type cosmetic compositions using the materials described above have also been reported (Patent Documents 14 to 17), but the respective materials serving as a foundation have the problems described above, so the degree of completion when used as a cosmetic composition is low. In particular, as a problem common to the technologies described above, the issue that the resulting emulsion develops a strong abnormal odor when the emulsion is stored for a long period of time remains unresolved.

On the other hand, a great deal of research into reducing the odor of non-elastomer type polyether-modified polysiloxanes (polyoxyalkylene group-containing organopolysiloxanes) has been conducted. The cause of odorization over time of a polyether-modified polysiloxane that was first reported was the aldehyde and acid produced as a result of oxidation degradation (rancidity) over time of the polyether moiety in the polyether-modified polysiloxane composition. Examples of technologies to suppress this oxidation degradation include the methods recited in Patent Documents 18 and 19 in which tocopherol, phytic acid, or a similar antioxidant component is added to the polyether-modified polysiloxane composition.

However, the use of only these anti-oxidizing agent results in the insufficient suppression of the odorization over time of a formulation based on the polyether-modified polysiloxane and, as a result, other causes were investigated. As a result, Patent Document 20 recites that propionaldehyde originating from unreacted propenyl-etherified polyoxyalkylene is a cause of the odor.

The polyether-modified polysiloxane composition is typically synthesized via a hydrosilylation reaction of an organohydrogenpolysiloxane having a silicon-bonded hydrogen group and a polyoxyalkylene having an allyl ether group at a terminal. Patent Document 20 recites that, in the production of the polyether-modified polysiloxane composition, a double bond of the allyl etherified polyoxyalkylene migrates inward due to the influence of a platinum catalyst and a portion of the allyl-etherified polyoxyalkylene becomes a propenyl-etherified polyoxyalkylene and remains in the polyether-modified polysiloxane composition as is without reacting with the organohydrogenpolysiloxane. Patent Document 12 also recites that the propenyl-etherified polyoxyalkylene degrades over time, thus producing ketones and aldehydes which results in the odorization. Moreover, hydrolysis in the presence of an acid is disclosed as a useful deodorization method.

However, while this deodorization method could be thought to be useful if all of the allyl groups of the polyoxyalkylene remaining in the composition were replaced with propenyl groups, in actuality, a significant proportion of the allyl-etherified polyoxyalkylene which is not easily hydrolyzed remains. As a result, the composition cannot be sufficiently deodorized using the deodorization method of Patent Document 20. If a strong acid is used that can hydrolyze the allyl-etherified polyoxyalkylene, the carbon-oxygen bond at the polyoxyalkylene site and/or the silicon-oxygen bond at the polysiloxane site may disconnect, so using such an acid is inappropriate. Additionally, in order to perform the hydrolysis reaction in a quantitative manner, excessive amounts of water and acid are needed. These excessive amounts of water and acid complicate post treatment processes and, therefore, this deodorization method is not preferable.

In order to resolve this problem, methods for suppressing the production of propionaldehyde have been disclosed (Patent Documents 21 to 24). In these methods, a hydrogenation treatment is performed as a deodorization method of the polyether-modified polysiloxane composition in order to alkylate the alkenyl groups (double bonds) included in the alkenyl group-containing polyoxyalkylene (including both propenyl-etherified polyoxyalkylene and allyl-etherified polyoxyalkylene) remaining in the composition. However, even with a polyether-modified polysiloxane composition deodorized using a hydrogenation reaction, in cases where a formulation including water and an alcohol is compounded, it may be difficult to achieve sufficient deodorization over time or under elevated temperature conditions.

A cause of the odorization is acetal and similar aldehyde condensation products that are free of unsaturated bonds that remain in the composition. Thus, for the purpose of completely eliminating the acetal and other aldehyde condensation products, technology in which treatment using the acid aqueous solution and hydrogenation treatment are combined (Patent Document 25) and technologies in which hydrogenation treatment and treatment using a solid acid catalyst are combined (Patent Documents 26 and 27) are disclosed. That is, it is acknowledged that performing at least hydrogenation treatment is preferable in the deodorization of polyether-modified polysiloxanes as a raw material suitable for use in cosmetic products.

Based on such a background, two technologies for reducing the odor of an organopolysiloxane elastomer having a hydrophilic group such as an elastomer-type polyether-modified polysiloxane have been reported. Patent Document 28 introduces a paste-like composition which is obtained by adding at least one type of acidic substance selected from the group consisting of organic acids, phosphoric acids, and phosphoric acid salts to a mixture consisting of a crosslinked organopolysiloxane polymer having a polyoxyalkylene chain and a liquid oil, adding a basic neutralizing salt so that the pH is from 5 to 8, and then removing volatile components by heating and/or decompression. The problems arising when applying a refining method using a non-elastomer-type polyether-modified polysiloxane to a crosslinked polymer are as described below. Specifically, there is a drawback in that unless a device with a glass lining is used when treating the substance with a corrosive acidic aqueous solution such as hydrochloric acid water, the device will be corroded. In addition, even if an effect is recognized with regard to the reduction of odor, it is not possible to suppress decreases in pH. Further, when performing a hydrogenation treatment method, heavy metal catalysts such as palladium or nickel become necessary, but it is not possible to remove these catalysts by filtration, and the heavy metal catalysts are left behind in the composition, which is not suitable for applications to cosmetic compositions.

In addition, Patent Document 29 introduces a composition obtained by adding at least one type of acidic substance selected from the group consisting of organic acids, inorganic acids, and inorganic acid salts to an organopolysiloxane polymer having a glycerin derivative and containing a liquid oil in an amount equal to or greater than its own weight so that the polymer is capable of swelling, and a paste-like composition prepared by adding a liquid oil agent to this polymer so that the polymer swells, adding a basic neutralizing salt so that the pH is from 5 to 8, and then removing volatile components by heating and/or decompression. The problems arising when treating the polyether-modified polysiloxane crosslinked polymer with hydrochloric acid water or the like are described in detail as follows. Specifically, with this method, it is not possible to suppress decreases in pH caused by the automatic oxidation characteristic to polyoxyethylene chains, and it is not possible to suppress decreases in viscosity or the generation of an acidified odor due to the decomposition of the polyoxyethylene chain. Although the problems described above can be suppressed by adding an antioxidant, the effect of doing so is insufficient.

This organopolysiloxane elastomer containing a glycerin derivative is assumed to have good solubility and emulsifiability in various oil agents in comparison to conventional polyoxyalkylene group-containing organopolysiloxane elastomers. Further, it has been reported that when comparing the sensation during use of W/O type creams using these substances as emulsifiers, elastomers having glycerin derivatives demonstrate better moisture and retention. However, this material has poor compatibility with oil agents other than silicone oils such as hydrocarbon oils or ester oils, for example, and there is a problem in that the viscosity-improving effect on these organic oils is insufficient. Further, there are cases in which the effect is also insufficient from the perspective of the capacity to stably emulsify/disperse water in an oil agent system containing large amounts of organic oils.

Therefore, an organopolysiloxane elastomer characterized by a branched structure based on a polydimethylsiloxyethyl group and further having a hydrophilic group has been proposed by Patent Document 30 as a material for solving these problems, and this elastomer demonstrates a high viscosity-improving effect on silicone oils and organic oils other than silicone oils and provides a paste free of stickiness. However, this paste demonstrates oily gooeyness and an unnatural warm sensation, so the paste is not necessarily satisfactory from the perspective of the tactile sensation. Further, in order to demonstrate an effect as an oil viscosity-improving agent, it is necessary to add at least 10 wt.% (mass%) of an oil phase as the elastomer content, so the paste can also be perceived as having a problem from the perspective of the viscosity-improving efficiency.

On the other hand, non-elastomer sugar alcohol-modified polysiloxanes and applications thereof to cosmetics and the like have been reported in Patent Documents 31 to 38. However, these documents are not related to organopolysiloxane elastomers having sugar alcohol-modified groups.

### BACKGROUND DOCUMENTS

### Patent Documents

Patent Document 1: Japanese Unexamined Patent Application Publication No. H04-272932A
Patent Document 2: Japanese Unexamined Patent Application Publication No. H05-104320A
Patent Document 3: Japanese Unexamined Patent Application Publication No. H01-207354A
Patent Document 4: Japanese Unexamined Patent Application Publication No. H02-043263A
Patent Document 5: US Patent No. 5811487B
Patent Document 6: Japanese Unexamined Patent Application Publication No. H11-049957A
Patent Document 7: Japanese Unexamined Patent Application Publication No. 2001-187842A
Patent Document 8: Japanese Unexamined Patent Application Publication No. 2000-319515A
Patent Document 9: Japanese Unexamined Patent Application Publication No. 2002-105318A
Patent Document 10: WO2001/014458
Patent Document 11: WO2007/061623
Patent Document 12: WO2007/109240
Patent Document 13: WO2009/006091
Patent Document 14: Japanese Unexamined Patent Application Publication No. H06-040847A
Patent Document 15: Japanese Unexamined Patent Application Publication No. H06-040848A
Patent Document 16: Japanese Unexamined Patent Application Publication No. 2001-064513A
Patent Document 17: Japanese Unexamined Patent Application Publication No. 2001-192459A
Patent Document 18: Japanese Examined Patent Application Publication No. S55-041210A
Patent Document 19: Japanese Unexamined Patent Application Publication No. S60-018525A
Patent Document 20: Japanese Unexamined Patent Application Publication No. H02-302438A
Patent Document 21: US Patent No. 5225509B
Patent Document 22: Japanese Unexamined Patent Application Publication No. H07-330907A
Patent Document 23: Japanese Unexamined Patent Application Publication No. H09-165315A
Patent Document 24: Japanese Unexamined Patent Application Publication No. H09-165318A
Patent Document 25: WO2002/055588
Patent Document 26: WO2004/046226
Patent Document 27: Japanese Unexamined Patent Application Publication No. 2005-120293A
Patent Document 28: WO2003/020828
Patent Document 29: WO2004/024798
Patent Document 30: Japanese Unexamined Patent Application Publication No. 2008-115358A
Patent Document 31: Japanese Unexamined Patent Application Publication No. S62-068820A
Patent Document 32: Japanese Unexamined Patent Application Publication No. S63-139106A
Patent Document 33: Japanese Unexamined Patent Application Publication No. H05-186596A
Patent Document 34: Japanese Unexamined Patent Application Publication No. H07-041417A
Patent Document 35: Japanese Unexamined Patent Application Publication No. 2002-119840A
Patent Document 36: Japanese Unexamined Patent Application Publication No. 2008-274241A
Patent Document 37: Japanese Unexamined Patent Application Publication No. 2002-179798A
Patent Document 38: Japanese Unexamined Patent Application Publication No. 2003-146991A

### SUMMARY OF INVENTION

### Technical Problem

The present invention was conceived in order to solve the problems described above, and a first object of the present invention is to provide a novel organomodified organopolysiloxane elastomer and a production method thereof, wherein the novel organomodified organopolysiloxane elastomer has affinity with various oil agents, has excellent viscosity-improving characteristics, gelification characteristics, and emulsification characteristics, imparts excellent tactile sensation - in particular, an excellent tactile sensation characterized by smoothness with a velvet-like thickness - and imparts an excellent sensation during use.

A second object of the present invention is to provide a raw material for an external use preparation or a cosmetic composition such as a thickening agent, a gelling agent, a tactile sensation improver, a surfactant, an emulsifier, or a powder dispersion stabilizer containing the organomodified organopolysiloxane elastomer, and a cosmetic composition or an external use preparation containing the organomodified organopolysiloxane elastomer.

A third object of the present invention is to provide an organomodified organopolysiloxane elastomer with a reduced odor, a raw material for an external use preparation or a cosmetic composition containing the same, and an external use preparation or a cosmetic composition containing the raw material.

### Solution To Problem

The present inventors arrived at the present invention as a result of conducting dedicated research in order to achieve the objective described above. Specifically, the first object of the present invention is achieved by an organopolysiloxane elastomer having a silicon-bonded sugar alcohol-modified group and having a crosslinked three-dimensional network structure comprising a carbon-silicon bond in the crosslinking portion.

The sugar alcohol-modified group is preferably expressed by the following general formula (4-1): 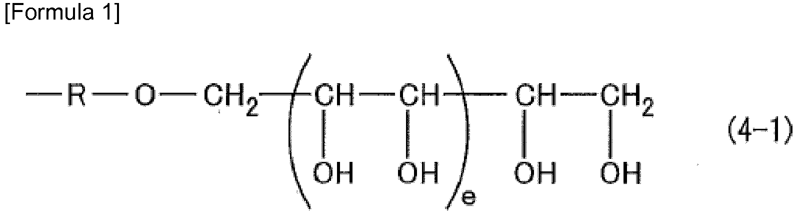 (wherein
R is a divalent organic group, and
e is 1 or 2) or the following general formula (4-2): 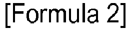 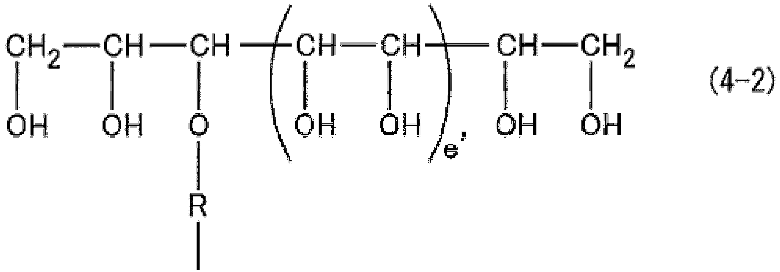
(wherein
R is as defined above, and
e' is 0 or 1).

In the general formula (4-1) or (4-2) described above, the divalent organic group represented by R is preferably a substituted or unsubstituted, straight-chain or branched divalent hydrocarbon group having from 3 to 5 carbon atoms.

The organopolysiloxane elastomer described above can be obtained by reacting:
(A) an organohydrogenpolysiloxane;
(B) a sugar alcohol group-containing organic compound having a reactive unsaturated group; and
(C) at least one type of organic compound selected from the group consisting of (C1) an organic compound having an average number of reactive unsaturated groups in the molecule that is greater than 1 and (C2) an organic compound having at least one reactive unsaturated group and at least one epoxy group in the molecule.

The average number of silicon-bonded hydrogen atoms per molecule of the component (A), which reacts with reactive unsaturated groups of the component (C) constituting the crosslinking portion, is preferably at least 1.5.

The component (A) is preferably expressed by the average composition formula (1):
[Formula 3]

R¹ₐH_{b}SiO_{(4-a-b)/2} (1)

(wherein R¹ moieties are each independently monovalent organic groups, 1.0≤a≤3.0, and 0.001≤b≤1.5).

The component (C) is preferably at least one organic compound selected from the following formulas (C1-1) to (C1-5) and (C2-1) to (C2-2):
(C1-1) an α,ω-diene expressed by the general formula (2-1):
   [Formula 4]

   CH₂=CH(CH₂)ₓCH=CH₂ (2-1)

   (wherein 1≤x≤20);
(C1-2) an α,ω-diyne expressed by the general formula (2-2):
   [Formula 5]

   CH≡C(CH₂)ₓC≡CH (2-2)

   (wherein 1≤x≤20);
(C1-3) an α,ω-ene-yne expressed by the general formula (2-3):
   [Formula 6]

   CH₂=CH(CH₂)ₓC≡CH (2-3)

   (wherein 1≤x≤20);
(C1-4) a bisalkenyl polyether compound expressed by the general formula (2-4):
   [Formula 7]

   CₘH₂ₘ₋₁O(CₙH₂ₙO)_{y}CₘH₂ₘ₋₁ (2-4)

   (wherein 2≤m≤20, 2≤n≤4, y is the total value of the number of repetitions of the oxyethylene unit, the oxypropylene unit, and the oxybutylene unit, and 1≤y≤180);
(C1-5) an unsaturated group-containing silicone-compound expressed by the average composition formula (2-5):
   [Formula 8]

   R²ₚR³_{q}SiO_{(4-p-q)/2} (2-5)

   (wherein R² moieties may each be independent from one another but are monovalent organic groups that are different from R³;
   R³ moieties are each independently monovalent unsaturated aliphatic hydrocarbon groups having from 2 to 30 carbon atoms, 1.0≤p≤2.5, and 0.001≤q≤1.5);
(C2-1) an unsaturated epoxy compound expressed by the general formula (2-6): 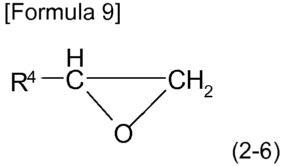 (wherein R⁴ is a substituted or unsubstituted, straight-chain or branched monovalent hydrocarbon group having one reactive unsaturated group and from 2 to 20 carbon atoms); and (C2-2) an unsaturated group-containing cycloaliphatic epoxy compound expressed by the general formula (2-7):
(C2-2) an unsaturated group-containing cycloaliphatic epoxy compound expressed by the general formula (2-7): 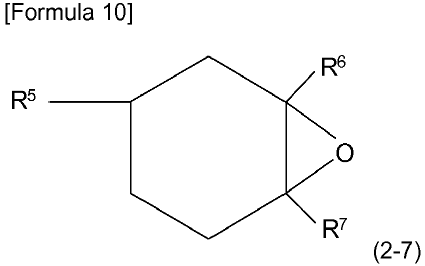 (wherein R⁵ is a substituted or unsubstituted, straight-chain or branched monovalent hydrocarbon group having one reactive unsaturated group and from 2 to 20 carbon atoms;
   R⁶ is a hydrogen atom or a methyl group; and
   R⁷ is a hydrogen atom or a methyl group).

In the average composition formula (1), the monovalent organic group represented by R¹ is preferably selected from the following (D1) to (D10):
(D1) a substituted or unsubstituted, straight-chain or branched monovalent hydrocarbon group having from 1 to 60 carbon atoms;
(D2) a polyoxyalkylene group expressed by -R⁸O(AO)_{z}R⁹ (wherein AO is an oxyalkylene group having from 2 to 4 carbon atoms; R⁸ is a substituted or unsubstituted, straight-chain or branched divalent hydrocarbon group having from 3 to 5 carbon atoms; R⁹ is a hydrogen atom, a substituted or unsubstituted, straight-chain or branched monovalent hydrocarbon group having from 1 to 24 carbon atoms, or a substituted or unsubstituted, straight-chain or branched acyl group having from 2 to 24 carbon atoms; and z=1 to 100);
(D3) a substituted or unsubstituted, straight-chain or branched alkoxy group having from 1 to 30 carbon atoms;
(D4) a hydroxyl group;
(D5) an ester group expressed by -R¹⁰-COOR¹¹ (wherein R¹⁰ is a substituted or unsubstituted, straight-chain or branched divalent hydrocarbon group having from 2 to 20 carbon atoms, and
   R¹¹ is a substituted or unsubstituted, straight-chain or branched monovalent hydrocarbon group having from 1 to 30 carbon atoms);
(D6) an ester group expressed by -R¹⁷-OCOR¹⁸ (wherein R¹⁷ substituted or unsubstituted, straight-chain or branched divalent hydrocarbon group having from 2 to 20 carbon atoms, and
   R¹⁸ is a substituted or unsubstituted, straight-chain or branched monovalent hydrocarbon group having from 1 to 30 carbon atoms);
(D7)L¹
   here, L¹ is a silylalkyl group having a siloxane dendron structure and, when i=1, is expressed by the following general formula (3): 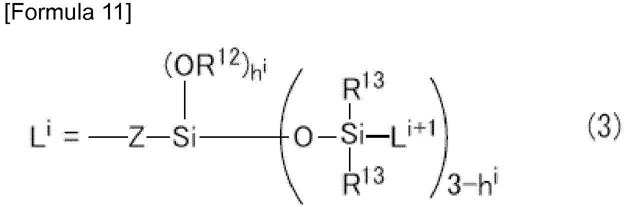 (wherein
   R¹² is a substituted or unsubstituted, straight-chain or branched monovalent hydrocarbon group having from 1 to 30 carbon atoms;
   R¹³ moieties each independently represents an alkyl group or a phenyl group having from 1 to 6 carbon atoms;
   Z is a divalent organic group;
   i represents a generation of the silylalkyl group represented by Lⁱ and is an integer of 1 to k when k is the number of generations, which is the number of repetitions of the silylalkyl group; the number of generations k is an integer from 1 to 10; Lⁱ⁺¹ is the silylalkyl group when i is less than k, and R¹³ when i=k; and hⁱ is a number in a range from 0 to 3);
(D8) an alkyl group substituted by a chain polysiloxane structure expressed by the following general formula (4): 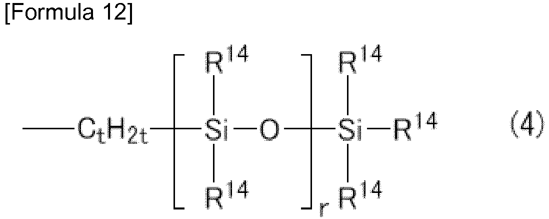 (wherein R¹⁴ moieties are each independently substituted or unsubstituted, straight-chain or branched monovalent hydrocarbon groups having from 1 to 30 carbon atoms, hydroxyl groups, or hydrogen atoms, and at least one of the R¹⁴ moieties is the monovalent hydrocarbon group; t is a number in a range from 2 to 10; and r is a number in a range from 1 to 100);
(D9) an epoxy group expressed by the following general formula (5): 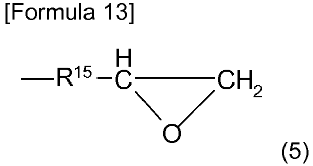 (wherein R¹⁵ is a substituted or unsubstituted, straight-chain or branched divalent hydrocarbon group having from 2 to 20 carbon atoms); and
(D10) a cycloaliphatic epoxy group expressed by the following general formula (6): 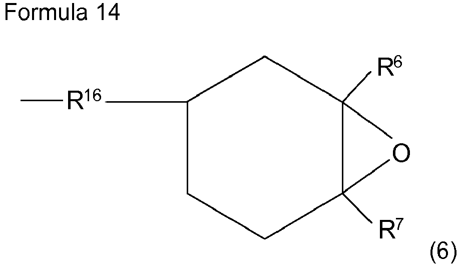 (wherein R¹⁶ is a substituted or unsubstituted, straight-chain or branched divalent hydrocarbon group having from 2 to 20 carbon atoms, and R⁶ and R⁷ are synonymous with those described above).

The organopolysiloxane elastomer of the present invention is preferably in a particulate form and is more preferably in a particulate form with a volume average particle size in the range of from 20 to 1000 µm.

The organopolysiloxane elastomer of the present invention is preferably swellable with an oil agent content of at least the weight of the elastomer itself.

Further, the first object of the present invention can be achieved by a production method for a liquid organopolysiloxane comprising a process of reacting:
(A) an organohydrogenpolysiloxane;
(B) a sugar alcohol group-containing organic compound having a reactive unsaturated group; and
(C) at least one type of organic compound selected from the group consisting of (C1) an organic compound having an average number of reactive unsaturated groups in the molecule that is greater than 1 and (C2) an organic compound having at least one reactive unsaturated group and at least one epoxy group in the molecule.

In the production method for the organopolysiloxane elastomer described above, it is preferable for part or all of the reaction to be performed in the presence of at least one solvent selected from the group indicated by (P-1) to (P-2) below.
(P-1): organic compound
(P-2): compound containing silicon atoms

In one aspect of the production method for the organopolysiloxane elastomer described above, the component (A) and the component (B) are reacted first, and a crosslinking reaction is then performed by adding the component (C). In this case, any component (Q) shown below may be reacted with the component (A) prior to the reaction between the component (A) and the component (B), further reacted after the reaction between the component (A) and the component (B), or further reacted after crosslinking with the component (C). (Q): a compound having one unsaturated group in the molecule (excluding the compound of (C2))

In another aspect of the production method for the organopolysiloxane elastomer described above, a reaction between the component (A) and the component (C) is first performed to derive a crosslinked portion, and the component (B) is then added and reacted thereafter. In this case, any component (Q) shown below may be reacted with the component (A) prior to the reaction between the component (A) and the component (C), further reacted after the reaction between the component (A) and the component (C), or further reacted after the reaction of the component (B). (Q): a compound having one unsaturated group in the molecule (excluding the compound of (C2))

The second object of the present invention can be achieved by a raw material for an external use preparation, a raw material for a cosmetic composition, an external use preparation, or a cosmetic composition containing the organopolysiloxane elastomer; a composition containing at least one type of oil agent in addition to the organopolysiloxane elastomer; and a raw material for an external use preparation or a cosmetic composition, an external use preparation, or a cosmetic composition containing the composition.

The composition described above can be obtained by mixing the organopolysiloxane elastomer of the present invention with at least one type of oil agent after grinding the elastomer using a mechanical force or grinding a mixture of the organopolysiloxane elastomer and at least one type of oil agent using a mechanical force.

The composition may be in the form of an emulsion or a paste.

The raw material for use in an external use preparation or a cosmetic composition can be a thickening agent, a gelling agent, a tactile sensation improver, a surfactant, an emulsifier, or a powder dispersion stabilizer.

The third object of the present invention can be achieved by further performing an acidizing process of treating the organopolysiloxane elastomer obtained by the production method for the organopolysiloxane elastomer described above with at least one type of an acidic substance and a raw material for an external use preparation or a cosmetic composition, an external use preparation, or a cosmetic composition containing the composition described above. A process of heating and/or decompressing the composition is preferably performed after the acidizing process. Further, it is preferable to neutralize the composition by adding at least one type of a basic substance after the acidizing treatment process.

### Advantageous Effects of Invention

With the present invention, it is possible to provide a novel organomodified organopolysiloxane elastomer, wherein the novel organomodified organopolysiloxane elastomer has affinity with various oil agents, has excellent viscosity-improving characteristics, gelification characteristics, and emulsification characteristics, imparts excellent tactile sensation - in particular, an excellent tactile sensation characterized by smoothness with a velvet-like thickness - and imparts an excellent sensation during use.

The organomodified organopolysiloxane elastomer of the present invention has a thickening or gelling effect on various oil agents and can efficiently and stably thicken or gelate various oil agents.

In addition, the organomodified organopolysiloxane elastomer of the present invention can demonstrate excellent emulsifying performance with respect to both nonpolar oil agents and polar oil agents in emulsions in which both water and an oil agent are present. The emulsion may also contain a polyhydroxy alcohol. Accordingly, it is possible to design an external use preparation or a cosmetic composition with various formulations by compounding the organomodified organopolysiloxane elastomer of the present invention in an external use preparation or a cosmetic composition. It is unnecessary for the preparation for external use or the cosmetic of the present invention to include a polyether-containing compound.

In particular, a composition containing the organomodified organopolysiloxane elastomer of the present invention has the contradictory characteristics of having an extremely soft tactile sensation while being in a highly viscous form capable of guaranteeing stability. This effect is prominent when the organomodified organopolysiloxane elastomer is in a particulate form.

Further, the organomodified organopolysiloxane elastomer of the present invention has an excellent effect of maintaining the dispersion state of a powder dispersed in a medium, which makes it possible to enhance the storage stability of a composition containing the powder, in particular.

Due to the functionality thereof, the organomodified organopolysiloxane elastomer of the present invention can be advantageously used as a raw material for an external use preparation or a cosmetic composition such as a thickening agent, a gelling agent, a tactile sensation improver, a surfactant, an emulsifier, or a powder dispersion stabilizer and can be compounded in a cosmetic composition or an external use preparation as necessary. In particular, the present invention can provide a nonaqueous emulsion composition with excellent stability that is usable as a drug delivery system. Similarly, the present invention can provide a water-in-oil type or an oil-in-water type emulsion composition having excellent stability.

Additionally, the organomodified organopolysiloxane elastomer of the present invention can be used as a composition with various oil agents because it can be uniformly mixed with a wide range of types of oil agents. Furthermore, a composition comprising an oil agent in conjunction with the organomodified organopolysiloxane elastomer of the present invention has superior storage stability.

With the present invention, the odor of the organomodified organopolysiloxane elastomer can be reduced. The organomodified organopolysiloxane elastomer of the present invention having a reduced odor is particularly suitable as a raw material for an external use preparation or a cosmetic composition or as a component of an external use preparation or a cosmetic composition. In particular, in the present invention, it is possible to provide an organomodified organopolysiloxane elastomer or a composition containing the same which is essentially odorless and demonstrates suppressed odor generation at high temperatures or over time by a simple acidizing.

The odor-reducing effect of the organomodified organopolysiloxane elastomer of the present invention is very high, and the odor-reducing effect achieved in the present invention cannot be achieved even by acidizing another modified organopolysiloxane elastomer in the same manner as in the present invention. The present invention is advantageous in industrial scale implementation and can provide an organomodified organopolysiloxane elastomer or a composition containing the same which is deodorized simply and at low cost.

In addition, the odor of the organomodified organopolysiloxane elastomer of the present invention having a reduced odor does not need to be masked when compounded in an external use preparation or a cosmetic composition, and there is a high degree of flexibility in the design of the formulations of an external use preparation or a cosmetic composition. This is particularly advantageous in cosmetic compositions, in which functions that contain an odor are emphasized, and, therefore, design of a fragrance free cosmetic composition, a faintly scented cosmetic composition, or a cosmetic composition with a desired fragrance is easy.

### DETAILED DESCRIPTION OF THE INVENTION

(Organopolysiloxane elastomer and production method thereof)

A first aspect of the present invention is an organopolysiloxane elastomer having a silicon-bonded sugar alcohol-modified group and having a crosslinked three-dimensional network structure comprising a carbon-silicon bond in the crosslinking portion.

The organopolysiloxane elastomer of the present invention is an elastic substance with a relatively high crosslinking density and is a gel-like, rubber-like, or powder-like solid that is insoluble in solvents or the like. The organopolysiloxane elastomer of the present invention preferably does not have fluidity (non-liquid form) at 25°C. "Not having fluidity at 25°C" means that after the organopolysiloxane elastomer is introduced into a prescribed container and the surface thereof is made horizontal using an instrument such as a trowel, the container is tilted, and the surface does not become horizontal once again after 24 hours. Here, "horizontal" means to form a plane that intersects the direction of gravitational force at a right angle.

In addition, the organopolysiloxane elastomer of the present invention has a crosslinked three-dimensional network structure having a crosslinking portion containing a carbon-silicon bond. The crosslinked three-dimensional network structure also contains a polysiloxane chain. However, the organopolysiloxane elastomer of the present invention has a high crosslinking molecular structure in which the polysiloxane chains are relatively densely crosslinked in a three-dimensional mesh. Accordingly, the organopolysiloxane is a gel-like, rubber-like, or powder-like solid that is insoluble in solvents or the like.

The organopolysiloxane elastomer of the present invention is preferably swellable with an oil agent content of at least the weight of the elastomer itself (mass of the elastomer itself). The organopolysiloxane elastomer of the present invention containing an oil agent can exist in a paste form. The types and the like of the oil agents are as described below, and silicone oils are preferable.

The organopolysiloxane elastomer of the present invention has a silicon-bonded sugar alcohol-modified group. The sugar alcohol-modified group constitutes a hydrophilic site of the organopolysiloxane elastomer of the present invention. The structure of the sugar alcohol-modified group is not limited as long as the structure has a sugar alcohol site, but the sugar alcohol residue is preferably bonded to a silicon atom via a divalent organic group.

Accordingly, the sugar alcohol-modified group is preferably expressed by the following general formula (4-1): 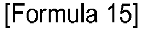 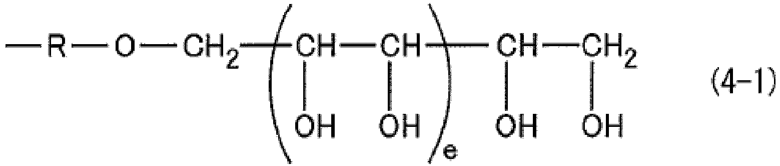 (wherein
R is a divalent organic group, and
e is 1 or 2), or the following general formula (4-2): 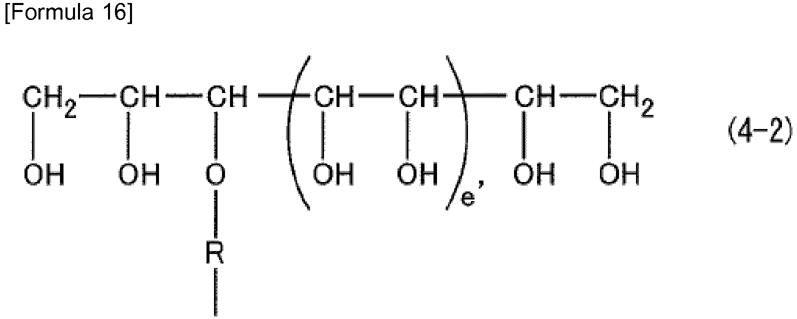 (wherein
R is as defined above, and
e' is 0 or 1).

The organopolysiloxane elastomer of the present invention is characterized in that at least one type of a sugar alcohol-modified group expressed by the general formula (4-1) or (4-2) above is bonded to a silicon atom. Further, the organopolysiloxane may also have two or more types of sugar alcohol-modified groups selected from these sugar alcohol-modified groups in the molecule. Similarly, a mixture of organopolysiloxanes having different sugar alcohol-modified groups may also be used.

The divalent organic group represented by R in the general formula (4-1) or (4-2) is not particularly limited, but examples include substituted or unsubstituted, straight-chain or branched divalent hydrocarbon groups having from 1 to 30 carbon atoms. A substituted or unsubstituted, straight-chain or branched divalent hydrocarbon group having from 3 to 5 carbon atoms is preferable. Examples of the substituted or unsubstituted, and straight-chain or branched divalent hydrocarbon group having from 1 to 30 carbon atoms include: straight-chain or branched alkylene groups having from 1 to 30 carbon atoms such as the methylene group, dimethylene group, trimethylene group, tetramethylene group, pentamethylene group, hexamethylene group, heptamethylene group, octamethylene group, or the like; alkenylene groups having from 2 to 30 carbon atoms such as the vinylene group, allylene group, butenylene group, hexenylene group, octenylene group, or the like; arylene groups having from 6 to 30 carbon atoms such as the phenylene group, diphenylene group, or the like; alkylenearylene groups having from 7 to 30 carbon atoms such as the dimethylenephenylene group or the like; and groups thereof in which hydrogen atoms bonded to carbon atoms of the groups are at least partially substituted by a halogen atom such as a fluorine atom or the like, or an organic group containing the carbinol group, epoxy group, glycidyl group, acyl group, carboxyl group, amino group, methacryl group, mercapto group, amide group, oxyalkylene group, or the like. The divalent hydrocarbon groups are preferably alkylene groups having from 1 to 30 carbon atoms, more preferably are alkylene groups having from 1 to 6 carbon atoms, and even more preferably alkylene groups having from 3 to 5 carbon atoms.

In the sugar alcohol-modified group of the general formula (4-1), it is particularly preferable for R to be a propylene group and for e=1. Similarly, in the sugar alcohol-modified group of the general formula (4-2), it is particularly preferable for R to be a propylene group and for e'=0. The sugar alcohol-modified group in this case is a xylitol residue expressed by the structural formula: -C₃H₆-OCH₂[CH(OH)]₃CH₂OH or the structural formula: -C₃H₆-OCH[CH(OH)CH₂OH]₂ corresponding to the general formula (4-1) or (4-2) (hereinafter, simply referred to as a "xylitol residue" or a "xylitol modified group").

The bond position of the sugar alcohol-modified group may be either the side chain or the terminal of the polysiloxane, which is the main chain, and the organopolysiloxane of the present invention may have two or more sugar alcohol-modified groups. Further, these two or more sugar alcohol-modified groups may be the same or different sugar alcohol-modified groups. These two or more sugar alcohol-modified groups can be structured such that bonding occurs only in a side chain of polysiloxane, which is the main chain, only at a terminal, or in a side chain and at a terminal.

The organopolysiloxane elastomer of the present invention can be produced by reacting:
(A) an organohydrogenpolysiloxane;
(B) a sugar alcohol group-containing organic compound having a reactive unsaturated group; and
(C) at least one type of organic compound selected from the group consisting of (C1) an organic compound having an average number of reactive unsaturated groups in the molecule that is greater than 1 and (C2) an organic compound having at least one reactive unsaturated group and at least one epoxy group in the molecule.

The (A) organohydrogenpolysiloxane is not particularly limited as long as it has silicon-bonded hydrogen atoms, but an organohydrogenpolysiloxane having more than one - preferably from 1.01 to 100, more preferably from 1.1 to 50, even more preferably from 1.2 to 25, and particularly preferably from 1.3 to 10 - silicon-bonded hydrogen atoms in the molecule on average is preferable, and a straight-chain, branched, or reticulated organopolysiloxane may be used. The positions of the silicon-bonded hydrogen atoms in the organohydrogenpolysiloxane are not limited, and can be on the main chain or at the terminals. However, the atoms are preferably positioned at the terminals from the perspective of reducing the degree of crosslinking. One type or two or more types of organohydrogenpolysiloxanes may be used as the component (A).

Examples of the component (A) include 1,1,3,3-tetramethyldisiloxane, 1,3,5,7-tetramethylcyclotetrasiloxane, methylhydrogenpolysiloxane capped at both molecular terminals with trimethylsiloxy groups, dimethylsiloxane-methylhydrogensiloxane copolymers capped at both molecular terminals with trimethylsiloxy groups, dimethylsiloxane capped at both molecular terminals with dimethylhydrogensiloxy groups, dimethylpolysiloxane capped at both molecular terminals with dimethylhydrogensiloxy groups, dimethylsiloxane-methylhydrogensiloxane copolymers capped at both molecular terminals with dimethylhydrogensiloxy groups, methylhydrogensiloxane-diphenylsiloxane copolymers capped at both molecular terminals with trimethylsiloxy groups, methylhydrogensiloxane-diphenylsiloxane-dimethylsiloxane copolymers capped at both molecular terminals with trimethylsiloxy groups, copolymers comprising (CH₃)₂HSiO_{1/2} units and SiO_{4/2} units, and copolymers comprising (CH₃)₂HSiO_{1/2} units, SiO_{4/2} units, and (C₆H₅)SiO_{3/2} units.

The component (A) is preferably expressed by the average composition formula (1):
[Formula 17]

R¹ₐH_{b}SiO_{(4-a-b)/2} (1)

(wherein R¹ moieties are each independently monovalent organic groups, 1.0≤a≤3.0, and 0.001≤b≤1.5).

Although the molecular structure of the (A) organohydrogenpolysiloxane is not limited, examples include straight-chain, partially branching straight-chain, branched-chain, cyclic, and dentric structures, and straight-chain is preferable. The molecular weight is not particularly limited, and products having a low molecular weight to products having a high molecular weight can be used. Specifically, the number-average molecular weight is preferably in a range from 100 to 1,000,000 and more preferably in a range from 300 to 500,000.

Examples of such organohydrogenpolysiloxanes include those expressed by the following structural formulas:
(i) R¹₃SiO(R¹₂SiO)ᵥ(R¹SiHO)_{w}SiR¹₃
(ii) HR¹₂SiO(R¹₂SiO)ᵥ(R¹SiHO)_{z}SiR¹₃
(iii) HR¹₂SiO(R¹₂SiO)ᵥ(R¹SiHO)_{z}SiR¹₂H
   (wherein R¹ is as described above, v is 0 or a positive integer, w is a positive integer, and z is 0 or a positive integer). These organohydrogenpolysiloxanes are straight-chain organohydrogenpolysiloxanes having a silicon-bonded hydrogen atom on (i) only the side chain, (ii) the side chain or one molecular terminal, or (iii) the side chain or both molecular terminals.

The monovalent organic group is not particularly limited but is preferably selected from the following (D1) to (D10):
(D1) a substituted or unsubstituted, straight-chain or branched monovalent hydrocarbon group having from 1 to 60 carbon atoms;
(D2) a polyoxyalkylene group expressed by -R⁸O(AO)_{z}R⁹ (wherein AO is an oxyalkylene group having from 2 to 4 carbon atoms; R⁸ is a substituted or unsubstituted, straight-chain or branched divalent hydrocarbon group having from 3 to 5 carbon atoms; R⁹ is a hydrogen atom, a substituted or unsubstituted, straight-chain or branched monovalent hydrocarbon group having from 1 to 24 carbon atoms, or a substituted or unsubstituted, straight-chain or branched acyl group having from 2 to 24 carbon atoms; and z=1 to 100);
(D3) a substituted or unsubstituted, straight-chain or branched alkoxy group having from 1 to 30 carbon atoms;
(D4) a hydroxyl group;
(D5) an ester group expressed by -R¹⁰-COOR¹¹ (wherein R¹⁰ is a substituted or unsubstituted, straight-chain or branched divalent hydrocarbon group having from 2 to 20 carbon atoms, and R¹¹ is a substituted or unsubstituted, straight-chain or branched monovalent hydrocarbon group having from 1 to 30 carbon atoms);
(D6) an ester group expressed by -R¹⁷-OCOR¹⁸ (wherein R¹⁷ is a substituted or unsubstituted, straight-chain or branched divalent hydrocarbon group having from 2 to 20 carbon atoms, and R¹⁸ is a substituted or unsubstituted, straight-chain or branched monovalent hydrocarbon group having from 1 to 30 carbon atoms);
(D7) L¹
   here, L¹ is a silylalkyl group having a siloxane dendron structure and, when i=1, is expressed by the following general formula (3): 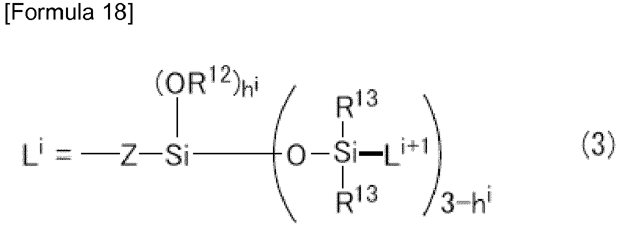 (wherein
   R¹² is a substituted or unsubstituted, straight-chain or branched monovalent hydrocarbon group having from 1 to 30 carbon atoms;
   R¹³ moieties each independently represents an alkyl group or a phenyl group having from 1 to 6 carbon atoms;
   Z is a divalent organic group;
   i represents a generation of the aforementioned silylalkyl group represented by Lⁱ and is an integer of 1 to k when k is the number of generations, which is the number of repetitions of the silylalkyl group; the number of generations k is an integer from 1 to 10; Lⁱ⁺¹ is the silylalkyl group when i is less than k, and R¹³ when i=k; and hⁱ is a number in a range from 0 to 3); (D8) an alkyl group substituted by a chain polysiloxane structure expressed by the following general formula (4): 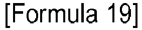 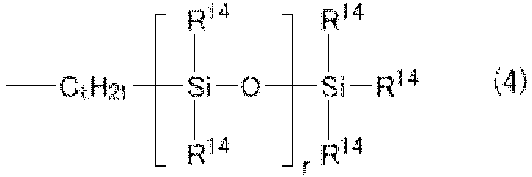
   (wherein R¹⁴ moieties are each independently substituted or unsubstituted, straight-chain or branched monovalent hydrocarbon groups having from 1 to 30 carbon atoms, hydroxyl groups, or hydrogen atoms, and at least one of the R¹⁴ moieties is the monovalent hydrocarbon group; t is a number in a range from 2 to 10; and r is a number in a range from 1 to 100);
(D9) an epoxy group expressed by the following general formula (5): 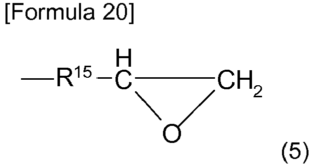 (wherein R¹⁵ is a substituted or unsubstituted, straight-chain or branched divalent hydrocarbon group having from 2 to 20 carbon atoms); and
(D10) a cycloaliphatic epoxy group expressed by the following general formula (6): 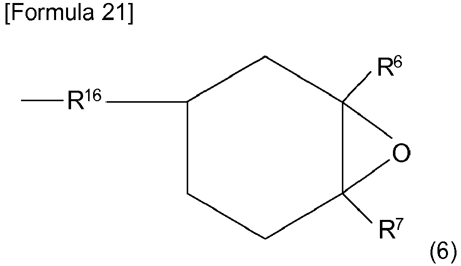 (wherein R¹⁶ is a substituted or unsubstituted, straight-chain or branched divalent hydrocarbon group having from 2 to 20 carbon atoms, and R⁶ and R⁷ are synonymous with those described above).

Examples of the substituted or unsubstituted, straight-chain or branched monovalent hydrocarbon group in (D1), (D2), and (D5) to (D8) include alkyl groups such as methyl groups, ethyl groups, propyl groups, butyl groups, pentyl groups, hexyl groups, heptyl groups, and octyl groups; cycloalkyl groups such as cyclopentyl groups and cyclohexyl groups; alkenyl groups such as vinyl groups, allyl groups, and butenyl groups; aryl groups such as phenyl groups and tolyl groups; aralkyl groups such as benzyl groups; and groups in which the hydrogen atoms bonded to the carbon atoms of these groups are substituted at least partially by halogen atoms such as fluorine atoms or organic groups such as epoxy groups, glycidyl groups, acyl groups, carboxyl groups, amino groups, methacryl groups, and mercapto groups. The monovalent hydrocarbon group is preferably a group other than an alkenyl group, and is particularly preferably a methyl group, an ethyl group, or a phenyl group.

The substituted or unsubstituted, straight-chain or branched divalent hydrocarbon group in (D2), (D5), (D6), (D9), and (D10) is as described above.

Examples of the substituted or unsubstituted, straight-chain or branched alkoxy group in (D3) include lower alkoxy groups such as methoxy groups, ethoxy groups, isopropoxy groups, and butoxy groups and higher alkoxy groups such as lauryl alkoxy groups, myristyl alkoxy groups, palmityl alkoxy groups, oleyl alkoxy groups, stearyl alkoxy groups, and behenyl alkoxy groups.

Among the phenyl group or the alkyl group having from 1 to 6 carbon atoms of (D7), examples of the alkyl group having from 1 to 6 carbon atoms include methyl, ethyl, n-propyl, i-propyl, n-butyl, i-butyl, s-butyl, pentyl, neopentyl, cyclopentyl, hexyl, and similar straight, branched, or cyclic alkyl groups.

In the aforementioned general formula (3), in the case of i = k, R⁴ is preferably a methyl group or a phenyl group. In particular, R4 is preferably a methyl group when i=k.

From a technical standpoint, the number of generations k is preferably an integer from 1 to 3, and more preferably is 1 or 2. In each of the number of generations, the group represented by L¹ is expressed as follows. In the formulae, R¹², R¹³, and Z are the same groups as described above.

When the number of generations is k=1, L¹ is expressed by the following general formula (3-1).

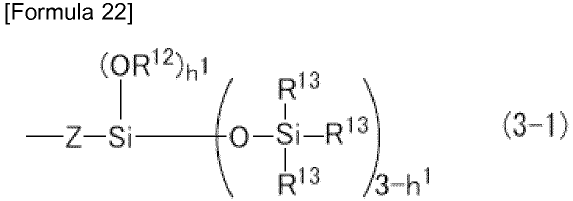

When the number of generations is k=2, L1 is expressed by the following general formula (3-2).

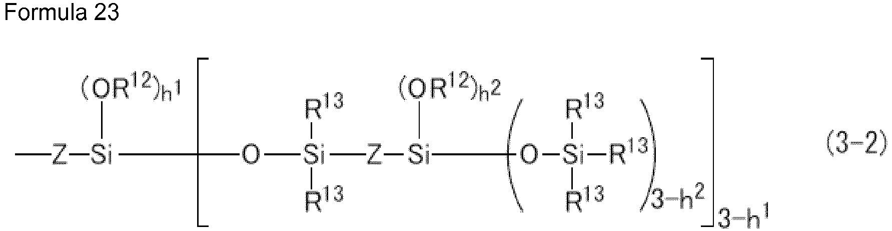

When the number of generations is k=3, L1 is expressed by the following general formula (3-3).

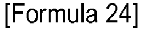

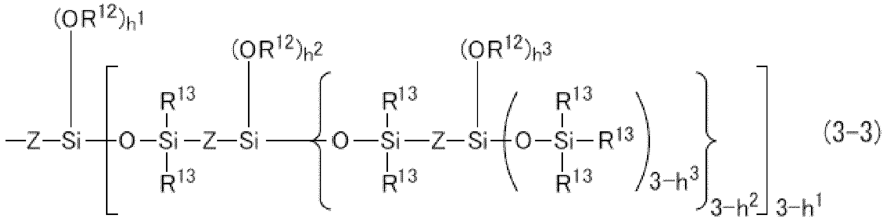

In the structures expressed by the general formulae (3-1) to (3-3) in the case of the number of generations is from 1 to 3, each of h¹, h² and h³ moieties is independently a number in a range from 0 to 3. These hⁱ moieties are preferably a number in a range from 0 to 1, and hⁱ is, in particular, preferably 0.

In general formulae (3) and (3-1) to (3-3), Z are each independently a divalent organic group, and specific examples thereof include a divalent organic group formed by addition-reacting a silicon-bonded hydrogen atom and a functional group having an unsaturated hydrocarbon group such as an alkenyl group, an acryloxy group, a methacryloxy group, or the like at the terminal. Depending on the method for introducing the silylalkyl group having a siloxane dendron structure, the functional group can be appropriately selected and is not restricted to the functional groups described above. Preferably, Z are each independently a group selected from divalent organic groups expressed by the following general formula.
[Formula 25]
-R¹⁹-
-R¹⁹-CO-
-R¹⁹-COO-R²⁰-
-CO-R¹⁹-
-R¹⁹-COO-R²⁰-
-R¹⁹-CONH-R²⁰-
-R¹⁹-R²⁰-
Of these, Z in L¹ is preferably a divalent organic group expressed by general formula -R¹⁹- that is introduced by a reaction between a silicon-bonded hydrogen atom and an alkenyl group. Likewise, Z is preferably a divalent organic group expressed by general formula -R¹⁹-COO-R²⁰- that is introduced by a reaction between a silicon-bonded hydrogen atom and an unsaturated carboxylic ester group. On the other hand, in the silylalkyl group represented by Lⁱ, in which the number of generations k is 2 or more, and Lⁱ is L² to L^{k}, Z is preferably an alkylene group having from 2 to 10 carbon atoms and, in particular, is preferably a group selected from an ethylene group, a propylene group, a methylethylene group, and a hexylene group, and most preferably is an ethylene group.

In the general formula described above, R¹⁹ moieties are each independently a substituted or unsubstituted, straight or branched chain alkylene group or alkenylene group having from 2 to 22 carbon atoms or an arylene group having from 6 to 22 carbon atoms. More specifically, examples of R¹⁹ include an ethylene group, a propylene group, a butylene group, a hexylene group, and similar straight alkylene groups; a methylmethylene group, a methylethylene group, a 1-methylpentylene group, a 1,4 -dimethylbutylene group, and similar branched alkylene groups. R⁷ is preferably a group selected from an ethylene group, a propylene group, a methylethylene group, and a hexylene group.

In the general formula described above, R²⁰ is a group selected from divalent organic groups expressed by the following formula.

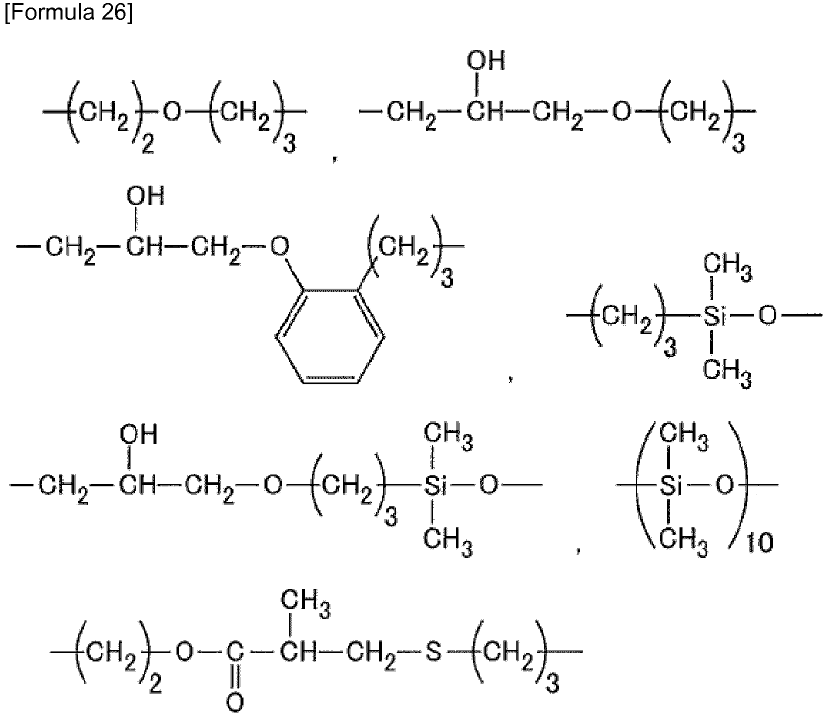

The (B) sugar alcohol-modified group-containing compound having a reactive unsaturated group is not particularly limited as long as the compound has at least one reactive unsaturated group and one sugar alcohol-modified group in the molecule, but a monounsaturated ether compound of the sugar alcohol represented by the following general formula (4'-1): 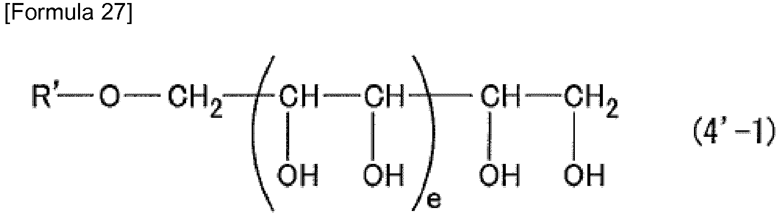 (wherein
R' is an unsaturated organic group, and
e is 1 or 2 and more preferably 1) or the following general formula (4'-2): 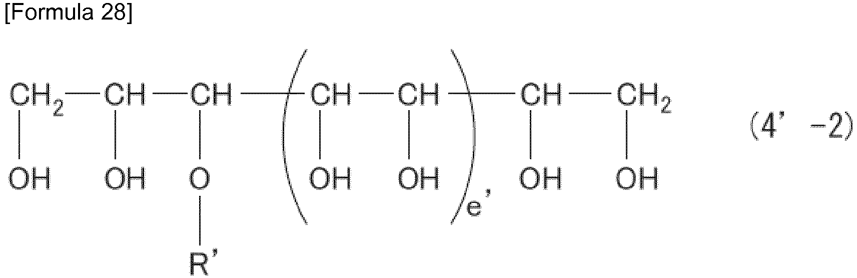 (wherein
R' is an unsaturated organic group, and
e' is 0 or 1 and more preferably 0) is preferable.

The unsaturated organic groups are not particularly limited as long as it has an unsaturated group, but a substituted or unsubstituted, straight-chain or branched unsaturated hydrocarbon group having from 3 to 5 carbon atoms is preferable. Examples of the unsaturated hydrocarbon group having from 3 to 5 carbon atoms include vinyl groups, allyl groups, butenyl groups, methallyl groups and similar alkenyl groups. Among these, allyl groups are preferable.

As the monounsaturated ether compound of the sugar alcohol, a monoallyl ether of a sugar alcohol is preferable, and a xylitol monoallyl ether expressed by the structural formula: CH₂=CH-CH₂-OCH₂[CH(OH)]₃CH₂OH or the structural formula: CH₂=CH-CH₂-OCH[CH(OH)CH₂OH]₂ (hereinafter, referred to as a "xylitol monoallyl ether") is more preferable. The xylitol monoallyl ether can be synthesized with a conventionally known method, and a commercially available product may also be used.

The xylitol monoallyl ether may be only one of the compounds represented by the structural formula: CH₂=CH-CH₂-OCH₂[CH(OH)]₃CH₂OH and the structural formula: CH₂=CH-CH₂-OCH[CH(OH)CH₂OH]₂, or mixtures thereof can be used without any particularly restrictions. In particular, it is preferable to refine a xylitol monoallyl ether represented by the structural formula: CH₂=CH-CH₂-OCH₂[CH(OH)]₃CH₂OH or the structural formula: CH₂=CH-CH₂-OCH[CH(OH)CH₂OH]₂ and use the product as a raw material or to use a xylitol monoallyl ether containing xylitol monoallyl ethers represented by the structural formula: CH₂=CH-CH₂-OCH₂[CH(OH)]₃CH₂OH and the structural formula: CH₂=CH-CH₂-OCH[CH(OH)CH₂OH]₂ at a compositional ratio in a range from 5:5 to 10:0 as a raw material. In the latter case, it is more preferable to use a xylitol monoallyl ether containing the substances at a ratio in a range from 8:2 to 10:0. Moreover, if the compositional ratio is 10:0, the raw material is essentially a refined product comprising only a xylitol monoallyl ether represented by the structural formula: CH₂=CH-CH₂-OCH₂[CH(OH)]₃CH₂OH.

In order to obtain the organopolysiloxane elastomer of the present invention, a derivative (ketal derivative) of a sugar alcohol compound prepared by protecting a hydroxyl group of the sugar alcohol compound corresponding to the introduced sugar alcohol-modified group with a ketalizing agent such as 2,2-dimethoxypropane in the presence of an acid catalyst can be used as a raw material. Specifically, a ketal derivative of a sugar alcohol having a carbon-carbon double bond in the molecule obtained by refining the reaction product of the ketal compound described above and an alkenyl halide is subjected to an addition reaction with an organohydrogenpolysiloxane instead of the monounsaturated ether compound of the sugar alcohol described above. After the addition reaction, the organopolysiloxane can be produced by performing a deketalation reaction by acid hydrolysis treatment to deprotect the hydroxyl group. Even with a production method using the ketal derivative described above, an organopolysiloxane elastomer having a sugar alcohol-modified group can be obtained after deprotection, but this method is typically inefficient. Either production method may be selected in accordance with conditions such as the desired yield, the production equipment, and the refinement of the raw material. Either production method may also be selected for the purpose of improving the quality such as the purity or the desired characteristics of the organopolysiloxane elastomer.

There are no particularly restrictions regarding the structure of (C1) the organic compound having an average number of reactive unsaturated groups in the molecule that is greater than 1 serving as the component (C) as long as the compound has more than 1 - preferably from 1.01 to 10, more preferably from 1.2 to 8, even more preferably from 1.5 to 6, and particularly preferably from 2.0 to 4.5 - reactive unsaturated groups and preferably carbon-carbon double bonds on average in the molecule, and straight-chain, branched, or reticulated organic compounds may be used. An organopolysiloxane or an unsaturated aliphatic hydrocarbon is preferable as an organic compound. There are also no restrictions regarding the position of the organic compound, preferably the organopolysiloxane, the unsaturated aliphatic hydrocarbon, or the aforementioned reactive unsaturated group, and the component may be positioned on the main chain or on a terminal. However, from the perspective of the ease of controlling the crosslinking density, it is preferable to use a compound of high purity having two unsaturated groups in the molecule, each of which is positioned at either terminal, for example.

A reactive unsaturated group is preferably present in an unsaturated aliphatic hydrocarbon group. The unsaturated aliphatic hydrocarbon group preferably has from 2 to 30 carbon atoms and more preferably has from 2 to 20 carbon atoms. Examples of the monovalent unsaturated aliphatic hydrocarbon group having from 2 to 30 carbon atoms include straight-chain or branched alkenyl groups such as vinyl groups, 1-propenyl groups, allyl groups, isopropenyl groups, 1-butenyl groups, 2-butenyl groups, pentenyl groups, and hexenyl groups; cycloalkenyl groups such as cyclopentenyl groups and cyclohexenyl groups; cycloalkenylalkyl groups such as cyclopentenylethyl groups, cyclohexenylethyl groups, and cyclohexenylpropyl groups; and alkynyl groups such as ethynyl groups and propargyl groups. Alkenyl groups are preferred, and vinyl groups and hexenyl groups are particularly preferred.

When the component (C1) is an organopolysiloxane, the unsaturated aliphatic hydrocarbon group containing a reactive unsaturated group is preferably bonded to a silicon atom. In addition, when the component (C1) is an organopolysiloxane, the group bonding to silicon atoms other than the unsaturated aliphatic hydrocarbon may be a substituted or unsubstituted monovalent hydrocarbon group or a monovalent organic group having a reactive functional group.

Substituted or unsubstituted monovalent hydrocarbon groups are typically substituted or unsubstituted, straight or branched monovalent saturated hydrocarbon groups having from 1 to 30 carbon atoms, preferably from 1 to 10 carbon atoms, and more preferably from 1 to 4 carbon atoms, and substituted or unsubstituted monovalent aromatic hydrocarbon groups having from 6 to 30 carbon atoms, and more preferably from 6 to 12 carbon atoms. Moreover, component (C1) may contain, as a monovalent organic group, a hydroxyl group or an alkoxy group having from 1 to 12 carbon atoms, such as a methoxy group, an ethoxy group, a propoxy group or a butoxy group.

Examples of the monovalent saturated hydrocarbon group having from 1 to 30 carbon atoms include straight chain or branched chain alkyl groups such as methyl groups, ethyl groups, n-propyl groups, isopropyl groups, n-butyl groups, isobutyl groups, sec-butyl groups, tert-butyl groups, pentyl groups, hexyl groups, heptyl groups, octyl groups, nonyl groups, decyl groups, and the like; and cycloalkyl groups such as cyclopentyl groups, cyclohexyl groups, cycloheptyl groups, cyclooctyl groups, and the like.

Examples of the monovalent aromatic hydrocarbon group having from 6 to 30 carbon atoms include aryl groups such as phenyl groups, tolyl groups, xylyl groups, mesityl groups, and the like. Of these, a phenyl group is preferable. Note that, in the present specification, "aromatic hydrocarbon group" also includes groups in which an aromatic hydrocarbon and a saturated aliphatic hydrocarbon are conjugated in addition to groups formed only from an aromatic hydrocarbon. Examples of groups in which an aromatic hydrocarbon and a saturated hydrocarbon are conjugated include aralkyl groups such as benzyl groups, phenethyl groups, and the like.

Hydrogen atoms in the above-mentioned monovalent hydrocarbon groups may be substituted by one or more substituted groups, and said substituted groups may be selected from the group consisting of, for example, a halogen atom (a fluorine atom, a chlorine atom, a bromine atom, or an iodine atom), a hydroxyl group, an amide group, an ester group, a carboxyl group and an isocyanate group. A monovalent saturated or aromatic hydrocarbon group having at least one of the above-mentioned substituted groups is preferred. Specifically, it is possible to use a 3,3,3-trifluoropropyl group, a 3-chloropropyl group, a 3-hydroxypropyl group, a 3-(2-hydroxyethoxy)propyl group, a 3-carboxypropyl group, a 10-carboxydecyl group, a 3-isocyanatopropyl group and the like.

Examples of monovalent organic groups having reactive functional groups include monovalent saturated or aromatic hydrocarbon groups having reactive functional groups selected from the group consisting of, for example, hydroxyl groups, mercapto groups, epoxy groups, amino groups, amide groups, ester groups, carboxyl groups and isocyanate groups. One or a plurality of reactive functional groups may exist in the monovalent organic group. R¹ is preferably a monosaturated or aromatic hydrocarbon group having at least one of the reactive functional groups described above. Specific examples of the reactive functional group include 3-hydroxypropyl groups, 3-(2-hydroxyethoxy)propyl groups, 3-mercaptopropyl groups, 2,3-epoxypropyl groups, 3,4-epoxybutyl groups, 4,5-epoxypentyl groups, 2-glycidoxyethyl groups, 3-glycidoxypropyl groups, 4-glycidoxybutyl groups, 2-(3,4-epoxycyclohexyl) ethyl groups, 3-(3,4-epoxycyclohexyl)propyl groups, aminopropyl groups, N-methylaminopropyl groups, N-butylaminopropyl groups, N,N-dibutylaminopropyl groups, 3-(2-aminoethoxy)propyl groups, 3-(2-aminoethylamino)propyl groups, 3-carboxypropyl groups, 10-carboxydecyl groups, 3-isocyanate propyl groups, and the like.

A straight-chain or branched polysiloxane is preferable as the component (C1). A straight-chain component (C1) is preferably a polymer having a diorganosiloxane unit and a triorganosiloxane unit, examples of which include dimethylpolysiloxanes capped at both molecular terminals with dimethylvinylsiloxy groups, copolymers of dimethylsiloxane and methylphenylsiloxane capped at both molecular terminals with dimethylvinylsiloxy groups, copolymers of dimethylsiloxane and methylvinylsiloxane capped at both molecular terminals with dimethylvinylsiloxy groups, copolymers of dimethylsiloxane and methylvinylsiloxane capped at both molecular terminals with trimethylsiloxy groups, copolymers of dimethylsiloxane, methylvinylsiloxane and methylphenylsiloxane capped at both molecular terminals with trimethylsiloxy groups, copolymers of dimethylsiloxane and methylvinylsiloxane capped at both molecular terminals with silanol groups, polymers in which some of the methyl groups in these polymers are substituted by alkyl groups other than methyl groups, such as ethyl groups or propyl groups, or halogenated alkyl groups such as 3,3,3-trifluoropropyl groups, and mixtures of two or more of these polymers, with straight-chain diorganopolysiloxanes having unsaturated aliphatic hydrocarbon groups, and especially alkenyl groups, at both molecular terminals only being particularly preferred.

It is particularly preferable for a branched chain polysiloxane of component (C1) to be a polymer that contains a diorganosiloxane unit, an organosilsesquioxane unit and a triorganosiloxy unit. Silicon-bonded organic groups in these units are preferably monovalent hydrocarbon groups including alkyl groups such as methyl groups, ethyl groups and propyl groups; alkenyl groups such as vinyl groups, allyl groups, butenyl groups and hexenyl groups; aryl groups such as phenyl groups and tolyl groups; and halogenated alkyl groups such as 3,3,3-trifluoropropyl groups, and the like, and may contain extremely small quantities of hydroxyl groups and alkoxy groups such as methoxy groups, but at least two silicon-bonded organic groups in this polymer must be unsaturated aliphatic hydrocarbon groups, and especially alkenyl groups. In addition, the proportions of these units are not limited, but in this polymer, it is preferable for diorganosiloxane units to account for in the range of 80.00 to 99.65 mol% and organosilsesquioxane units to account for in the range of 0.10 to 10.00 mol%, with the balance comprising triorganosiloxy units.

Examples of the component (C1) include (C1-5) unsaturated group-containing silicone compounds expressed by the average composition formula (2-5):
[Formula 29]

R²ₚR³_{q}SiO_{(4-p-q)/2} (2-5)

(wherein R² moieties may each be independent from one another but are monovalent organic groups that are different from R³;
R³ moieties are each independently monovalent unsaturated aliphatic hydrocarbon groups having from 2 to 30 carbon atoms, 1≤p≤2.5, and 0.001≤q≤1.5). The monovalent unsaturated aliphatic hydrocarbon group having from 2 to 30 carbon atoms is as described above.

In the average composition formula (2-5), the monovalent organic group represented by R² is not particularly limited, but is preferably selected from the following (E1) to (E6):
(E1) a substituted or unsubstituted, straight-chain or branched monovalent hydrocarbon group having from 1 to 60 carbon atoms (excluding monovalent hydrocarbon groups having from 2 to 20 carbon atoms and an aliphatic unsaturated group);
(E2) a hydroxyl group;
(E3) an ester group expressed by -R¹⁰-COOR¹¹ (wherein R¹⁰ and R¹¹ are as defined above);
(E4) an ester group expressed by -R¹⁷-OCOR¹⁸ (wherein R¹⁷ and R¹⁸ are as defined above);
(E5) an amide group expressed by -R²¹-NR²²COR²³ (wherein R²¹ is a substituted or unsubstituted, straight-chain or branched divalent hydrocarbon group having from 2 to 20 carbon atoms, R²² is a hydrogen atom, or a substituted or unsubstituted, straight-chain or branched monovalent hydrocarbon group having from 1 to 20 carbon atoms, and R²³ is a substituted or unsubstituted, straight-chain or branched monovalent hydrocarbon group having from 1 to 30 carbon atoms); and
(E6) an amide group expressed by -R²⁴-CONR²⁵R²⁶ (wherein R²⁴ is a substituted or unsubstituted, straight-chain or branched divalent hydrocarbon group having from 2 to 20 carbon atoms, and R²⁵ and R²⁶ are each independently a hydrogen atom or a substituted or unsubstituted, straight-chain or branched monovalent hydrocarbon group having from 1 to 20 carbon atoms).

The definitions, types, and the like of the substituted or unsubstituted, straight-chain or branched monovalent hydrocarbon groups or divalent hydrocarbon groups are as described above.

On the other hand, the component (C1) may be an unsaturated aliphatic hydrocarbon. Examples of unsaturated aliphatic hydrocarbons include various dienes, diynes, enynes and similar products having two or more reactive unsaturated groups. In view of crosslinking, dienes, diynes, and enynes are preferable. Dienes, diynes, and enynes are compounds having a structure in which at least two reactive unsaturated groups are separated by one or more, and preferably two or more single bonds in a molecule. The unsaturated aliphatic hydrocarbon group may be present at the terminal of the molecular chain, or as a pendant group in the molecular chain.

Examples of unsaturated aliphatic hydrocarbons serving as the component (C1) include α,ω-unsaturated alkenes and alkynes having from 2 to 30 carbon atoms. Examples of the component (C1) include (C1-1) an α,ω-diene expressed by the general formula (2-1):
[Formula 30]

CH₂=CH(CH₂)ₓCH=CH₂ (2-1)

(wherein 1≤x≤20); (C1-2) an α,ω-diyne expressed by the general formula (2-2):
[Formula 31]

CH≡C(CH₂)ₓC≡CH (2-2)

(wherein 1≤x≤20); (C1-3) an α,ω-ene-yne expressed by the general formula (2-3):
[Formula 32]

CH₂=CH(CH₂)ₓC≡CH (2-3)

(wherein 1≤x≤20); and (C1-4) a bisalkenyl polyether compound expressed by the general formula (2-4):
[Formula 33]

CₘH₂ₘ₋₁O(CₙH₂ₙO)ᵥCₘH₂ₘ₋₁ (2-4)

(wherein 2≤m≤20, 2≤n≤4, y is the total value of the number of repetitions of the oxyethylene unit, the oxypropylene unit, and the oxybutylene unit, and 1≤y≤180).

Specific examples of unsaturated aliphatic hydrocarbons serving as the component (C1) include 1,4-pentadiene, 1,5-hexadiene, 1,6-heptadiene, 1,7-octadiene, 1,8-nonadiene, 1,9-decadiene, 1,11-dodecadiene, 1,13-tetradecadiene, 1,19-eicosadiene, 1,3-butadiene, 1,5-hexadiyne, and 1-hexene-5-yne.

The component (C1) may be a single component, but may also be a mixture of two or more components having different structures. That is, the component (C1) may be a mixture of one or more types of organopolysiloxanes and one or more types of unsaturated aliphatic hydrocarbons. Therefore, "having a number of reactive unsaturated groups greater than 1 on average" means having more than one reactive unsaturated group on average per molecule when two or more types of organopolysiloxanes and/or unsaturated aliphatic hydrocarbons are used.

The (C2) organic compound having at least one reactive unsaturated group and at least one epoxy group in the molecule serving as the component (C) is not structurally limited as long as the compound has a total of two or more - preferably from 2 to 10, more preferably from 2 to 7, even more preferably from 2 to 5, and particularly preferably from 2 to 4 - reactive unsaturated groups and epoxy groups in the molecule, and straight-chain, branched, or reticulated organic compounds can be used. An organopolysiloxane or an unsaturated aliphatic hydrocarbon is preferable as an organic compound. There are also no restrictions regarding the position of the organic compound, preferably, the organopolysiloxane, the unsaturated aliphatic hydrocarbon, or the aforementioned reactive unsaturated group, and the component may be positioned on the main chain or on a terminal. However, from the perspective of the ease of controlling the crosslinking density, it is preferable to use a compound of high purity in which the total of unsaturated groups and epoxy groups in the molecule is two.

A reactive unsaturated group is preferably present in an unsaturated aliphatic hydrocarbon group. Examples of unsaturated aliphatic hydrocarbon groups are as described above.

When the component (C2) is an organopolysiloxane, the unsaturated aliphatic hydrocarbon group containing a reactive unsaturated group and/or the epoxy group is preferably bonded to the silicon atom. In addition, when the component (C2) is an organopolysiloxane, the group bonding to silicon atoms other than the unsaturated aliphatic hydrocarbon or the epoxy group may be a substituted or unsubstituted monovalent hydrocarbon group or a monovalent organic group having a reactive functional group as described above.

The component (C2) is preferably an epoxy group-containing unsaturated aliphatic hydrocarbon having at least one epoxy group. Examples of the unsaturated aliphatic hydrocarbon include compounds having the unsaturated aliphatic hydrocarbon groups described above. A compound having a monovalent unsaturated aliphatic hydrocarbon group is preferable.

Examples of the component (C1) include: (C2-1) an unsaturated epoxy compound expressed by the general formula (2-6): 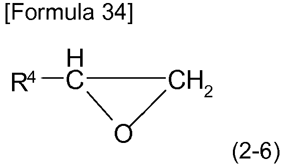 (wherein R⁴ is a substituted or unsubstituted, straight-chain or branched monovalent hydrocarbon group having one reactive unsaturated group and from 2 to 20 carbon atoms); and (C2-2) an unsaturated group-containing cycloaliphatic epoxy compound expressed by the general formula (2-7): 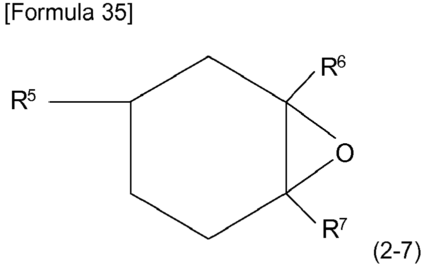 (wherein R⁵ is a substituted or unsubstituted, straight-chain or branched monovalent hydrocarbon group having one reactive unsaturated group and from 2 to 20 carbon atoms;
R⁶ is a hydrogen atom or a methyl group; and
R⁷ is a hydrogen atom or a methyl group). The definitions, types, and the like of the reactive unsaturated groups in general formulas above and the substituted or unsubstituted, straight-chain or branched monovalent hydrocarbon groups are as described above.

Specific epoxy group-containing unsaturated aliphatic hydrocarbons serving as the component (C2) include an allylglycidylether, methallylglycidylether, 1-methyl-4-isopropenylcyclohexene oxide, 1,4-dimethylcyclohexene oxide, 4-vinylcyclohexene oxide, vinylnorbornene monooxide, dicyclopentadiene monooxide, butadiene monooxide, 1,2-epoxy-5-hexene, 1,2-epoxy-9-decene, and 2,6-dimethyl-2,3-epoxy-7-octene. Among these, 4-vinyl cyclohexane oxide is preferable.

The component (C2) may be a single component, but may also be a mixture of two or more components having different structures.

The reaction for producing the organopolysiloxane elastomer of the present invention may be performed in the presence or in the absence of a reaction solvent in accordance with a known method. The reaction between the unsaturated group and the Si-H group in the present invention is a hydrosilylation reaction. In addition, when crosslinking is performed using an epoxide of (C2) the organic compound having one or more reactive unsaturated groups and one or more epoxy groups in the molecule, bonding caused by the reaction of the unsaturated group and the Si-H group and ether bond generation caused by the self ring-opening polymerization of the epoxy groups (cationic polymerization reaction that occurs in the presence of a SiH group and a platinum catalyst) both occur, resulting in crosslinking. In order to accelerate this reaction, irradiation using high energy beams such as ultraviolet light can be applied, or a common cation polymerization catalyst can be further added.

The reaction solvent is not particularly limited as long as the solvent is nonreactive, and examples thereof include alcohol-based solvents such as ethanol and isopropyl alcohol; aromatic hydrocarbon-based solvents such as toluene and xylene; ether-based solvents such as dioxane and THF; aliphatic hydrocarbon-based solvents such as n-hexane, cyclohexane, n-heptane, cycloheptane, and methylcyclohexane; and chlorinated hydrocarbon-based organic solvents such as carbon tetrachloride. An oil agent described below may also be used as a reaction solvent. When an oil agent is used as a reaction solvent, a composition consisting of an organopolysiloxane elastomer and an oil agent can be obtained directly after the crosslinking reaction, and a composition consisting of a particulate organopolysiloxane elastomer and an oil agent - preferably a paste-like composition - can be easily obtained by grinding the composition using a mechanical force.

The hydrosilylation reaction may be performed in the presence or absence of a catalyst, but preferably is performed in the presence of a catalyst because the reaction can be carried out at a low temperature and in a shorter period of time. Examples of the hydrosilylation reaction catalyst include platinum, ruthenium, rhodium, palladium, osmium, iridium, and similar compounds, and platinum compounds are particularly effective due to their high catalytic activity. Examples of the platinum compound include chloroplatinic acid; platinum metal; platinum metal supported on a carrier such as platinum supported on alumina, platinum supported on silica, platinum supported on carbon black, or the like; and a platinum complex such as platinum-vinylsiloxane complex, platinum-phosphine complex, platinum-phosphite complex, platinum alcoholate catalyst, or the like. A usage amount of the catalyst is about 0.5 to 1000 ppm in terms of platinum metal, when using a platinum catalyst.

A reaction temperature of the hydrosilylation reaction is typically from 30 to 150°C, and a reaction time is typically from 10 minutes to 24 hours and preferably from 1 to 10 hours.

The component (A) is crosslinked by the component (C) as a result of the hydrosilylation reaction or the cationic polymerization reaction of the epoxy groups, and the polysiloxane chains originating from the component (A) are linked by the crosslinking portion having a carbon-silicon bond originating from the component (C). The component (A) also comprises a sugar alcohol-modified group originating from the component (B). In this way, the organopolysiloxane elastomer of the present invention can be obtained.

Note that although the organopolysiloxane elastomer of the present invention typically has a structure that is linked by the crosslinking portion having a carbon-silicon bond originating from the component (C), it may also have a portion crosslinked by the Si-O-C bond. This is because when the structure has a condensation-reactable functional group such as a silanol group or an alkoxy group in the components (A) to (C), links can not only be formed between polysiloxane chains but can also be formed intermittently as a result of a partial reaction between the hydroxyl groups in the sugar alcohol-modified group originating from the component (B) and the Si-H groups of (A) when the crosslinking conditions are severe.

In the production of the organopolysiloxane elastomer of the present invention, the component (C) may be further reacted with the component (A) after the reaction between the component (A) and the component (B), or the component (B) can be further reacted with the component (A) after the reaction between the component (A) and the component (C).

When the component (C) is further reacted with the component (A) after the reaction between the component (A) and the component (B), the average value of the number of silicon-bonded hydrogen atoms per molecule of the component (A) reacting with the reactive unsaturated groups of the component (C) is preferably 1.5 or greater. That is, the number of silicon-bonded hydrogen atoms per molecule of the component (A) which constitute the crosslinking portion and react with the unsaturated bonds of the component (C) is, on average, at least 1.5, preferably in a range from 2.0 to 5.0, and particularly preferably in a range from 2.5 to 3.5.

Further, the organopolysiloxane elastomer may be subjected to hydrogenation treatment for the purpose of improving the odor after the reaction caused by the remaining unsaturated compound. Methods of hydrogenation treatment include a method using a pressurized hydrogen gas and a method using a hydrogenation agent such as a metal hydride, and hydrogenation treatment may also entail homogeneous reactions and heterogeneous reactions. One of these methods may be performed alone, or multiple methods may be performed in combination. However, taking into consideration the advantage that the catalyst that is used will not remain in the finished product, a heterogeneous catalytic hydrogenation reaction using a solid catalyst is most preferable.

Examples of solid catalysts (hydrogenation catalyst) that can be used include noble metal-based catalysts such as common platinum-based catalysts and palladium-based catalysts as well as nickel-based catalysts. More specific examples include single substances such as nickel, palladium, platinum, rhodium, and cobalt and catalysts combining a plurality of metals such as platinum-palladium, nickel-copper-chromium, nickel-copper-zinc, nickel-tungsten, and nickel-molybdenum. Examples of an optional catalyst carrier include activated carbon, silica, silica alumina, alumina, and zeolite. In addition, copper-containing hydrogenation catalysts such as Cu-Cr, Cu-Zn, Cu-Si, Cu-Fe-Al, and Cu-Zn-Ti may be used. The form of the hydrogenation catalyst differs depending on the type of the reaction vessel and therefore cannot be determined generally, but the form is typically selected appropriately from forms such as a powder, granules, or pellets. In addition, the platinum catalyst used in the synthesis process (hydrosilylation reaction) can be used directly. These hydrogenation catalysts may be used alone or as a combination of two or more types of catalysts.

The hydrogenation treatment can also be used to refine a crude product of the organopolysiloxane elastomer obtained by the hydrosilylation reaction described above. Specifically, a crude product can be refined by deodorization resulting from the hydrogenation treatment in a solvent or without a solvent in the presence of a hydrogenation catalyst, and such a refined product can be used much more preferably when applied to an external use preparation or a cosmetic composition in which the reduction of odor and compatibility with other components are desired. Moreover, the deodorizing treatment preferably has, as a pre-process or a post-process, a stripping treatment in which nitrogen gas is brought into contact with the crude organopolysiloxane elastomer or the hydrogenated product to remove light matter under reduced pressure.

In the production of the organopolysiloxane elastomer of the present invention, (Q) an organic compound having one reactive unsaturated group in the molecule (excluding the component (C2)) may be further reacted in addition to the components (A), (B), and (C). One type of the component (Q) may be used, or two or more types of the component (Q) may be used in combination. The reactions are preferably performed sequentially in the presence of a hydrosilylation reaction catalyst. The definitions, types, and the like of the reactive unsaturated groups in the component (Q) are as described above.

For example, when the component (C) is further reacted with the component (A) after the reaction between the component (A) and the component (B), the component (Q) may be reacted with the component (A) prior to the reaction between the component (A) and the component (B), the component (Q) may be reacted with the component (A) after the reaction between the component (A) and the component (B), or the component (Q) may be further reacted with the component (A) after the reaction of the component (C).

For example, when the component (B) is further reacted with the component (A) after the reaction between the component (A) and the component (C), the component (Q) may be reacted with the component (A) prior to the reaction between the component (A) and the component (C), the component (Q) may be reacted with the component (A) after the reaction between the component (A) and the component (C), or the component (Q) may be further reacted with the component (A) after the reaction of the component (B).

Examples of the component (Q) include (Q1) a siloxane dendron compound having one reactive unsaturated group in the molecule and (Q2) a hydrocarbon compound having one reactive unsaturated group in the molecule, a chain organopolysiloxane having one reactive unsaturated group in the molecule, or the like.

Preferable examples of (Q1) the siloxane dendron compound having one reactive unsaturated group in the molecule include compounds having a siloxane dendron structure that have one carbon-carbon double bond at a molecular terminal, the compounds being expressed by the following general formula (3'): 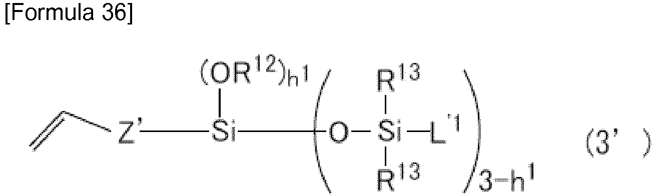 (wherein
R¹² and R¹³ are synonymous with those described above;
Z' is a divalent organic group;
h¹ is a number in a range from 0 to 3;
L^{'1} is the R¹³ moiety or, when j=1, a silylalkyl group expressed by the general formula (3") below: 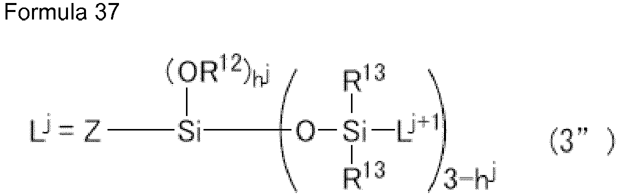 (wherein R¹² and R¹³ are synonymous with those described above;
Z is a divalent organic group;
j indicates the number of generations of the silylalkyl group represented by L^{j}, when the number of generations (the number of repetitions) of the silylalkyl group is k', j is an integer of 1 to k', and the number of generations k' is an integer from 1 to 9; L^{j+1} is the silylalkyl group when j is less than k' and is the R¹³ moiety when j = k'; and h^{j} is a number in a range from 0 to 3). The divalent organic groups in the general formulas (3') and (3") are as described above.

Preferable examples of (Q2) the hydrocarbon compound having one reactive unsaturated group in the molecule or the chain organopolysiloxane having one reactive unsaturated group in the molecule include monounsaturated hydrocarbon compounds expressed by the following general formula:
Formula 38

R'-R^{2'}

(wherein R' is synonymous with that described above; and
R2' is a substituted or unsubstituted, straight or branched monovalent hydrocarbon group having from 7 to 58 carbon atoms, or the chain organosiloxane group represented by the following general formula (4-1): 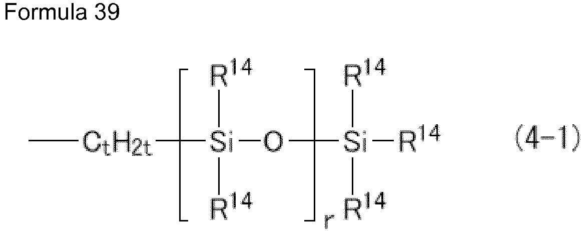 (wherein R¹⁴, t and r are synonymous with those described above); or the following general formula (4-2): 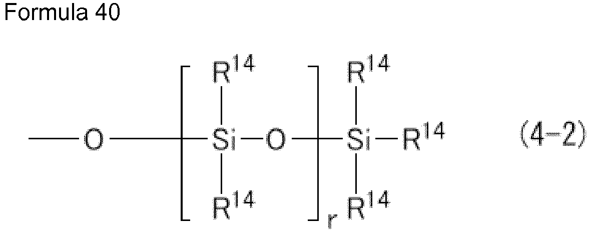 (wherein R¹⁴ and r are as defined above).

The hydrocarbon compound having one reactive unsaturated group in the molecule (Q2) is preferably a monounsaturated hydrocarbons having from 9 to 30 carbon atoms and is more preferably a 1-alkene. Examples of the 1-alkene include 1-nonene, 1-decene, 1-undecene, 1-dodecene, 1-tridecene, 1-tetradecene, and the like. Examples of the chain organopolysiloxane having one reactive unsaturated group in the molecule include a dimethylpolysiloxane capped at one molecular terminal with a vinyl group, a methylphenylpolysiloxane capped at one molecular terminal with a vinyl group, and the like.

In the production of the organopolysiloxane elastomer of the present invention, it is preferable to further perform an acidizing process of treating the organopolysiloxane elastomer of the present invention obtained by the hydrosilylation reaction of the component (A), the component (B), the component (C), and the optional component (C) using at least one type of acidic substance. As a result, the odor of the organopolysiloxane elastomer can be reduced.

Odor attenuating treating may also be performed with a method of performing an acidization process at the stage prior to the formation of the organopolysiloxane elastomer (prior to crosslinking), but since acid, water, and alcoholic hydroxyl groups are present, the Si-H groups are consumed by the dehydrogenation reaction, which makes it difficult to perform subsequent crosslinking as designed, even if odor reduction is achieved. Accordingly, it is more preferable to perform acidization after forming the elastomer.

The acidic substance is not particularly limited and may be any acid that matches the definition of a Lewis-acid, a Bronsted acid, or an Arrhenius acid. The acidic substance used in the present invention is preferably a water soluble acid. Therefore, the acidic substance used in the present invention is preferably an Arrheinius acid which releases protons in an aqueous solution. One type of the acidic substance may be used alone, or two or more types of acidic substances may be used in combination. In the present invention, by using such an acidic substance, the organopolysiloxane elastomer can be essentially deodorized and the generation of odor over time can be completely suppressed without severing the carbon-oxygen bonds or the silicon-oxygen bonds.

The acidic substance can be selected from the group consisting of inorganic acids, organic acids, acidic inorganic salts, solid acids, and acidic platinum catalysts.

The inorganic acid is not particularly limited, and examples thereof include hydrochloric acid, sulfuric acid, nitric acid, phosphoric acid, carbonic acid, boric acid, sulfonic acid, sulfinic acid, and the like. Note that substances containing organic groups such as benzene sulfonic acid are not preferable as inorganic acids.

The organic acid is not particularly limited, and can be a monocarboxylic acid (including monohydroxy monocarboxylic acid and dihydroxy monocarboxylic acid), a dicarboxylic acid (including monohydroxy dicarboxylic acid and dihydroxy dicarboxylic acid), a polycarboxylic acid, or the like. Examples thereof include:
Straight saturated aliphatic monocarboxylic acids (alkanoic acids) such as formic acid, acetic acid, trifluoroacetic acid, propionic acid, butyric acid, valeric acid, caproic acid, enanthic acid, caprylic acid, pelargonic acid, capric acid, undecanoic acid, and the like;
Branched saturated aliphatic monocarboxylic acids (alkanoic acids) such as 2-methylpropanoic acid, 2-methylbutanoic acid, trimethylpropanoic acid, 2-methylpentanoic acid, trimethyl acetic acid, and the like;
Unsaturated aliphatic monocarboxylic acids (alkenoic acids) such as acrylic acid, methacrylic acid, crotonic acid, isocrotonic acid, vinyl acetic acid, allyl acetic acid, hexenoic acid, heptenoic acid, octenoic acid, and the like;
Unsaturated aliphatic monocarboxylic acids (alkynoic acids) such as propiolic acid, tetrolic acid, allyl acetic acid, hexynoic acid, octynoic acid, and the like;
Polyunsaturated aliphatic monocarboxylic acids such as pentadienoic acid, sorbic acid, and the like;
α-hydroxymonocarboxylic acids such as citric acid, lactic acid, glycolic acid, α-oxybutyric acid, and the like;
β-hydroxymonocarboxylic acids such as 2-hydroxyvaleric acid, 2-hydroxycaproic acid, β-oxybutyric acid, and the like;
γ-hydroxymonocarboxylic acids such as γ-oxybutyric acid and the like;
Dihydroxymonocarboxylic acids such as glyceric acid and the like;
Other hydroxymonocarboxylic acids such as hydroxy(meth)acrylic acid and the like;
Saturated aliphatic dicarboxylic acids such as oxalic acid, malonic acid, succinic acid, glutaric acid, adipic acid, and the like;
Monohydroxy saturated aliphatic dicarboxylic acids such as tartronic acid, malic acid, and the like; Dihydroxy saturated aliphatic dicarboxylic acids such as tartaric acid and the like;
Unsaturated aliphatic dicarboxylic acids such as maleic acid, fumaric acid, and the like; Aromatic monocarboxylic acid such as benzoic acid and the like;
Aromatic dicarboxylic acids such as phthalic acid and the like;
Amino acids such as glycine, alanine, valine, leucine, glutamic acid, aspartic acid, PL-pyrrolidone carboxylic acid, and the like; and
Polycarboxylic acids such as gallic acid and the like.

In addition, alkyl sulfuric acid, alkyl phosphoric acid, phenol, and the like can be used as the organic acid. Note that higher fatty acids or salts thereof are not preferable as organic acids.

The acidic inorganic salt is not limited, but a water soluble salt is preferable. Particularly preferable is a water soluble acidic inorganic salt that is solid at 25°C and, when 50 g thereof is dissolved in 1 L of ion exchanged water, the solution has a pH at 25°C of not more than 4, preferably not more than 3.5, and more preferably not more than 2.0. When the acidic inorganic salt is solid at room temperature (25°C), the acidic inorganic salt can be easily removed by filtration as necessary. In addition, when the acidic inorganic salt is water soluble, the acidic inorganic salt can be easily rinsed off with water as necessary. Note that the pH values in the present invention are values measured using a pH meter having a glass electrode in a sample aqueous solution at room temperature (25°C).

Examples that can be used as the acidic inorganic salt include acidic inorganic salts in which at least a monovalent hydrogen atom of the inorganic acid that is at least divalent is neutralized by a base. Examples of the inorganic acid that is at least divalent include sulfuric acid, sulfurous acid, and the like. Examples of the base include an alkali metal, ammonia, and the like.

More specifically, the acidic inorganic salt is preferably at least one type of acidic inorganic salt comprising a hydrogensulfate ion (HSO₄⁻) or a hydrogensulfite ion (HSO₃⁻) and a monovalent cation (M⁺). Examples of the monovalent cation (M⁺) include alkali metal ions or an ammonium ion. Particularly, the monovalent cation is preferably at least one type selected from the group consisting of a sodium ion, a potassium ion, and an ammonium ion.

Specific examples of the acidic inorganic salt include lithium hydrogensulfate, sodium hydrogensulfate, potassium hydrogensulfate, rubidium hydrogensulfate, cesium hydrogensulfate, ammonium hydrogensulfate, sodium hydrogensulfite, or hydrates thereof, and Lewis-acids such as AlCl₃, FeCl₃, TiCl₄, and BF₃-Et₂O. The pH values of several aqueous solutions in which 50 g of acidic inorganic acid is dissolved in 1 L of ion exchanged water are as shown in the tables below. From the perspective of the technical benefit of reducing odor, the water soluble acidic inorganic salt having a pH of not higher than 2.0 is most preferably at least one type of acidic inorganic salt selected from the group consisting of sodium hydrogensulfate, potassium hydrogensulfate, and ammonium hydrogensulfate. [Table 1]

**Table 1**

| Acidic inorganic salt | pH (50g/L) |
|---|---|
| Sodium hydrogensulfate | 1.5 or lower |
| Potassium hydrogensulfate | 2.0 or lower |
| Ammonium hydrogensulfate | 1.5 or lower |
| Sodium hydrogensulfite | 3.5 |

Examples of solid acids that can be used include acidic solid substances such as activated clay, acidic clay, solid acidic zirconium dioxide, strong acidic cation exchange resins, fluorinated sulfonic acid resins, alumina, silica alumina, and zeolite. Of these, a solid acidic zirconium dioxide is preferable. Examples of the solid acidic zirconium dioxide include products prepared at a temperature of at least 300°C after treating zirconium hydroxide with sulfuric acid; more specifically, aluminum hydroxides or hydrous oxides, zirconium hydroxides or hydrous oxides, a solid acidic zirconium - specifically zirconia sulfate - prepared by first obtaining a molded product by mixing and molding a sulfuric acid-containing compound and then baking the molded product at a temperature at which a tetragonal-structured zirconia is formed - specifically at a temperature of at least 300°C. A commercially available product of the solid zirconium dioxide is SZA-60 (manufactured by Japan Energy Corporation). The strong acidic cation exchange resin is, for example, a cation exchange resin in which a functional group is a sulfonic acid group (-SO₃H), and examples of commercially available products thereof include Amberlyst 15, Amberlyst 16, Amberlyst 31, and Amberlyst 35 (manufactured by Organo Corporation). The fluorinated sulfonic acid resin is a perfluorinated polymer having a suspended sulfonic acid group bonded to the polymer chain, and specific examples thereof include the product described in Japanese Examined Patent Application Publication No. S59-4446 and the like.

Examples of the acidic platinum catalyst include chloroplatinic acids, alcohol-modified chloroplatinic acids, olefin complexes of chloroplatinic acids, ketone complexes of chloroplatinic acids, vinylsiloxane complexes of chloroplatinic acids, and platinum tetrachlorides. Of these, chloroplatinic acid is preferable.

The acidizing process described above can be performed by bringing the organopolysiloxane elastomer into contact with the acidic substance in a desired manner.

Specifically, the acidizing process can be performed by means of operations such as adding at least one type of the acidic substance described above and, optionally, an organic solvent such as water or alcohol to a reaction system containing the organopolysiloxane elastomer (for example, in a reaction vessel such as a flask, a kneading/grinding container, an emulsifier, or the like), mixing by stirring, kneading, and grinding or repeating these operations. Alternatively, the process can be more advantageously performed by first grinding the organopolysiloxane elastomer or a composition containing the elastomer and an oil agent in advance, adding at least one type of acidic substance and, optionally, an organic solvent such as water or alcohol, and performing an operation such as stirring while heating.

In particular, it is preferable to add at least one type of the acidic substance and water to a reaction system containing the organopolysiloxane elastomer and to then agitate or knead and pulverize the mixture using a mechanical force while heating. In addition, this treatment is preferably performed in the presence of a solvent such as a lower monohydric alcohol. The acid treatment can be carried out at any temperature and treatment time, and can be carried out at a temperature from 0 to 200°C and more preferably from 50 to 100°C for a reaction time of from 0.5 to 24 hours and more preferably from about 1 to 10 hours. The amount of the acidic substance that is used can be appropriately selected in accordance with the acid strength, the treatment apparatus, the treatment time, and the treatment temperature. However, when the acidic substance is one with moderate acid strength such as sodium hydrogensulfate, potassium hydrogensulfate, ammonium hydrogensulfate, citric acid, glycolic acid, or phosphoric acid, the content is preferably in a range from 10 to 500 ppm and more preferably in a range from 20 to 200 ppm in the organopolysiloxane elastomer of the present invention. In addition, when the acidic substance is an acidic substance with higher acid strength such as hydrochloric acid or sulfuric acid, the content is preferably in a range from 0.1 to 50 ppm in the organopolysiloxane elastomer, and when the acidic substance is a substance with weak acid strength or a solid acid exemplified by activated clay, acidic clay, solid acidic zirconium dioxide, strong acidic cation exchange resin, fluorinated sulfonic acid resin, zeolite, or the like, the content is preferably in a range from 500 to 10,000 ppm in the organopolysiloxane elastomer.

In the production method for an organopolysiloxane elastomer of the present invention, a process of heating and/or decompressing the composition (stripping process) is preferably included after the acidizing process. Low-boiling-point components, which are odor-causing substances, can be removed (stripped) by the heating and/or decompression described above. In addition, a large amount of the odor-causing substances can be removed by performing the acidizing process once again after stripping. At this time, when the acidic substance remains in the reaction system, it is unnecessary to newly add an acidic substance, which yields the advantage that it is sufficient to add only water. That is, the acidizing process and the stripping process can be respectively repeated two or more times for the purpose of increasing the degree of reduction in odor.

The "low-boiling-point components" removed by the stripping process include, in addition to carbonyl compounds such as propionaldehyde, which are thought to be odor-causing substances, volatile compounds such as the reaction solvent used in the synthesis and the like of the organopolysiloxane elastomer.

Note that the stripping process may be performed before the acidizing process.

Known reaction conditions may be used in the stripping method, but stripping under normal pressure or under reduced pressure is preferable, and stripping is preferably performed at a temperature of 120°C or lower. In order to effectively perform the stripping, the stripping is preferably performed under reduced pressure or, for example, performed under a nitrogen gas or similar inert gas stream. A specific example of the low-boiling-point component removal operation is one in which the organopolysiloxane elastomer or composition thereof or a hydrogenated product thereof comprising a low boiling point component is placed in a flask having a refluxing cooler, a nitrogen injection port, or the like. While nitrogen gas is supplied, the internal pressure is reduced and the temperature is increased, and the pressure and temperature are then kept constant so as to remove light matter. Here, typically, a pressure reduction parameter is from 0.1 to 10.0 kPa, a heating temperature is from 50 to 170°C, and a treatment time is from 10 minutes to 24 hours.

In the present invention, after the acidizing process, a basic substance may be used to neutralize the organopolysiloxane elastomer. One type of the basic substance may be used alone, or two or more types of substances may be used in combination. Examples of the basic substance include organic bases such as sodium hydroxide, potassium hydroxide, calcium hydroxide, barium hydroxide, ammonia water, and sodium hydrogen carbonate; basic buffers such as trisodium phosphate, tripotassium phosphate, trisodium citrate, and sodium acetate; and organic bases such as basic amino acids, amines and pyridines. An amount of the basic substance is preferably an amount needed to neutralize a reaction system comprising the organopolysiloxane elastomer but, as necessary, the amount of the basic substance may be adjusted to an amount by which weak acidity or weak alkalinity is obtained.

The organopolysiloxane elastomer of the present invention is preferably in a particulate form and is more preferably in the form of solid particles.

The particulate organopolysiloxane can be simply obtained by grinding the cured organopolysiloxane elastomer using a mechanical force, and solid particles of the organopolysiloxane elastomer with a desired particle size can be obtained by adjusting the grinding conditions using a publicly known method. Regarding grinding, after the organopolysiloxane elastomer prior to or following primary grinding is mixed with an oil agent, the organopolysiloxane elastomer in a state in which the elastomer is swelled or dispersed in the oil agent is subjected to primary grinding or finer secondary grinding using a mechanical force.

The mechanical means for grinding the organopolysiloxane elastomer or the composition is not particularly limited, but the substance is preferably ground by at least one method selected from the group consisting of shearing, kneading, and a means of passing the substance through an orifice under pressure.

On the other hand, it is also possible to obtain an aqueous dispersion of organopolysiloxane elastomer particles by emulsifying/dispersing the raw material composition of the organopolysiloxane elastomer prior to curing in water and then performing a crosslinking reaction and to obtain dried organopolysiloxane elastomer particles by removing the water from the aqueous dispersion.

The particle size of the organopolysiloxane elastomer particles can be selected in accordance with the application or tactile sensation and is not particularly limited, but from the perspective of the preparation of a composition with the oil agent described below, the volume average particle size measured by microscopic observation or using a particle size distribution measurement device is preferably in a range from 20 to 1000 µm and more preferably in a range from 25 to 300 µm.

### (Composition containing an organopolysiloxane elastomer)

The present invention relates to a composition comprising the organopolysiloxane elastomer described above. The compounded amount of the organopolysiloxane elastomer in the composition is not particularly limited but can be set in a range from 1 to 99 wt.% (mass%), for example, preferably from 5 to 95 wt.% (mass%), more preferably from 10 to 90 wt.% (mass%), even more preferably from 20 to 80 wt.% (mass%), and even more preferably from 30 to 70 wt.% (mass%) based on the total weight (mass) of the composition.

### The composition of the present invention may contain at least one type of oil agent in addition to the organopolysiloxane elastomer. The oil agent is not particularly limited, and a solid, semi-solid, or liquid oil agent may be used. Specific examples of oil agent include one type or two or more types of oil agent selected from the group consisting of include silicone oils, hydrocarbon oils, ester oils, vegetable oils and fats, animal oils and fats, fatty acids, higher alcohols, triglycerides, artificial sebums, and fluorine-based oil agents.

Specific examples of the silicone oil include straight organopolysiloxanes expressed by the following general formula (7), cyclic organopolysiloxanes expressed by the general formula (8), and branched organopolysiloxanes expressed by the general formula (9). 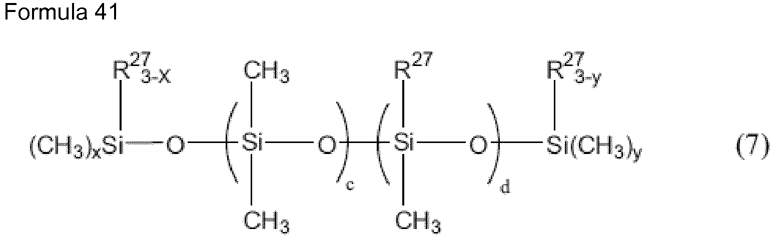 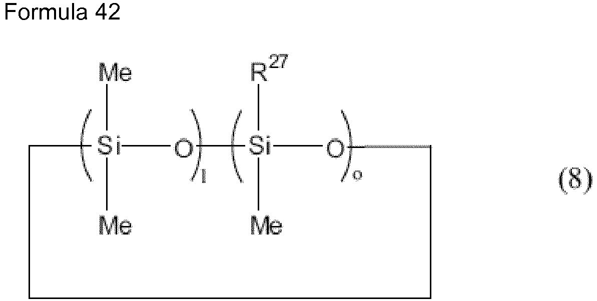 Formula 43

R²⁷₄₋ₛSi(OSiMe₃)ₛ (9)

In the formulas (7) to (9) above, R²⁷ is a hydrogen atom, hydroxyl group or a group selected from a monovalent unsubstituted or fluorine substituted alkyl group having from 2 to 30 carbon atoms, an aryl group, an amino substituted alkyl group, an alkoxy group, and a group expressed by (CH₃)₃SiO[(CH₃)₂SiO]ᵤSi(CH₃)₂CH₂CH₂-. Specific examples thereof include saturated aliphatic hydrocarbon groups such as ethyl groups, propyl groups, butyl groups, pentyl groups, hexyl groups, heptyl groups, octyl groups, decyl groups, and dodecyl groups; unsaturated aliphatic hydrocarbon groups such as vinyl groups, allyl groups, and hexenyl groups; saturated cycloaliphatic hydrocarbon groups such as cyclopentyl groups and cyclohexyl groups; aromatic hydrocarbon groups such as phenyl groups, tolyl groups, and naphthyl groups; and groups in which the hydrogen atoms bonded to the carbon atoms of these groups are substituted partially by an organic group having a halogen atom, an epoxy group, a carboxyl group, an amino group, a methacryl group, a mercapto group, or the like or a group substituted by a trimethylsiloxy group bonded via a divalent hydrocarbon group and/or a chain polydimethyl siloxane bond. Here, c is an integer from 0 to 1,000; d is an integer from 0 to 1,000; c+d is an integer from 1 to 2,000; f and g are each independently 0, 1, 2, or 3; l and o are each independently an integer from 0 to 8, provided that 3≤l+o≤8; s is an integer from 1 to 4.

Examples of silicone oils having the structure described above include cyclic organopolysiloxanes such as hexamethyl cyclotrisiloxane (D3), octamethyl cyclotetrasiloxane (D4), decamethyl cyclopentasiloxane (D5), dodecamethyl-cyclohexasiloxane (D6), 1,1-diethylhexamethyl cyclotetrasiloxane, phenylheptamethyl cyclotetrasiloxane, 1,1-diphenylhexamethyl cyclotetrasiloxane, 1,3,5,7-tetravinyltetramethyl cyclotetrasiloxane, 1,3,5,7-tetramethyl cyclotetrasiloxane, 1,3,5,7-tetracyclohexyltetramethyl cyclotetrasiloxane, tris(3,3,3-trifluoropropyl) trimethylcyclotrisiloxane, 1,3,5,7-tetra(3-methacryloxypropyl) tetramethyl cyclotetrasiloxane, 1,3,5,7-tetra(3-acryloxypropyl) tetramethyl cyclotetrasiloxane, 1,3,5,7-tetra(3-carboxypropyl) tetramethyl cyclotetrasiloxane, 1,3,5,7-tetra(3-vinyloxypropyl) tetramethyl cyclotetrasiloxane, 1,3,5,7-tetra(p-vinylphenyl) tetramethyl cyclotetrasiloxane, 1,3,5,7-tetra[3-(p-vinylphenyl) propyl] tetramethyl cyclotetrasiloxane, 1,3,5,7-tetra(N-acryloyl-N-methyl-3-aminopropyl) tetramethyl cyclotetrasiloxane, 1,3,5,7-tetra(N,N-bis (lauroyl)-3-aminopropyl) tetramethyl cyclotetrasiloxane, and the like. Examples of straight organopolysiloxanes include dimethylpolysiloxane in which both molecular terminals are capped with trimethylsiloxy groups (dimethylsilicone with a low viscosity such as 2 cst or 6 cst to dimethylsilicone with a high viscosity such as 1,000,000 cst), organohydrogenpolysiloxane, methylphenylpolysiloxane in which both molecular terminals are capped with trimethylsiloxy groups, a copolymer of methylphenylsiloxane and dimethylsiloxane in which both molecular terminals are capped with trimethylsiloxy groups, diphenylpolysiloxane in which both molecular terminals are capped with trimethylsiloxy groups, a copolymer of diphenylsiloxane and dimethylsiloxane in which both molecular terminals are capped with trimethylsiloxy groups, trimethylpentaphenyltrisiloxane, phenyl (trimethylsiloxy) siloxane, methylalkylpolysiloxane in which both molecular terminals are capped with trimethylsiloxy groups, a copolymer of methylalkylsiloxane and dimethylpolysiloxane in which both molecular terminals are capped with trimethylsiloxy groups, a copolymer of methyl (3,3,3-trifluoropropyl) siloxane and dimethylsiloxane in which both molecular terminals are capped with trimethylsiloxy groups, α,ω-dihydroxypolydimethylsiloxane, α,ω-diethoxypolydimethylsiloxane, 1,1,1,3,5,5,5-heptamethyl-3-octyltrisiloxane, 1,1,1,3,5,5,5-heptamethyl-3-dodecyltrisiloxane, 1,1,1,3,5,5,5-heptamethyl-3-hexadecyltrisiloxane, tristrimethylsiloxymethylsilane, tristrimethylsiloxyalkylsilane, tetrakistrimethylsiloxysilane, tetramethyl-1,3-dihydroxydisiloxane, octamethyl-1,7-dihydroxytetrasiloxane, hexamethyl-1,5-diethoxytrisiloxane, hexamethyldisiloxane, octamethyltrisiloxane, a higher alkoxy-modified silicone, a higher fatty acid-modified silicone, a carbinol-modified silicone (hydrocarbyl functional siloxane), long-chain alkyl-modified silicone, amino-modified silicone, amide-modified silicone, quaternary ammonium salt-modified silicone, and the like. The organopolysiloxane elastomer of the present invention is able to stably maintain various powders dispersed in an oil phase containing these silicone oils and to stably emulsify/disperse an aqueous phase in an oil phase containing these silicone oils.

Examples of the hydrocarbon oil include liquid paraffin, light liquid isoparaffin, heavy liquid isoparaffin, vaseline, n-paraffin, isoparaffin, isododecane, isohexadecane, polyisobutylene, hydrogenated polyisobutylene, polybutene, ozokerite, ceresin, microcrystalline wax, paraffin wax, polyethylene wax, polyethylene/polypropylene wax, squalane, squalene, pristane, polyisoprene, wax, and the like. The organopolysiloxane elastomer of the present invention is able to stably maintain various powders dispersed in an oil phase containing these hydrocarbon oils and to stably emulsify/disperse an aqueous phase in an oil phase containing these hydrocarbon oils.

Examples of the ester oil include hexyldecyl octanoate, cetyl octanoate, isopropyl myristate, isopropyl palmitate, butyl stearate, hexyl laurate, myristyl myristate, oleyl oleate, decyl oleate, octyldodecyl myristate, hexyldecyl dimethyloctanoate, cetyl lactate, myristyl lactate, diethyl phthalate, dibutyl phthalate, lanolin acetate, ethylene glycol monostearate, propylene glycol monostearate, propylene glycol dioleate, glyceryl monostearate, glyceryl monooleate, glyceryl tri(2-ethylhexanoate), trimethylolpropane tri(2-ethylhexanoate), ditrimethylolpropane triethylhexanoate, ditrimethylolpropane isostearate/sebacate, trimethylolpropane trioctanoate, trimethylolpropane triisostearate, diisopropyl adipate, diisobutyl adipate, 2-hexyldecyl adipate, di-2-heptylundecyl adipate, diisostearyl malate, hydrogenated castor oil monoisostearate, N-alkylglycol monoisostearate, octyldodecyl isostearate, isopropyl isostearate, isocetyl isostearate, ethylene glycol di-2-ethylhexanoate, cetyl 2-ethylhexanoate, pentaerythritol tetra-2-ethylhexanoate, octyldodecyl gum ester, ethyl oleate, octyldodecyl oleate, neopentylglycol dicaprate, triethyl citrate, 2-ethylhexyl succinate, dioctyl succinate, isocetyl stearate, diisopropyl sebacate, di-2-ethylhexyl sebacate, diethyl sebacate, dioctyl sebacate, dibutyloctyl sebacate, cetyl palmitate, octyldodecyl palmitate, octyl palmitate, 2-ethylhexyl palmitate, 2-hexyldecyl palmitate, 2-heptylundecyl palmitate, cholesteryl 12-hydroxystearate, dipentaerythritol fatty acid ester, 2-hexyldecyl myristate, ethyl laurate, 2-octyldodecylester N-lauroyl-L-glutamate, di(cholesteryl/behenyl/octyldodecyl) N-lauroyl-L-glutamate, di(cholesteryl/octyldodecyl) N-lauroyl-L-glutamate, di(phytosteryl/behenyl/octyldodecyl) N-lauroyl-L-glutamate, di(phytosteryl/octyldodecyl) N-lauroyl-L-glutamate, isopropyl N-lauroylsarcosinate, diisostearyl malate, neopentylglycol dioctanoate, isodecyl neopentanoate, isotridecyl neopentanoate, isostearyl neopentanoate, isononyl isononanoate, isotridecyl isononanoate, octyl isononanoate, isotridecyl isononanoate, diethylpentanediol dineopentanoate, methylpentanediol dineopentanoate, octyldodecyl neodecanoate, 2-butyl-2-ethyl-1,3-propanediol dioctanoate, pentaerythrityl tetraoctanoate, pentaerythrityl hydrogenated rosin, pentaerythrityl triethylhexanoate, dipentaerythrityl (hydroxystearate/stearate/rosinate), polyglyceryl tetraisostearate, polyglyceryl-10 nonaisostearate, polyglyceryl-8 deca(erucate/isostearate/ricinoleate), (hexyldecanoic acid/sebacic acid) diglyceryl oligoester, glycol distearate (ethylene glycol distearate), diisopropyl dimer dilinoleate, diisostearyl dimer dilinoleate, di(isostearyl/phytosteryl) dimer dilinoleate, (phytosteryl/behenyl) dimer dilinoleate, (phytosteryl/isostearyl/cetyl/stearyl/behenyl) dimer dilinoleate, dimer dilinoleyl dimer dilinoleate, dimer dilinoleyl diisostearate, dimer dilinoleyl hydrogenated rosin condensate, dimer dilinoleic acid hydrogenated castor oil, hydroxyalkyl dimer dilinoleyl ether, glyceryl triisooctanoate, glyceryl triisostearate, glyceryl trimyristate, glyceryl triisopalmitate, glyceryl trioctanoate, glyceryl trioleate, glyceryl diisostearate, glyceryl tri(caprylate/caprate), glyceryl tri(caprylate/caprate/myristate/stearate), hydrogenated rosin triglyceride (hydrogenated ester gum), rosin triglyceride (ester gum), glyceryl behenate eicosane dioate, glyceryl di-2-heptylundecanoate, diglyceryl myristate isostearate, cholesteryl acetate, cholesteryl nonanoate, cholesteryl stearate, cholesteryl isostearate, cholesteryl oleate, cholesteryl 12-hydroxystearate, cholesteryl ester of macadamia nut oil fatty acid, phytosteryl ester of macadamia nut oil fatty acid, phytosteryl isostearate, cholesteryl ester of soft lanolin fatty acid, cholesteryl ester of hard lanolin fatty acid, cholesteryl ester of long-chain branched fatty acid, cholesteryl ester of long-chain α-hydroxy fatty acid, octyldodecyl ricinoleate, octyldodecyl ester of lanolin fatty acid, octyldodecyl erucate, isostearic acid hydrogenated castor oil, ethyl ester of avocado fatty acid, isopropyl ester of lanolin fatty acid, and the like. The organopolysiloxane elastomer of the present invention is able to stably maintain various powders dispersed in an oil phase containing these ester oils and to stably emulsify/disperse an aqueous phase in an oil phase containing these ester oils.

Examples of natural animal or vegetable oils and fats and semi-synthetic oils and fats include oils and fats such as avocado oil, linseed oil, almond oil, ibota wax, perilla oil, olive oil, cacao butter, kapok wax, kaya oil, carnauba wax, liver oil, candelilla wax, beef tallow, neatsfoot oil, beef bone fat, hydrogenated beef tallow, apricot kernel oil, spermaceti wax, hydrogenated oil, wheat germ oil, sesame oil, rice germ oil, rice bran oil, sugar cane wax, sasanqua oil, safflower oil, shea butter, Chinese tung oil, cinnamon oil, jojoba wax, olive squalane, shellac wax, turtle oil, soybean oil, tea seed oil, camellia oil, evening primrose oil, corn oil, lard, rapeseed oil, Japanese tung oil, rice bran wax, germ oil, horse fat, persic oil, palm oil, palm kernel oil, castor oil, hydrogenated castor oil, castor oil fatty acid methyl ester, sunflower oil, grape oil, bayberry wax, jojoba oil, hydrogenated jojoba ester, macadamia nut oil, beeswax, mink oil, cottonseed oil, cotton wax, Japanese wax, Japanese wax kernel oil, montan wax, coconut oil, hydrogenated coconut oil, tri-coconut oil fatty acid glyceride, mutton tallow, peanut oil, lanolin, liquid lanolin, reduced lanolin, lanolin alcohol, hard lanolin, lanolin acetate, lanolin fatty acid isopropyl ester, POE lanolin alcohol ether, POE lanolin alcohol acetate, lanolin fatty acid polyethylene glycol, POE hydrogenated lanolin alcohol ether, egg yolk oil, and the like. Herein, "POE" means "polyoxyethylene".

Examples of higher fatty acids include lauric acid, myristic acid, palmitic acid, stearic acid, behenic acid, undecylenic acid, oleic acid, linolic acid, linolenic acid, arachidonic acid, eicosapentaenoic acid (EPA), docosahexaenoic acid (DHA), isostearic acid, 12-hydroxystearic acid, and the like.

Examples of higher alcohols include lauryl alcohol, myristyl alcohol, palmityl alcohol, stearyl alcohol, behenyl alcohol, hexadecyl alcohol, oleyl alcohol, isostearyl alcohol, hexyldodecanol, octyldodecanol, cetostearyl alcohol, 2-decyltetradecinol, cholesterol, sitosterol, phytosterol, lanosterol, POE cholesterol ether, monostearyl glycerol ether (batyl alcohol), monooleyl glycerol ether (selachyl alcohol), and the like.

Examples of the fluorine-based oil agent include perfluoropolyether, perfluorodecalin, perfluorooctane, and the like, and one or two or more types of these oil agents can be used as necessary.

The compounded amount of the oil agent in the composition of the present invention is not particularly limited but can be set in a range from 0.1 to 95 wt.% (mass%), for example, preferably from 1 to 90 wt.% (mass%), more preferably from 2 to 80 wt.% (mass%), even more preferably from 3 to 70 wt.% (mass%), and even more preferably from 5 to 60 wt.% (mass%) based on the total weight (mass) of the composition.

The composition of the present invention containing at least one type of oil agent in addition to the organopolysiloxane elastomer may be in the form of a paste.

The organopolysiloxane elastomer compounded in the composition of the present invention is preferably in a particulate form. The mixing ratio with the oil agent (weight (mass) ratio) is arbitrary, but from the perspective of obtaining a paste-like composition consisting of uniform, fine particles without a foreign body sensation, the ratio is preferably in a range from 5/95 to 95/5, particularly preferably from 20/80 to 80/20, and most preferably from 25/75 to 50/50. In particular, when the organopolysiloxane elastomer can be swelled with an oil agent in an amount equal to or greater than the weight (mass) of the elastomer itself, a composition which is swelled using an oil agent in an amount equal to or greater than the weight of the elastomer itself - preferably a paste-like composition - can be easily prepared.

The composition containing the particulate organopolysiloxane elastomer and an oil agent can be obtained by mixing the organopolysiloxane elastomer with an oil agent after grinding the elastomer using a mechanical force or grinding a mixture of the organopolysiloxane elastomer and an oil agent using a mechanical force.

The composition of the present invention can contain water. The compounded amount of the water in the composition of the present invention is not particularly limited but can be set in a range from 1 to 90 wt.% (mass%), for example, preferably from 5 to 80 wt.% (mass%), more preferably from 10 to 70 wt.% (mass%), even more preferably from 20 to 60 wt.% (mass%), and even more preferably from 30 to 50 wt.% (mass%) based on the total weight (mass) of the composition.

In particular, since the organopolysiloxane elastomer of the present invention has a hydrophobic silicone chain and a hydrophilic sugar alcohol-modified group, the organopolysiloxane elastomer functions as a surfactant or an emulsifier. That is, by mixing the organopolysiloxane elastomer of the present invention and water (or water and a hydrophilic medium), each component can be uniformly dispersed, so the present invention can be advantageously used as a hydrous composition.

The hydrous composition described above is a composition containing the organopolysiloxane elastomer of the present invention and water and can be in the form of a hydrous gel composition or an emulsion composition. The emulsion form is not particularly limited and can be a water-based-oil-based oil composition such as an oil-in-water emulsion or a water-in-oil emulsion; or an arbitrary form of emulsion such as an oil-in-alcohol (polyol, for example) emulsion or an alcohol(polyol, for example)-in-oil emulsion. In particular, a water-in-oil emulsion composition or an alcohol(polyol, for example)-in-oil emulsion is preferable.

The average particle size of the emulsion particle formed by emulsification using the organopolysiloxane elastomer of the present invention can be measured by a conventional measurement device using a laser diffraction/scattering method or the like. The emulsion composition according to the present invention is preferably a polar solvent-in-oil emulsion but may also be an oil-in-polar solvent emulsion. In addition, the emulsion composition of the present invention may be a transparent micro-emulsion in which the measured average particle size is not more than 0.1 µm or may be a large particulate white turbid emulsion in which the average particle size is more than 10.0 µm. Furthermore, the emulsion particles may be micronized for the purpose of improving the stability and transparency of the appearance of the emulsion. An emulsion having a particle diameter from 0.5 to 20 µm can be selected for the purpose of improving sensation during use and adhesion characteristics to hair and skin.

The emulsion and the like described above may be produced by blending the organopolysiloxane elastomer of the present invention or composition comprising the same and water by means of a mechanical force using an apparatus such as a homomixer, a paddle mixer, a Henschel mixer, a homo-disper, a colloid mill, a propeller stirrer, a homogenizer, an in-line continuous emulsifier, an ultrasonic emulsifier, or a vacuum kneader. In addition, in the production method of the emulsion composition, the content and compounding ratio of the water is as described above and is preferably appropriately selected within a range from 1 to 99 wt.% (mass%) of the entire emulsion composition in accordance with the form and use of the emulsion.

The composition of the present invention can contain at least one type of alcohol. The alcohol preferably has water miscibility and more preferably is a lower alcohol or a polyhydric alcohol.

Examples of lower alcohols include ethanol, isopropanol, n-propanol, t-butanol, s-butanol, and the like. Examples of polyhydric alcohols include divalent alcohols such as 1,3-butylene glycol, 1,2-butylene glycol, propylene glycol, trimethylene glycol, tetramethylene glycol, 2,3-butylene glycol, pentamethylene glycol, 2-buten-1,4-diol, dibutylene glycol, pentyl glycol, hexylene glycol, octylene glycol, and the like; trivalent alcohols such as glycerol, trimethylol propane, 1,2,6-hexanetriol, and the like; polyhydric alcohols having 4 or more valences such as pentaerythritol, xylitol, and the like; and sugar alcohols such as sorbitol, mannitol, maltitol, maltotriose, sucrose, erythritol, glucose, fructose, a starch-decomposed product, maltose, xylitose, starch-decomposed sugar-reduced alcohol, and the like. Furthermore, examples other than these low-molecule polyhydric alcohols include polyhydric alcohol polymers such as diethylene glycol, dipropylene glycol, triethylene glycol, polypropylene glycol, tetraethylene glycol, diglycerol, polyethylene glycol, triglycerol, tetraglycerol, polyglycerol, and the like.

The compounded amount of the alcohol in the composition of the present invention is not particularly limited but can be set in a range from 0.1 to 50 wt.% (mass%), for example, preferably from 1 to 40 wt.% (mass%), more preferably from 2 to 30 wt.% (mass%), even more preferably from 3 to 20 wt.% (mass%), and even more preferably from 4 to 10 wt.% (mass%) based on the total weight (mass) of the composition.

### (Carbonyl value measurement method)

The degree of odor of the organopolysiloxane elastomer of the present invention or the composition containing the same can be determined by the carbonyl value measured from the absorbance of a reaction solution obtained by reacting the organopolysiloxane elastomer or the composition containing the same and 2,4-dinitrophenylhydrazine (2,4-DNPH) in a reaction medium containing at least one type of monovalent lower alcohol having from 1 to 4 carbon atoms. Furthermore, in addition to a compound having a carbonyl group such as an aldehyde or a ketone, the "carbonyl compound" also includes a potential carbonyl compound such as an acetal, propenyl ether, or a similar compound that does not comprise a carbonyl group but generates a carbonyl group by decomposing under certain conditions.

Accordingly, in order to assay the degree of odor of the organopolysiloxane elastomer of the present invention or the composition containing the same, it is possible to measure the carbonyl value of the organopolysiloxane elastomer or the composition containing the same from the absorbance of a reaction solution obtained by reacting the organopolysiloxane elastomer containing a carbonyl compound or the composition containing the same and 2,4-dinitrophenylhydrazine in a reaction medium containing at least one type of monovalent lower alcohol having from 1 to 4 carbon atoms.

The carbonyl value measured with the method described above for the organopolysiloxane elastomer of the present invention, which is a sugar alcohol-modified silicone, or the composition containing the same is preferably not more than 4.0 Abs/g, more preferably not more than 3.0 Abs/g, and even more preferably not more than 2.0 Abs/g.

In the measurement method described above, carbonyl value of an organopolysiloxane elastomer or a composition comprising the same is determined based on the absorbance of the reaction solution obtained by reacting 2,4-DNPH and a carbonyl compound in the organopolysiloxane elastomer or the composition comprising the same, and from this carbonyl value, it is possible to measure the total amount of carbonyl (calculated on a propanal basis) in the organopolysiloxane elastomer or in the composition by using a pre-plotted calibration curve.

The "carbonyl value" is the carbonyl content index value, and is a value obtained by converting the absorbance (absorbance at 430 nm or 460 nm) of the reaction solution, obtained by reacting 2,4-DNPH with the sample, to per 1 g of sample.

Measurement of the carbonyl value uses the property of hydrazone, produced by reacting a carbonyl and 2,4-DNPH in the presence of an acid, that the hydrazone becomes quinoid ions in a base and gives color. The carbonyl value is determined based on the absorbance, which indicates the degree of coloring at 430 nm (a maximum wavelength attributable to saturated carbonyl exists in the vicinity thereof) and at 460 nm (a maximum wavelength attributable to unsaturated carbonyl exists in the vicinity thereof).

The "total amount of carbonyl" is the total amount of carbonyl compound for an organopolysiloxane elastomer or a composition comprising the same. The concentration of carbonyl compounds (total amount of carbonyl) of various samples (an organopolysiloxane elastomer or a composition comprising the same) can be measured by obtaining a calibration curve by measuring carbonyl values of standard samples having known concentrations of the carbonyl compound (concentration of propionaldehyde).

In the measurement method described above, at least a monovalent lower alcohol having from 1 to 4 carbon atoms is used as the reaction solvent in the reaction between a carbonyl compound and 2,4-DNPH, but it is preferable to use water at the same time.

By using water together with a monovalent lower alcohol having from 1 to 4 carbon atoms as the reaction solvent, it is possible to reliably determine the carbonyl value with high precision, even for a sample containing an aldehyde condensation product (potential carbonyl compound) such as an acetal. Thus, it is possible to quantitate the total amount of carbonyls, while taking into consideration carbonyl compounds attributable to these odor-causing substances. Although the reason is unclear, it is inferred that, because water is present in the reaction system, an aldehyde condensation product is decomposed, and the reaction with 2,4-DNPH is reliably performed. In the reaction solvent comprising a monovalent lower alcohol having from 1 to 4 carbon atoms and water, the mixing ratio (mass ratio) of the monovalent lower alcohol having from 1 to 4 carbon atoms to water is preferably from 99.9:0.1 to 50:50, and more preferably from 99:1 to 75:25.

In the measurement method described above, "reaction solvent" indicates a solvent that is present in the reaction system containing 2,4-DNPH and a carbonyl compound in a sample. A reaction solvent is formed by using, in addition to (a) a solvent used to prepare a sample solution, (b) a solvent used to prepare the added acid solution, (c) a solvent used to prepare a solution of 2,4-DNPH, and the like.

Each of (a) an alcohol that forms a sample solution, (b) an alcohol that forms an acid solution, and (c) an alcohol that forms a 2,4-DNPH solution need not be a monovalent lower alcohol having from 1 to 4 carbon atoms, provided that the alcohol in the reaction solvent resulting from mixture thereof contains a monovalent lower alcohol having from 1 to 4 carbon atoms.

In the measurement method described above, it is preferable to use an alcohol as the solvent (hereinafter, also referred to as "diluent solvent") added to make the volume of a reaction solution to be a prescribed amount when measuring the absorbance of a reaction solution, and it is preferable to use a monovalent lower alcohol having from 1 to 4 carbon atoms. Furthermore, all of the diluent solvent need not be an alcohol. Water and/or an organic solvent (organic solvent not having a carbonyl group in its structure, and having low toxicity) can be used as a part of the diluent solvent.

The monovalent lower alcohol having from 1 to 4 carbon atoms is preferably a saturated alcohol. Examples thereof include methanol, ethanol, n-propanol, i-propanol, n-butanol, i-butanol, and sec-butanol. Of these, n-butanol is preferable. As the monovalent lower alcohol having from 1 to 4 carbon atoms, one type can be used independently, or two or more types can be mixed and used.

These alcohols lack toxicity (toxicity such as the toxicity that benzene has), and the alcohols are capable of dissolving various substances having different polarities and molecular weights. Consequently, by using these alcohols as the reaction solvent and diluent solvent, it is possible to safely and easily perform each operation for determining the carbonyl value.

As a monovalent lower alcohol having from 1 to 4 carbon atoms, it is preferable to use a monovalent lower alcohol having a total aldehyde/ketone content of not more than 3 ppm, preferably not more than 2 ppm, and more preferably not more than 1 ppm (hereinafter, also referred to as "ultrapure alcohol"). When the total aldehyde/ketone content exceeds 3 ppm, the baseline increases when measuring the absorbance for the resulting reaction solution, and it may not be possible to obtain proper (highly precise) index values due to the absorbance measured for a reaction solution containing a sample with a low carbonyl content being smaller than the absorbance measured for a solution for a blank test (solution not containing the sample). By using an ultrapure alcohol having a total aldehyde/ketone content of not more than 3 ppm as a reaction solvent, it is possible to properly and easily determine the carbonyl value for a sample with a low carbonyl content (for example, a carbonyl value of less than 3).

As a method of preparing (refining) an ultrapure alcohol, a method that distills an alcohol under normal pressure or under reduced pressure after adding proper quantities of 2,4-DNPH and an acid lacking oxidative action (e.g., hydrochloric acid or trichloroacetic acid) to the alcohol to be refined and heating and stirring this system over several hours, can be used. Furthermore, it is preferable to perform these refining treatments within 24 hours before measuring the absorbance of the reaction solution.

Additionally, as the ultrapure alcohol, it is preferable to use a commercially available highly pure reagent that has been refined so that a total amount of aldehydes and ketones is 3 ppm or less. Examples of commercially available highly pure reagents that can be used as an ultrapure alcohol include ethanol (99.8%) Infinity Pure; ethanol (99.8%) for precise analysis; ethanol (99.5%) for high-performance liquid chromatography; ethanol (99.5%) for spectrometry; 2-propanol (99.9%) Infinity Pure, 2-propanol (99.9%) for precise analysis; 2-propanol (99.5%) for high-performance liquid chromatography; 2-propanol (99.5%) for spectrometry; 1-propanol (99.8%) Infinity Pure, 1-propanol (99.5%) for high-performance liquid chromatography; methanol (99.8%) Infinity Pure, methanol (99.8%) for precise analysis; methanol (99.5%) for high-performance liquid chromatography; methanol (99.5%) for spectrometry, n-butyl alcohol for high-performance liquid chromatography; n-butyl alcohol for spectrometry (all manufactured by Wako Pure Chemical Industries, Ltd.), and the like.

Furthermore, even for such highly pure reagents, the total amount of aldehydes and ketones increases over time, and sometimes exceeds 3 ppm. In addition, the total amount of aldehydes and ketones exceeds 3 ppm in a relatively short time period after opening (e.g. within 24 hours). Therefore, from the perspective of satisfying the essential requirement for ultrapure alcohols (that is, the total aldehyde/ketone content is not more than 3 ppm), the following commercially available highly pure reagents are preferable:
(a) a reagent produced within 6 months before use, and
(b) a reagent opened within 24 hours before use.

In the measurement method described above, the solvent used as the reaction solvent need not be composed solely of a monovalent lower alcohol having from 1 to 4 carbon atoms, or a mixed solvent of water and a monovalent lower alcohol having from 1 to 4 carbon atoms. A low toxicity organic solvent also may be used as a part of the reaction solvent as long as it does not have a carbonyl group in the structure thereof. However, when an organic solvent other than a monovalent lower alcohol having from 1 to 4 carbon atoms is used as a part of the reaction solvent, the total amount of aldehydes and ketones contained in the reaction solvent (total excluding water), which is formed by mixing the organic solvent (part) and a monovalent lower alcohol having from 1 to 4 carbon atoms (remaining part), is preferably not more than 3 ppm.

In the measurement method described above, a basic reaction solution (the reaction solution for absorbance measurement) can be prepared by adding an acid and 2,4-DNPH to a sample solution prepared by uniformly dispersing a sample in a solvent, reacting the carbonyl compound in the sample and 2,4-DNPH by heat treating this system, adding an alkali in the system after cooling, and then adjusting the volume of to a prescribed volume with a diluent solvent. Here, it is preferable to use a volumetric flask with a volume of 10 to 100 mL as the container for preparing a basic reaction solution.

### (1) Sample solution

The solvent used to prepare the sample solution also constitutes the reaction solvent as is, so such a solvent is preferably a mixed solvent of an ultrapure alcohol and water. The mass of the sample solution (sample and solvent) used to measure absorbance is normally about 2 to 6 g, and preferably about 5 g. The mass of the sample contained in a sample solution differs according to the carbonyl content of the sample (i.e., the carbonyl value) and the prepared quantity of reaction solution for absorbance measurement (i.e., the volume of the used volumetric flask). However, when, for example, a 50 mL volumetric flask is used to prepare a reaction solution (i.e., the reaction solution for absorbance measurement), the mass of the sample contained in a sample solution is preferably from 5 to 250 mg, and more preferably from 10 to 150 mg.

### (2) Acids

Examples of acids added to the sample solution include mineral acids such as dilute sulfuric acid, hydrochloric acid, dilute nitric acid, phosphoric acid, and the like; organic acids such as trichloroacetic acid, trifluoroacetic acid, formic acid, acetic acid, sulfonic acid, phenolic acid, and the like; and Lewis acids such as AlCl₃, FeCl₃, TiCl₄, and the like; and the like. These acids can be used alone or in combinations of two or more. Of these, from the standpoint of accurately quantitating the total amount of carbonyl in a highly refined organopolysiloxane elastomer or a composition comprising the same, trichloroacetic acid, dilute sulfuric acid (particularly, the dilute sulfuric acid with a concentration of 20% or less), and hydrochloric acid (particularly, the hydrochloric acid with a concentration of 37% or less) are preferable. In addition, the acid used in the measurement method described above is preferably an acid with the highest purity (reagent-grade or higher purity).

These acids may be added as is to a sample solution. However, from the perspective of performing accurate measurement or the like, it is preferable to add the acids in a solution state obtained by dissolving in an appropriate solvent. Furthermore, the solvent used to prepare an acid solution also constitutes the reaction solvent as is, thus, as such the solvent, it is preferable to use a monovalent lower alcohol having from 1 to 4 carbon atoms or a mixed solvent of monovalent lower alcohol having from 1 to 4 carbon atoms and water. When preparing a reaction solution (a reaction solution that contains from 5 to 250 mg of sample) in a 50 mL volumetric flask, the added amount of acid is preferably from 0.03 to 5.0 g.

### (3) 2,4-DNPH

The 2,4-DNPH added to the sample solutions is preferably a 2,4-DNPH having a purity of at least a reagent-grade and containing an equal amount of water. Also, the purity can be further raised by recrystallization or a similar refining operation. 2,4-DNPH can be added as is to a sample solution. However, from the standpoint of performing an accurate measurement, it is preferable to add the 2,4-DNPH in a solution state obtained by dissolving in an appropriate solvent. Furthermore, the solvent used to prepare the 2,4-DNPH solution also constitutes the reaction solvent as is. Thus, as such the solvent, it is preferable to use a monovalent lower alcohol having from 1 to 4 carbon atoms, or a mixed solvent of water and a monovalent lower alcohol having from 1 to 4 carbon atoms. When preparing a reaction solution (reaction solution that contains from 5 to 250 mg of sample) in a 50 mL volumetric flask, it is preferable to add 0.5 to 100 mg of 2,4-DNPH.

### (4) Heat treatment

A condition for the heat treatment of a mixed solution containing a sample, acid, and 2,4-DNPH is 20 to 180 minutes at 30 to 120°C (however, the temperature is lower than the boiling point of the reaction solvent). At a treatment temperature below 30°C, it takes a long time to react the 2,4-DNPH and the carbonyl compound in a sample, which is inefficient. On the other hand, when heating at a temperature above 120°C, there is a risk that the generated hydrazone will decompose. When the treatment time is less than 20 minutes, it becomes difficult to complete the reaction with 2,4-DNPH. On the other hand, if the treatment time exceeds 180 minutes, there is a risk that the generated hydrazone will decompose.

### (5) Alkali

It is preferable to use an inorganic strong base such as potassium hydroxide, sodium hydroxide, and the like as the alkali added to the reaction solution resulting from the reaction between the 2,4-DNPH and carbonyl compound in a sample. These alkalis can be added as is to a sample solution. However, from the perspective of performing accurate measurement or the like, it is preferable to add these alkalis in a solution state obtained by dissolving in an appropriate solvent. As such solvents, one or two or more solvents can be selected from among solvents that can dissolve an alkali, do not have a carbonyl group in the structure thereof, are compatible with the solvent used as the reaction solvent, and that have low toxicity. Specific examples thereof include monovalent saturated lower alcohols such as methanol, ethanol, 2-propanol, 1-propanol, 1-butanol, 2-butanol, and the like, or mixed solvents prepared by mixing a proper quantity of water and/or other organic solvent (the organic solvent without a carbonyl group in their structure and having low toxicity) in these solvents. It is preferable to use an ultrapure alcohol or a mixed solvent of water and ultrapure alcohol. When a reaction solution (reaction solution that contains from 5 to 250 mg of sample) is prepared in a 50 mL volumetric flask, the added amount of alkali is preferably from 0.05 to 5.0 g.

### (6) Diluent solvent

The reaction solution to which alkali was added is adjusted to a specific volume (for example, 50 mL) with a diluent solvent having an alcohol as a main constituent. The alcohol forming the diluent solvent is preferably an ultrapure alcohol.

### (7) Specific preparation method

An example of a method of preparing a reaction solution for absorbance measurement is as follows. In a 50 mL volumetric flask, 5 g of a sample solution is placed, prepared by uniformly dispersing from 5 to 250 mg of a sample in a mixed solvent of water and a monovalent lower alcohol having from 1 to 4 carbon atoms. Next, a solution prepared by dissolving from 0.03 to 5.0 g of acid in a monovalent lower alcohol having from 1 to 4 carbon atoms and a solution prepared by dissolving from 0.5 to 500 mg of 2,4-DNPH in a monovalent lower alcohol having from 1 to 4 carbon atoms are added to the flask. This solution is then mixed well and homogenized, and heat treatment is performed for 20 to 180 minutes at 30 to 120°C, thereby reacting the 2,4-DNPH and the carbonyl compound in the sample, while the volumetric flask is capped. After this mixture is cooled to room temperature, a solution prepared by dissolving from 0.05 to 5.0 g of an alkali in an alcohol is added. A diluent solvent composed of an alcohol is then added, mixed well, and homogenized to adjust the volume of the mixture to 50 mL.

In the measurement method described above, the absorbance at 430 nm or 460 nm is measured for the basic reaction solution obtained as described above. Here, when the carbonyl compound contained in a sample is estimated to be mainly a saturated carbonyl compound, the absorbance at 430 nm is measured for the reaction solution, and when the carbonyl compound contained in a sample is estimated to be mainly an unsaturated carbonyl compound, the absorbance at 460 nm is measured for the reaction solution. When absorbance is measured, it is preferable that the absorbance cell that contains the reaction solution is made of quartz. Also, the length (thickness) of the liquid layer specified by the absorption cell is preferably 1 cm.

It is preferable to measure the absorbance within 10 to 20 minutes after the addition of an alkali to the reaction solution resulting from the reaction of the 2,4-DNPH and the carbonyl compound in a sample. Absorbance measured before 10 minutes after the addition of an alkali sometimes lacks stability. Also, more than 20 minutes after the addition of an alkali, the absorbance tends to drop as a result of the discoloration of the reaction solution. Based on experience with various samples, if the absorbance is measured 15 minutes after the addition of an alkali, the value with best reproducibility is obtained.

In the measurement method described above, the carbonyl value of a sample (an organopolysiloxane elastomer or a composition comprising the same) is determined based on the absorbance measured as described above. It is possible to measure the total amount of carbonyl in the sample, by using a premeasured calibration curve based on this carbonyl value. Here, the calibration curve is obtained by measuring, according to the method (carbonyl value measurement method) described above, the carbonyl values of a plurality of standard samples whose total amount of carbonyl (propionaldehyde concentration) is known.

For example, a calibration curve is obtained by measuring the carbonyl values of standard samples, for which total amounts of carbonyl (propionaldehyde concentrations) are known, by a method that determines the carbonyl value (CV) by substituting the absorbance (A₁) and the absorbance (A₂), which are measured by the following processes (1) to (9), into the formula CV = (A₁ - -A₂)/B (where B is the mass (g) of the sample contained in 5.000 g of sample solution). The same method as aforementioned, which was adopted to obtain this calibration curve, is used to measure the carbonyl value of a sample (an organopolysiloxane elastomer or a composition comprising the same) for which the total amount of carbonyl is unknown. Based on this carbonyl value and the calibration curve, it is possible to measure the total amount of carbonyl in the sample. Furthermore, it is preferable that the solvent described below for use in the following processes (1) and (9) contains an ultrapure alcohol and water, and that the solvent described below for use in the following process (7) contains an ultrapure alcohol.

[Processes]
(1) Process of preparing a sample solution by uniformly dispersing a sample in a solvent
(2) Process of adding 3 mL of an alcohol solution of 4.3% (wt/vol) trichloroacetic acid to 5.000 g of the sample solution and homogenizing by mixing well
(3) Process of adding 5 mL of an alcohol solution of 2,4-DNPH ([0.025% (wt/vol)] to the mixed solution obtained in the aforementioned process (2) and homogenizing by mixing well
(4) Process that reacts the carbonyl compound in a sample and 2,4-DNPH by heating the mixed solution obtained in the aforementioned process (3) for 30 minutes at 60°C
(5) Process that lets stand the reaction solution obtained in the aforementioned process (4) for 30 to 70 minutes at room temperature
(6) Process of adding 10 mL of an alcohol solution of potassium hydroxide [4.0% (wt/vol)] to the reaction solution after being left to stand in the aforementioned process (5) and homogenizing by mixing well
(7) Process of preparing a reaction solution with a total volume of 50 mL by adding a solvent to the reaction solution and homogenizing by mixing well, 5 to 10 minutes after the aforementioned process (6)
(8) Process that measures the absorbance (A₁) at 430 nm or 460 nm, of the reaction solution obtained in the aforementioned process (7), 10 to 20 minutes after the aforementioned process (6)
(9) Process that, as a blank measurement, measures the absorbance (A₂) at 430 nm or 460 nm of a solution obtained by using 5.000 g of solvent instead of using the sample solution and performing the same operations as those in the aforementioned processes (2) to (7)

Furthermore, either the calibration curve measurements (measurements of carbonyl value of standard samples) or the measurements of carbonyl values of unknown samples can be performed before the other measurements.

Next, each process will be described below. Furthermore, the processes (2) to (7) for the preparation of the reaction solution are normally performed using a 50 mL volumetric flask.

Process (1) is a process of preparing a sample solution by uniformly dispersing a sample in a solvent containing a monovalent lower alcohol (and preferably, water) having from 1 to 4 carbon atoms. The proportion of the sample in a sample solution is modified in accordance with the carbonyl value estimated for the sample. For example, the following settings are preferable: if the carbonyl value is estimated to be less than 6 in the sample, from 2 to 3 wt.% (mass%) (from 100 to 150 mg per 5.000 g of sample solution); if the carbonyl value is estimated to be in a range from 6 to 15 in the sample, from 0.8 to 2 wt.% (mass%) (from 40 to 100 mg per 5.000 g of sample solution); if the carbonyl value is estimated to be in a range from 15 to 30 in the sample, from 0.4 to 0.8 wt.% (mass%) (from 20 to 40 mg per 5.000 g of sample solution); if the carbonyl value is estimated to be in a range from 30 to 60 in the sample, from 0.2 to 0.4 wt.% (mass%) (from 10 to 20 mg per 5.000 g of sample solution); and if the carbonyl value is estimated to be greater than 60 in the sample, less than 0.2 wt.% (mass%) (less than 10 mg per 5.000 g of sample solution).

Additionally, when preparing a sample solution, it is preferable to dilute the sample stepwise. For example, as a method of preparing 5.000 g of a 2 wt.% (mass%) sample solution, 25.00 g of an 8 wt.% (mass%) dispersion is first prepared by uniformly dispersing 2.00 g of a sample in 21.00 g of a monovalent lower alcohol having from 1 to 4 carbon atoms and 2.00 g of water. Next, 1.250 g of the 8 wt.% (mass%) dispersion and 3.750 g of a monovalent lower alcohol having from 1 to 4 carbon atoms are accurately added to a 50 mL volumetric flask and mixed well to dilute the solution four-fold.

Process (2) is a process of adding 3 mL of an alcohol solution of 4.3% (wt/vol) of trichloroacetic acid to 5.000 g (sample: 0.100 g) of the 2 wt.% (mass%) sample obtained in the aforementioned process (1) using a volumetric pipette or the like and homogenizing the solution by mixing well. The solvent (a monovalent lower alcohol having from 1 to 4 carbon atoms) of this alcohol solution is preferably an ultrapure alcohol. It is preferable to prepare the alcohol solution of trichloroacetic acid by opening a bottle containing 100 mL of ultrapure alcohol, directly adding 4.3 g of trichloroacetic acid to this bottle, covering the bottle, and then shaking the bottle to uniformly mix the contents in the bottle. Also, alcohol solution of trichloroacetic acid is preferably prepared within 24 hours before measuring the absorbance of the reaction solution.

Process (3) is a process of adding 5 mL of an alcohol solution [0.025% (wt/vol)] of 2,4-DNPH to the mixed solution obtained in the aforementioned process (2) using a volumetric pipette or the like and homogenizing the solution by mixing well. The solvent (a monovalent lower alcohol having from 1 to 4 carbon atoms) of this alcohol solution of 2,4-DNPH is preferably an ultrapure alcohol. The alcohol solution of 2,4-DNPH is preferably prepared by opening a bottle containing 100 mL of ultrapure alcohol, directly adding 50 mg of 2,4-DNPH (reagent-grade product containing an equal amount of water) to this bottle, covering the bottle, and then putting the bottle into an ultrasonic bath for about 5 minutes to completely dissolve the 2,4-DNPH in the bottle. In addition, the alcohol solution of 2,4-DNPH is preferably prepared within 24 hours before measuring the absorbance of the reaction solution. It is preferable to add more water to hydrolyze the precursors of the carbonyl compound of acetal and the like present in the sample, to detect the precursors as carbonyl compounds.

In process (4), the mixed solution obtained in the aforementioned process (3) is heated for 30 minutes at 60°C to react the 2,4-DNPH and the carbonyls in the sample, thereby yielding a reaction solution containing hydrazone.

In process (5), the reaction solution obtained in the process (4) is cooled by letting the reaction solution stand for 30 to 70 minutes at room temperature.

In process (6), 10 mL of alcohol solution of potassium hydroxide (4.0% (wt/vol)) was added and mixed to the reaction solution after letting it stand, using a volumetric pipette or the like. As a result, the reaction solution exhibits basicity, and the generated hydrazone becomes quinoid ions and gives color. The solvent (a monovalent lower alcohol having from 1 to 4 carbon atoms) of this alcohol solution of potassium hydroxide is preferably an ultrapure alcohol. The alcohol solution of potassium hydroxide is preferably prepared by opening a bottle containing 100 mL of ultrapure alcohol, directly adding 4.0 g of potassium hydroxide (pellet shaped reagent-grade product) to this bottle, and covering the bottle, and after shaking until the pellets disappear, this bottle is placed in an ultrasonic bath for about 5 to 10 minutes, to completely dissolve the potassium hydroxide in the bottle. Also, it is preferable to prepare the alcohol solution of potassium hydroxide within 24 hours before measuring the absorbance of the reaction solution.

Process (7) is a process of preparing a reaction solution (basic reaction solution) with a total volume of 50 mL by adding a diluent solvent consisting of a monovalent lower alcohol having from 1 to 4 carbon atoms (preferably, an ultrapure alcohol) to the reaction solution and homogenizing the solution by mixing well, 5 to 10 minutes after the aforementioned process (6).

In process (8), for the reaction solution obtained in the aforementioned process (7), the absorbance (A₁) is measured at 430 nm (when the carbonyl in the sample is estimated to be mainly a saturated carbonyl) or at 460 nm (when the carbonyl in the sample is estimated to be mainly an unsaturated carbonyl). The absorbance must be measured 10 to 20 minutes after addition of the alcohol solution of potassium hydroxide of the aforementioned process (6), and it is most preferable to measure the absorbance 15 minutes after the addition of the alcohol solution of potassium hydroxide.

Process (9) is a process of measuring the absorption (A₂) at 430 nm and 460 nm for a solution obtained by performing the same operations as in the aforementioned processes (2) to (7) (addition of an alcohol solution of trichloroacetic acid; addition of an alcohol solution of 2,4-DNPH; heating and cooling of the obtained mixed solution; addition of an alcohol solution of potassium hydroxide; and addition of a diluent solvent consisting of a monovalent lower alcohol having from 1 to 4 carbon atoms) using 5.000 g of a monovalent lower alcohol having from 1 to 4 carbon atoms (preferably, a mixed solution with monovalent lower alcohol water having from 1 to 4 carbon atoms) instead of the sample solution described above as a blank test.

It is possible to determine the carbonyl value (CV) by substituting the absorbance (A₁) obtained by the aforementioned processes (1) to (8) and the absorbance (A₂) obtained by the aforementioned process (9), respectively, into the formula: CV = (A₁ - A₂)/B.) In the above formula, B is the mass (g) of the sample contained in 5.000 g of sample solution, and in a 2 wt.% (mass%) sample solution, B is 0.1 (5.000 × 0.02).

The organopolysiloxane elastomer of the present invention or the composition containing the same has, in essence, a small tendency to deteriorate due to oxidation caused by the oxygen in the air. Thus, it is not necessary to add a phenol, a hydroquinone, a benzoquinone, an aromatic amine, a vitamin, or similar antioxidant in order to prevent oxidation deterioration; or take steps to increase oxidation stability. However, adding such an antioxidant, for example, BHT(2,6-di-t-butyl-p-cresol), vitamin C, vitamin E, or the like, will result in a further increase in stability. In this case, an added amount of the antioxidant that is used is in a range (by weight (mass)) from 10 to 1,000 ppm, and preferably from 50 to 500 ppm, of the organopolysiloxane elastomer.

(Raw material for external use preparation and raw material for cosmetic composition) The organopolysiloxane elastomer of the present invention or the composition comprising the same can be advantageously used as a raw material for an external use preparation and a raw material for a cosmetic composition for use on a human body. In particular, a paste-like composition containing the particulate organopolysiloxane elastomer of the present invention and an oil agent can be used directly as a raw material for an external use preparation and a raw material for a cosmetic composition.

A proportion of the organopolysiloxane elastomer or the composition comprising the same in the raw material for an external use preparation and the raw material for a cosmetic composition is preferably from 50 to 100 wt.% (mass%), more preferably from 80 to 100 wt.% (mass%), and even more preferably from 90 to 100 wt.% (mass%) based on the total weight (mass) of the raw material. A proportion of the raw material compounded in the external use preparation or the cosmetic composition is not particularly limited but, for example, can be from 0.1 to 90 wt.% (mass%), and is preferably from 1 to 80 wt.% (mass%), more preferably from 2 to 70 wt.% (mass%), and even more preferably from 5 to 50 wt.% (mass%) based on the total weight (mass) of the external use preparation or the cosmetic composition.

Examples of the raw material for an external use preparation and the raw material for a cosmetic composition of the present invention include a thickening agent, a gelling agent, a tactile sensation improver, a surfactant, an emulsifier, and a powder dispersion stabilizer.

### External use preparation and cosmetic composition

The organopolysiloxane elastomer of the present invention or the composition comprising the same, or the raw material for use in an external use preparation and a cosmetic composition comprising the organopolysiloxane elastomer or the composition comprising the same, can be advantageously compounded in an external use preparation or a cosmetic composition and can constitute the external use preparation or the cosmetic composition of the present invention. The external use preparation or the cosmetic composition of the present invention is preferably stored in a container formed from a thermoplastic material or a container formed from a non-thermoplastic material. In addition, at least one compartment can be defined in the container so as to constitute a cosmetic product unit or external use preparation unit consisting of the container and the cosmetic composition or the external use preparation according to the present invention. The external use preparation or the cosmetic composition of the present invention can be primarily applied to and used on keratinous substances such as skin or hair as a non-therapeutic beauty technique for the purpose of doing applying cosmetics (makeup) or performing care (that is, dry skin care).

The external use preparation is a product to be applied to human skin, nails, hair, and the like and, for example, medicament active components can be compounded therein and used in the treatment of various disorders. The cosmetic composition is also a product to be applied to human skin, nails, hair, and the like, and is used for beauty purposes. The external use preparation or the cosmetic composition is preferably a skin external use preparation or a skin cosmetic composition, skin care cosmetic composition, sun care cosmetic composition, antiperspirant, foundation, color cosmetic, or a hair external use preparation or a hair cosmetic composition product.

The skin external use preparation or the skin cosmetic composition product of the present invention comprises the organopolysiloxane elastomer of the present invention or the composition comprising the same and, although the form thereof is not particularly limited, the product may be in the form of a solution, cream-like, solid, semi-solid, paste-like, gel-like, powder-like, multi-layer, mousse-like, or spray-like form. Specific examples of the skin external use preparation or the skin cosmetic composition product according to the present invention include skin lotions, emulsions, creams, sunscreen emulsions, sunscreen creams, hand creams, cleansing compositions, massage lotions, cleansing agents, anti-perspirants, deodorants, and similar basic cosmetic products; foundations, make-up bases, blushers, rouges, eye shadows, eye liners, mascaras, nail enamels, and similar make-up cosmetic products; and the like.

Similarly, the hair external use preparation or the hair cosmetic composition product according to the present invention comprises the organopolysiloxane elastomer of the present invention or the composition comprising the same and can be used in various forms. For example, the hair external use preparation or the hair cosmetic composition product according to the present invention may be dissolved or dispersed in an alcohol, a hydrocarbon, a volatile cyclic silicone, or the like and used, or the product may also be used in the form of an emulsion by dispersing the organopolysiloxane elastomer of the present invention and a desired emulsifier in water. Additionally, the hair external use preparation or the hair cosmetic composition product according to the present invention can be used as a spray by using propane, butane, trichloromonofluoromethane, dichlorodifluoromethane, dichlorotetrafluoroethane, carbonic acid gas, nitrogen gas, or a similar propellant. Examples of other forms include milk-like, cream-like, solid, semi-solid, paste-like, gel-like, powder-like, multi-layer, mousse-like, and similar forms. There various forms can be used as shampooing agents, rinsing agents, conditioning agents, setting lotions, hair sprays, permanent wave agents, mousses, hair colorants, and the like.

The following other components generally used in external use preparations or cosmetic compositions may be added to the external use preparation or the cosmetic composition of the present invention, provided that such components do not inhibit the effectiveness of the present invention: water, powders or coloring agents, alcohols, water-soluble polymers, film-forming agents, oil agents, oil-soluble gelling agents, organomodified clay minerals, surfactants, resins, mediums allowable in cosmetic products, adipose phases, film-forming polymers, fibers, light protection systems capable of blocking UV rays, UV absorbers, moisturizing agents, preservatives, antimicrobial agents, perfumes, salts, antioxidants, pH adjusting agents, chelating agents, refreshing agents, anti-inflammatory agents, skin beautifying components (skin-lightening agents, cell activating agents, agents for ameliorating skin roughness, circulation promoters, skin astringents, antiseborrheic agents, and the like), vitamins, amino acids, nucleic acids, hormones, clathrates, and the like; bioactive substances, medicament active ingredients, and perfumes. However, the additives are not particularly limited thereto.

The water must be free of components that are harmful to the human body and to be clean, and examples thereof include tap water, purified water, mineral water, and deep sea water. When the external use preparation or the cosmetic composition of the present invention is water-based, water soluble additives can be compounded as needed, provided that such components do not inhibit the effectiveness of the present invention. Examples of components that can be compound to constitute the aqueous phase include water soluble active materials such as vitamin Bs (described below), vitamin C and derivatives thereof, pantothenic acid and derivatives thereof, vitamins such as biotins; antiperspiration active components, water soluble UV absorbers, and various water soluble pigments, but the components are not limited thereto. In addition, a known pH adjusting agent, preservative, antimicrobial agent, or antioxidant can be compounded as needed for the purpose of improving the storage stability of the external use preparation or the cosmetic composition.

The powder and/or coloring agent can be any powder provided that it is normally used in external use preparations and cosmetic compositions, and is not limited to form (sphere, bar, needle, plate, amorphous, spindle, or the like), particle diameter (aerosol, micro-particle, pigment-grade particle, or the like), or particle structure (porous, nonporous, or the like) thereof. When compounding the powder and/or coloring agent as a pigment, preferably, one or two or more selected from an inorganic pigment powder, an organic pigment powder, and a resin powder having an average particle diameter in a range from 1 nm to 20 µm is compounded. In addition, when a pigment is used, a coated pigment is more preferable. Note that recessed fine particles formed from a silicone material, and particularly recessed fine particles with a structure that is partially spherical and hollow having an average particle size of less than 5 µm (having an arch shape or a horse hoof-shaped cross section), can also be advantageously used for the purpose of thickening the oil phase, improving tactile sensation, or the like.

Examples of the powder or coloring agent include flakes, inorganic powders, organic powders, surfactant metal salt powders (metallic soaps), colored pigments, pearl pigments, organo-modified clay minerals, and metal powder pigments. Further, compounded products of these pigments can also be used. Specific examples of inorganic powders include titanium oxide, zirconium oxide, zinc oxide, cerium oxide, magnesium oxide, barium sulfate, calcium sulfate, magnesium sulfate, calcium carbonate, magnesium carbonate, talc, mica, kaolin, sericite, white mica, synthetic mica, phlogopite, lepidolite, black mica, lithia mica, silicic acid, silicic acid anhydride, aluminum silicate, sodium silicate, magnesium sodium silicate, magnesium silicate, aluminum magnesium silicate, calcium silicate, barium silicate, strontium silicate, metal salts of tungstic acid, hydroxyapatite, vermiculite, higilite, bentonite, montmorillonite, hectorite, zeolite, ceramic powder, dicalcium phosphate, alumina, aluminum hydroxide, boron nitride, and the like. Examples of organic powders include polyamide powder, polyester powder, polyethylene powder, polypropylene powder, polystyrene powder, polyurethane powder, benzoguanamine powder, polymethylbenzoguanamine powder, polytetrafluoroethylene powder, poly (methyl methacrylate) powder, cellulose, silk powder, nylon powder, nylon 12, nylon 6, silicone powder, silicone rubber spherical powder, silicone rubber spherical powder that is surface-coated with polymethylsilsesquioxane, polymethylsilsesquioxane spherical powder, copolymers of styrene and acrylic acid, copolymers of divinylbenzene and styrene, vinyl resin, urea resin, phenol resin, fluorine resin, silicone resin, acrylic resin, melamine resin, epoxy resin, polycarbonate resin, macrocrystalline fiber powder, starch powder, lauroyl lysine, and the like. Examples of surfactant metal salt powders include zinc stearate, aluminum stearate, calcium stearate, magnesium stearate, zinc myristate, magnesium myristate, zinc palmitate, zinc laurate, zinc cetylphosphate, calcium cetylphosphate, sodium zinc cetylphosphate, and the like. Examples of colored pigments include inorganic red pigments such as red iron oxide, iron oxide, iron hydroxide, iron titanate, and the like; inorganic brown pigments such as gamma-iron oxide and the like; inorganic yellow pigments such as yellow iron oxide, ocher, and the like; inorganic black pigments such as black iron oxide, carbon black and the like; inorganic purple pigments such as manganese violet, cobalt violet, and the like; inorganic green pigments such as chromium hydroxide, chromium oxide, cobalt oxide, cobalt titanate, and the like; inorganic blue pigments such as Prussian blue, ultramarine blue, and the like; laked pigments of tar pigments such as Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 3, Green No. 201, Green No. 204, Green No. 205, Orange No. 201, Orange No. 203, Orange No. 204, Orange No. 206, Orange No. 207, and the like, laked pigments of natural pigments such as carminic acid, laccaic acid, carthamin, brazilin, crocin, and the like. Examples of pearl pigments include a pearl essence agent, titanium oxide-coated mica, titanium mica, iron oxide-coated titanium mica, titanium oxide-coated mica, bismuth oxychloride, titanium oxide-coated bismuth oxychloride, titanium oxide-coated talc, fish scale foil, titanium oxide-coated colored mica, and the like. Examples of the metal powder pigment include powders of metals such as aluminum, gold, silver, copper, platinum, stainless steel, and the like.

In particular, a powder that absorbs and scatters ultraviolet light, such as microparticulate titanium oxide, microparticulate iron-containing titanium oxide, microparticulate zinc oxide, microparticulate cerium oxide, compound products thereof, and the like may be used as the inorganic powder. More specifically, an inorganic ultraviolet light blocking component may be compounded as an ultraviolet light scattering agent such as the inorganic powder pigments and metal powder pigments described above, and examples thereof include metal oxides such as titanium oxide, zinc oxide, cerium oxide, titanium suboxide, and iron-doped titanium oxides; metal hydroxides such as iron hydroxides; metal flakes such as platy iron oxide and aluminum flakes; and ceramics such as silicon carbide. Of these, at least one type of a material selected from fine particulate metal oxides and fine particulate metal hydroxides with an average particle size in a range from 1 to 100 nm is particularly preferable.

Examples of the organo-modified clay mineral include dimethylbenzyl dodecylammonium montmorillonite clay, dimethyldioctadecylammonium montmorillonite clay, dimethylalkylammonium hectorite, benzyldimethylstearylammonium hectorite, distearyldimethylammonium chloride-treated aluminum magnesium silicate, and the like. Examples of commercially available products include Benton 27 (benzyldimethylstearylammonium chloride-treated hectorite, manufactured by Nationalred Co.), Benton 38 (distearyldimethylammonium chloride-treated hectorite, manufactured by Nationalred Co.), and the like.

The silicone rubber spherical powder (also known as a silicone elastomer spherical powder) preferably has a primary particle size in a range from 0.1 to 50 µm. Examples of commercially available products of the silicone rubber spherical powder include Trefil E-506S, Trefil E-508, 9701 Cosmetic Powder, 9702 Powder, EP-9215 Cosmetic Powder, EP-9261 TI Cosmetic Powder, EP-9293 AL Cosmetic Powder, EP-9289 LL Cosmetic Powder (all manufactured by Dow Coming Toray Co., Ltd.). In addition, the silicone rubber spherical powder can also be used in the external use preparation or cosmetic composition of the present invention in the form of an aqueous dispersion liquid. Examples of such commercially available aqueous dispersion liquids include BY29-129, and PF-2001 PIF Emulsion manufactured by Dow Corning Toray Co., Ltd.

Furthermore, the powder or coloring agent is preferably subjected to a water-repellent treatment. Additionally, a product can be used in which these powders and/or coloring agents are compounded together; or subjected to surface treatment using a general oil agent, a silicone compound other than the organopolysiloxane elastomer according to the present invention, a fluorine compound, a surfactant, or the like. One type thereof or two or more types thereof can be used, as necessary.

Examples of these water-repellent treatments include various treatments in which the powder and/or coloring agent is surface treated with a water repellency agent. Specific examples thereof include organosiloxane treatments such as a methylhydrogenpolysiloxane treatment, a silicone resin treatment, a silicone gum treatment, an acryl silicone treatment, a fluorinated silicone treatment, a glycerin-modified silicone treatment, and the like; metallic soap treatments such as a zinc stearate treatment and the like; silane treatments such as a silane coupling agent treatment, an alkylsilane treatment, and the like; fluorine compound treatments such as a perfluoroalkylsilane treatment, a perfluoroalkyl phosphate treatment, a perfluoro polyether treatment, and the like; amino acid treatments such as an N-lauroyl-L-lysine treatment and the like; oil agent treatments such as a squalane treatment and the like; and acryl treatments such as an alkyl acrylate treatment and the like. A combination of one or more of the treatments described above can be used.

Particularly preferable examples of these powders or coloring agents include at least one type of powder or coloring agent selected from the group consisting of a silicone resin powder, a silicone rubber powder, an organic resin powder (excluding silicone resin powders), an organo-modified clay mineral, titanium oxide, zinc oxide, a titanated mica, a metal soap, an inorganic body pigment, an inorganic coloration pigment and a coated pigment.

Examples of alcohols include at least one type selected from a lower alcohol, a sugar alcohol, and a higher alcohol. Specific examples include lower alcohols such as ethanol and isopropanol; sugar alcohols such as sorbitol and maltose; and higher alcohols such as lauryl alcohol, myristyl alcohol, palmityl alcohol, stearyl alcohol, behenyl alcohol, hexadecyl alcohol, oleyl alcohol, isostearyl alcohol, hexyldodecanol, octyldodecanol, cetostearyl alcohol, 2-decyltetradecinol, cholesterol, sitosterol, phytosterol, lanosterol, POE cholesterol ether, monostearyl glycerol ether (batyl alcohol), and monooleyl glycerol ether (selachyl alcohol).

The water-soluble polymer can be compounded for the purpose of enhancing sensation during use of the external use preparation or the cosmetic composition or as a water soluble moisturizing agent or film-forming polymer. Any of amphoteric, cationic, anionic, and nonionic polymers, and water-swellable clay minerals can be used, provided that the water-soluble polymer is one that is commonly used in external use preparations or cosmetic products, and it is possible to use one or two or more types of these water-soluble polymers. These water-soluble polymers have an effect of thickening the hydrous component, so the polymers are particularly useful for stabilizing the system when obtaining a gel-like hydrous external use preparation or cosmetic composition, a water-in-oil emulsion external use preparation or cosmetic composition, and an oil-in-water emulsion external use preparation or cosmetic composition.

Examples of amphoteric water-soluble polymers include amphoteric starches, dimethyldiallylammonium chloride derivatives (for example, acrylamide-acrylic acid-dimethyldiallylammonium chloride copolymers and acrylic acid-dimethyldiallylammonium chloride copolymers), and methacrylic acid derivatives (for example, polymethacryloylethyldimethylbetaines, N-methacryloyloxyethyl-N,N-dimethylammonium-α-methylcarboxybetaine-alkyl methacrylate copolymers, and the like).

Examples of cationic water-soluble polymers include quaternary nitrogen-modified polysaccharides (for example, cation-modified cellulose, cation-modified hydroxyethylcellulose, cation-modified guar gum, cation-modified locust bean gum, cation-modified starch, and the like); dimethyldiallylammonium chloride derivatives (for example, copolymers of dimethyldiallylammonium chloride and acrylamide, poly(dimethylmethylene piperidinium chloride), and the like); vinylpyrrolidone derivatives (for example, copolymers of vinylpyrrolidone and dimethylaminoethyl methacrylic acid, copolymers of vinylpyrrolidone and methacrylamide propyltrimethylammonium chloride, copolymers of vinylpyrrolidone and methylvinylimidazolium chloride, and the like); and methacrylic acid derivatives (for example, methacryloylethyldimethylbetaine-methacryloylethyltrimethyl ammonium chloride-2-hydroxyethyl methacrylate copolymers, methacryloylethyldimethylbetaine-methacryloylethyltrimethyl ammonium chloride-methoxy polyethylene glycol methacrylate copolymers, and the like).

Examples of anionic water-soluble polymers include poly(acrylic acid) and alkali metal salts thereof, poly(methacrylic acid) and alkali metal salts thereof, hyaluronic acid and alkali metal salts thereof, acetylated hyaluronic acid and alkali metal salts thereof, water-soluble polymers of aliphatic carboxylic acids or metal salts thereof, such as hydrolysates of methyl vinyl ether-maleic anhydride copolymers, carboxymethyl cellulose and alkali metal salts thereof, methyl vinyl ether-maleic acid half ester copolymers, alkanolamine solutions of acrylic resins, carboxyvinyl polymers, 2-acrylamide-2-methylpropane sulfonate polymers, or the like.

Examples of nonionic water-soluble polymers include poly(vinyl pyrrolidone), highly polymerized polyethylene glycols, PEG/PPG-36/41 dimethyl ethers, PEG/PPG-14/7 dimethyl ethers, vinyl pyrrolidone-vinyl acetate copolymers, vinyl pyrrolidone-dimethylaminoethyl methacrylate copolymers, vinyl caprolactam-vinyl pyrrolidone-dimethylaminoethyl methacrylate copolymers, cellulose and derivatives thereof (for example, methyl cellulose, ethyl cellulose, hydroxyethyl cellulose, hydroxypropyl cellulose, and carboxymethyl cellulose), keratin and collagen and derivatives thereof, calcium alginate, pullulan, agar, gelatin, tamarind seed polysaccharides, xanthan gum, carrageenan, high-methoxylpectin, low-methoxylpectin, guar gum, pectin, gum arabic, crystalline cellulose, arabinogalactan, karaya gum, gum tragacanth, alginic acid, albumin, casein, curdlan, gellan gum, dextran, pyrus cydonia seed gum, gum tragacanth, chitin/chitosan derivatives, starches (rice, corn, potato, wheat and the like), keratin and collagen and derivatives thereof, and similar natural polymer compounds.

The water-swellable clay mineral is an inorganic water-soluble polymer and is a type of colloid-containing aluminum silicate having a three-layer structure. Typical examples thereof are expressed by the following formula (A).

(X,Y)₂₋₃(Si,Al)₄O₁₀(OH)₂Z_{1/3}·nH₂O (A)

(Wherein X is Al, Fe(III), Mn(III), or Cr(III), Y is Mg, Fe(II), Ni, Zn, or Li, and Z is K, Na, or Ca). Specific examples of such inorganic water-soluble polymers include bentonite, montmorillonite, beidellite, nontronite, saponite, hectorite, magnesium aluminum silicate, and silicic anhydride, and these may be natural or synthetic products.

An oil agent described above can be used, and one type or two or more types thereof can be used as necessary.

Examples of the oil-soluble gelling agent include metal soaps such as aluminum stearate, magnesium stearate, and zinc myristate; amino acid derivatives such as N-lauroyl-L-glutamic acid, and α,γ-di-n-butylamine; dextrin fatty acid esters such as dextrin palmitate esters, dextrin stearate esters, and dextrin 2-ethylhexanoate palmitate esters; sucrose fatty acid esters such as sucrose palmitate esters and sucrose stearate esters; fructooligosaccharide fatty acid esters such as inulin stearate esters and fructooligosaccharide 2-ethylhexanoate esters; semicrystalline homopolymers or copolymers obtained by the polymerization of a monomer comprising a long chain alkyl acrylate and/or a long chain alkyl methacrylate having from 14 to 24 carbon atoms or the like; benzylidene derivatives of sorbitol such as monobenzylidene sorbitol and dibenzylidene sorbitol; and organo-modified clay minerals such as dimethylbenzyl dodecylammonium montmorillonite clay and dimethyldioctadecylammonium montmorillonite clay. One type or two or more types thereof can be used as necessary.

Surfactants other than the components described above can be compounded in the external use preparation or the cosmetic composition of the present invention. In particular, one type or two or more types of surfactants selected from the group consisting of a silicone-based surfactant other than the organopolysiloxane elastomer according to the present invention, an anionic surfactant, a cationic surfactant, a nonionic surfactant, an amphoteric surfactant, and a semipolar surfactant can be used in combination as the surfactant.

The silicone-based surfactant is a silicone-based surfactant other than the organopolysiloxane elastomer according to the present invention. This silicone-based surfactant is often used as a component for the emulsification or washing of an oil agent, and the dispersion or surface treatment of a powder, and representative examples include polyglyceryl-modified silicones, glyceryl-modified silicones, sugar-modified silicones, fluoropolyether-modified silicones, polyether-modified silicones, carboxylic acid-modified silicones, sugar-modified silicones, block copolymers of straight-chain silicones and polyethers (polysilicone-13 and the like), and long-chain alkyl-polyether comodified silicones. Since the organopolysiloxane elastomer according to the present invention consists of elastomer particles having a hydrophilic portion and a hydrophobic portion, the elastomer functions as a stabilizer for a powder dispersed in an oil. Thus, in cases where used in combination with a silicone-based nonionic surfactant, the organopolysiloxane also functions as an aid to enhance the compounding stability of the nonionic surfactant and may improve overall stability of the formulation. In particular, the organopolysiloxane elastomer according to the present invention can be advantageously used in combination with a glycerin derivative-modified silicone, a sugar-modified silicone, a sugar alcohol-modified silicone, a carboxylic acid-modified silicone, and a polyglycerin-modified silicone elastomer (otherwise known as a polyglycerated silicone elastomer), and a silicone-based nonionic surfactant in which alkyl branching, straight-chain silicone branching, siloxane dendrimer branching or the like is performed along with the hydrophilic group as necessary can be advantageously used. Note that it is also possible to use the substance in combination with a polyoxyalkylene-modified silicone (a polyether-modified silicone, a fluorine polyether-modified silicone, or the like), a polyether-modified silicone elastomer (otherwise known as a polyoxyalkylated silicone elastomer), or an organopolyoxyalkylene group-containing surfactant.

Examples of the anionic surfactants include those in which carboxylic acid-modified silicone is neutralized using an alkaline substance; saturated or unsaturated fatty acid salts (for example, sodium laurate, sodium stearate, sodium oleate, sodium linolenate, and the like); alkylsulfuric acid salts; alkylbenzene sulfonic acids (for example, hexylbenzenesulfonic acid, octylbenzenesulfonic acid, dodecylbenzenesulfonic acid, and the like) and salts thereof; polyoxyalkylene alkyl ether sulfuric acid salts; polyoxyalkylene alkenyl ether sulfuric acid salts; polyoxyethylene alkylsulfuric ester salts; sulfosuccinic acid alkyl ester salts; polyoxyalkylene sulfosuccinic acid alkyl ester salts; polyoxyalkylene alkylphenyl ether sulfuric acid salts; alkanesulfonic acid salts; octyltrimethylammonium hydroxide; dodecyltrimethylammonium hydroxide; alkyl sulfonates; polyoxyethylene alkylphenyl ether sulfuric acid salts; polyoxyalkylene alkyl ether acetic acid salts; alkyl phosphoric acid salts; polyoxyalkylene alkyl ether phosphoric acid salts; acylglutamic acid salts; α-acylsulfonic acid salts; alkylsulfonic acid salts; alkylallylsulfonic acid salts; α-olefinsulfonic acid salts; alkylnaphthalene sulfonic acid salts; alkanesulfonic acid salts; alkyl- or alkenylsulfuric acid salts; alkylamide sulfuric acid salts; alkyl- or alkenyl phosphoric acid salts; alkylamide phosphoric acid salts; alkyloylalkyl taurine salts; N-acylamino acid salts; sulfosuccinic acid salts; alkyl ether carboxylic acid salts; amide ether carboxylic acid salts; α-sulfofatty acid ester salts; alanine derivatives; glycine derivatives; and arginine derivatives. Examples of salts include alkali metal salts such as sodium salts and the like, alkaline earth metal salts such as magnesium salts and the like, alkanolamine salts such as triethanolamine salts and the like, and ammonium salts.

Examples of cationic surfactants include alkyltrimethylammonium chloride, stearyltrimethylammonium chloride, lauryltrimethylammonium chloride, cetyltrimethylammonium chloride, beef tallow alkyltrimethylammonium chloride, behenyltrimethylammonium chloride, stearyltrimethylammonium bromide, behenyltrimethylammonium bromide, distearyldimethylammonium chloride, dicocoyldimethylammonium chloride, dioctyldimethylammonium chloride, di(POE)oleylmethylammonium (2 EO) chloride, benzalkonium chloride, alkyl benzalkonium chloride, alkyl dimethylbenzalkonium chloride, benzethonium chloride, stearyl dimethylbenzylammonium chloride, lanolin derivative quaternary ammonium salt, diethylaminoethylamide stearate, dimethylaminopropylamide stearate, behenic acid amide propyldimethyl hydroxypropylammonium chloride, stearoyl colaminoformyl methylpyridinium chloride, cetylpyridinium chloride, tall oil alkylbenzyl hydroxyethylimidazolinium chloride, and benzylammonium salt.

Examples of nonionic surfactants include polyglyceryl diisostearate and polyhydroxy diglyceryl stearate, isostearyl glyceryl ethers, polyoxyalkylene ethers, polyoxyalkylene alkyl ethers, polyoxyalkylene fatty acid esters, polyoxyalkylene fatty acid diesters, polyoxyalkylene resin acid esters, polyoxyalkylene (cured) castor oils, polyoxyalkylene alkyl phenols, polyoxyalkylene alkyl phenyl ethers, polyoxyalkylene phenyl ethers, polyoxyalkylene alkyl esters, polyoxyalkylene alkyl esters, sorbitan fatty acid esters, polyoxyalkylene sorbitan alkyl esters, polyoxyalkylene sorbitan fatty acid esters, polyoxyalkylene sorbitol fatty acid esters, polyoxyalkylene glycerol fatty acid esters, polyglycerol alkyl ethers, polyglycerol fatty acid esters, sucrose fatty acid esters, fatty acid alkanolamides, alkylglucosides, polyoxyalkylene fatty acid bisphenyl ethers, polypropylene glycol, diethyleneglycol, polyoxyethylene-polyoxypropylene block polymers, alkylpolyoxyethylene-polyoxypropylene block polymer ethers, and fluorine-based surfactants.

Examples of amphoteric surfactants include imidazoline-type, amidobetaine-type, alkylbetaine-type, alkylamidobetaine-type, alkylsulfobetaine-type, amidosulfobetaine-type, hydroxysulfobetaine-type, carbobetaine-type, phosphobetaine-type, aminocarboxylic acid-type, and amidoamino acid-type amphoteric surfactants. Specifically, examples include imidazoline-type amphoteric surfactants such as 2-undecyl-N,N,N-(hydroxyethylcarboxymethyl)-2-imidazoline sodium, 2-cocoyl-2-imidazolinium hydroxide-1-carboxyethyloxy disodium salt, and the like; alkylbetaine-type amphoteric surfactants such as lauryl dimethylaminoacetic betaine, myristyl betaine, and the like; amidobetaine-type amphoteric surfactants such as coconut oil fatty acid amidopropyl dimethylamino acetic acid betaine, palm kernel oil fatty acid amidopropyl dimethylamino acetic acid betaine, beef tallow fatty acid amidopropyl dimethylamino acetic acid betaine, hardened beef tallow fatty acid amidopropyl dimethylamino acetic acid betaine, lauric acid amidopropyl dimethylamino acetic acid betaine, myristic acid amidopropyl dimethylamino acetic acid betaine, palmitic acid amidopropyl dimethylamino acetic acid betaine, stearic acid amidopropyl dimethylamino acetic acid betaine, oleic acid amidopropyl dimethylamino acetic acid betaine, and the like; alkylsulfobetaine-type amphoteric surfactants such as coconut oil fatty acid dimethyl sulfopropyl betaine and the like; alkyl hydroxy sulfobetaine-type amphoteric surfactants such as lauryl dimethylaminohydroxy sulfobetaine and the like; phosphobetaine-type amphoteric surfactants such as laurylhydroxy phosphobetaine and the like; and amidoamino acid-type amphoteric surfactants such as sodium N-lauroyl-N'-hydroxyethyl-N'-carboxymethyl ethylenediamine, sodium N-oleoyl-N'-hydroxyethyl-N'-carboxymethyl ethylenediamine, sodium N-cocoyl-N'-hydroxyethyl-N'-carboxymethyl ethylenediamine, potassium N-lauroyl-N'-hydroxyethyl-N'-carboxymethyl ethylenediamine, potassium N-oleoyl-N'-hydroxyethyl-N'-carboxymethyl ethylenediamine, sodium N-lauroyl-N-hydroxyethyl-N'-carboxymethyl ethylenediamine, sodium N-oleoyl-N-hydroxyethyl-N'-carboxymethyl ethylenediamine, sodium N-cocoyl-N-hydroxyethyl-N'-carboxymethyl ethylenediamine, monosodium N-lauroyl-N-hydroxyethyl-N',N'-dicarboxymethyl ethylenediamine, monosodium N-oleoyl-N-hydroxyethyl-N',N'-dicarboxymethyl ethylenediamine, monosodium N-cocoyl-N-hydroxyethyl-N',N'-dicarboxymethyl ethylenediamine, disodium N-lauroyl-N-hydroxyethyl-N',N'-dicarboxymethyl ethylenediamine, disodium N-oleoyl-N-hydroxyethyl-N',N'-dicarboxymethyl ethylenediamine, disodium N-cocoyl-N-hydroxyethyl-N',N'-dicarboxymethyl ethylenediamine, and the like.

Examples of semipolar surfactants include alkylamine oxide-type surfactants, alkylamine oxides, alkylamide amine oxides, alkylhydroxyamine oxides, and the like. Alkyldimethylamine oxides having from 10 to 18 carbon atoms, alkoxyethyl dihydroxyethylamine oxides having from 8 to 18 carbon atoms, and the like are preferably used. Specific examples thereof include dodecyldimethylamine oxide, dimethyloctylamine oxide, diethyldecylamine oxide, bis-(2-hydroxyethyl)dodecylamine oxide, dipropyltetradecylamine oxide, methylethylhexadecylamine oxide, dodecylamidopropyldimethylamine oxide, cetyldimethylamine oxide, stearyldimethylamine oxide, tallow dimethylamine oxide, dimethyl-2-hydroxyoctadecylamine oxide, lauryldimethylamine oxide, myristyldimethylamine oxide, stearyldimethylamine oxide, isostearyldimethylamine oxide, coconut fatty acid alkyldimethylamine oxide, caprylic amide propyldimethylamine oxide, capric amide propyldimethylamine oxide, lauric amide propyldimethylamine oxide, myristic amide propyldimethylamine oxide, palmitic amide propyldimethylamine oxide, stearic amide propyldimethylamine oxide, isostearic amide propyldimethylamine oxide, oleic amide propyldimethylamine oxide, ricinoleic amide propyldimethylamine oxide, 12-hydroxystearic amide propyldimethylamine oxide, coconut fatty acid amide propyldimethylamine oxide, palm kernel oil fatty acid amide propyldimethylamine oxide, castor oil fatty acid amide propyldimethylamine oxide, lauric amide ethyldimethylamine oxide, myristic amide ethyldimethylamine oxide, coconut fatty acid amide ethyldimethylamine oxide, lauric amide ethyldiethylamine oxide, myristic amide ethyldiethylamine oxide, coconut fatty acid amide ethyldiethylamine oxide, lauric amide ethyldihydroxyethylamine oxide, myristic amide ethyldihydroxyethylamine oxide, and coconut fatty acid amide ethyldihydroxyethylamine oxide.

The ultraviolet light blocking agent can be an inorganic ultraviolet light blocking agent or an organic ultraviolet light blocking agent. When the external use preparation or the cosmetic composition of the present invention is to be used for sunblocking, it is preferable for the composition to contain at least one type of organic ultraviolet light blocking agent. In particular, using both inorganic and organic ultraviolet light blocking agents in combination is preferable, and using a UV-A blocking agent in combination with a UV-B blocking agent is more preferable.

The inorganic ultraviolet light blocking agent may be compounded as an ultraviolet light scattering agent such as the inorganic pigment powders and metal powder pigments mentioned above. Examples thereof include metal oxides such as titanium oxide, zinc oxide, cerium oxide, titanium suboxide, iron-doped titanium oxides, and the like; metal hydroxides such as iron hydroxides and the like; metal flakes such as platy iron oxide, aluminum flake, and the like; and ceramics such as silicon carbide, and the like. Of these, at least one type of a material selected from fine particulate metal oxides and fine particulate metal hydroxides with an average particle diameter in a range from 1 to 100 nm and a particulate, plate-like, needle-like, or fiber form is particularly preferable. These powders are preferably subjected to conventionally known surface treatment such as, for example, fluorine compound treatment (of which perfluoroalkyl phosphate treatment, perfluoroalkylsilane treatment, perfluoropolyether treatment, fluorosilicone treatment, and fluorinated silicone resin treatment are preferable), silicone treatment (of which methylhydrogen polysiloxane treatment, dimethylpolysiloxane treatment, vapor-phase tetramethyltetrahydrogen cyclotetrasiloxane treatment, and glycerin-modified silicone treatment are preferable), silicone resin treatment (of which trimethylsiloxysilicic acid treatment is preferable), pendant treatment (which is a method of adding alkyl chains after vapor-phase silicone treatment), silane coupling agent treatment, titanium coupling agent treatment, silane treatment (of which alkylsilane or alkylsilazane treatment is preferable), oil agent treatment, N-acylated lysine treatment, polyacrylic acid treatment, metal soap treatment (of which a stearic acid or myristic acid salt is preferable), acrylic resin treatment, metal oxide treatment, or the like, and the powders are preferably treated with a plurality of these treatments. For example, the surface of the fine particulate titanium oxide can be coated with a metal oxide such as silicon oxide and alumina, and, thereafter, surface treating using an alkylsilane can be carried out. A total amount of the surface treatment agent is preferably in a range from 0.1 to 50 wt.% (mass%) of the powder.

The organic ultraviolet light blocking agent is a lipophilic ultraviolet light blocking agent, and examples thereof include benzoic acid-based UV absorbers such as paraaminobenzoic acid (hereinafter abbreviated as "PABA"), PABA monoglycerol ester, N,N-dipropoxy-PABA ethyl ester, N,N-diethoxy-PABA ethyl ester, N,N-dimethyl-PABA ethyl ester, N,N-dimethyl-PABA butyl ester, and 2-[4-(diethylamino)-2-hydroxybenzoyl]hexylester benzoate (trade name: Uvinul A plus); anthranilic acid-based UV absorbers such as homomenthyl-N-acetylanthranilate; salicylic acid-based UV absorbers such as amyl salicylate, menthyl salicylate, homomenthyl salicylate, octyl salicylate, phenyl salicylate, benzyl salicylate, and p-isopropanolphenyl salicylate; cinnamic acid-based UV absorbers such as octyl cinnamate, ethyl-4-isopropylcinnamate, methyl-2,5-diisopropylcinnamate, ethyl-2,4-diisopropylcinnamate, methyl-2,4-diisopropylcinnamate, propyl-p-methoxycinnamate, isopropyl p-methoxycinnamate, isoamyl-p-methoxycinnamate, octyl-p-methoxycinnamate (2-ethylhexyl-p-methoxycinnamate), 2-ethoxyethyl-p-methoxycinnamate, cyclohexyl-p-methoxy cinnamate, ethyl-α-cyano-β-phenylcinnamate, 2-ethylhexyl-α-cyano-β-phenylcinnamate, glyceryl mono-2-ethylhexanoyl-diparamethoxycinnamate, 3-methyl-4-[methylbis(trimethylsiloxy) silyl]butyl 3,4,5-trimethoxycinnamate, and dimethicodiethyl benzalmalonate (trade name: Parsol SLX (INCI name: Polysilicone-15)); benzophenone-based UV absorbers such as 2,4-dihydroxybenzophenone, 2,2' -dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenylbenzophenone-2-carboxylate, hydroxy-4-n-octoxybenzophenone, and 4-hydroxy-3-carboxybenzophenone; benzotriazole-based UV absorbers such as 3-(4'-methylbenzylidene)-d,l-camphor, 3-benzylidene-d,l-camphor, urocanic acid, urocanic acid ethyl ester, 2-phenyl-5-methylbenzoxazole, 2,2'-hydroxy-5-methylphenyl benzotriazole, 2-(2'-hydroxy-5'-t-octylphenyl)benzotriazole, 2-(2'-hydroxy-5'-methylphenyl)benzotriazole, dibenzaladine, dianisoylmethane, 4-methoxy-4'-t-butyldibenzoylmethane, 5-(3,3-dimethyl-2-norbornylidene)-3-pentan-2-one, 2,2'-methylene bis(6-(2H-benzotriazole-2-yl)-4-(1,1,3,3-tetramethylbutyl)phenol) (trade name: Tinosorb® M), 2-[4-(2-ethylhexyloxy)-2-hydroxyphenyl]-2H-benzotriazole, and 2-(2-hydroxy-4-isobutoxyphenyl)-2H-benzotriazole; triazine-based UV absorbers such as 2,4,6-tris[4-(2-ethylhexyloxycarbonyl)anilino]1,3,5-triazine (INCl: octyltriazone) and 2,4-bis-6-(4-methoxyphenyl)-1,3,5-triazine (INCl: bis-ethylhexyloxyphenol methoxyphenyl triazine, trade name: Tinosorb® S); and 2-cyano-3,3-diphenylprop-2-enoate-2-ethylhexyl ester (INCl: octocrylene).

Generally, the organic-based UV absorber has high polarity and does not readily dissolve. Therefore, conventionally, it has been difficult to stably compound a desired (high) amount of the organic-based UV absorber in water-in-oil (W/O) emulsion cosmetic compositions. However, when the organopolysiloxane elastomer of the present invention is used as an emulsifier and a medium polarity oil such as an ester oil or the like is combined therewith as a binding agent, a stable UV absorber-containing W/O emulsion cosmetic composition can be obtained even when the oil phase contains a low polarity oil such as a silicone oil, a hydrocarbon oil, or the like. At this time, it is preferable to use a xylitol-modified silicone having a siloxane dendron structure and a long-chain alkyl group or a diglycerin-modified silicone having a siloxane dendron structure and a long-chain alkyl group as a second emulsifying agent and to use this agent in combination with the organopolysiloxane elastomer of the present invention. In this case, the compounded amount of the organic-based UV absorber is preferably in a range from 0.1 to 10 mass% and the compounded amount of the binding agent is preferably in a range from 0.005 to 5 mass%.

Additionally, it is possible to use a product in which the organo-ultraviolet light blocking agent is comprised in a hydrophobic polymer powder. The polymer powder may be or may not be hollow, and preferably has an average primary particle size in a range from 0.1 to 50 µm. Particle size distribution may be broad or sharp. Types of polymer include acrylic resins, methacrylic resins, styrene resins, polyurethane resins, polyethylene, polypropylene, polyethylene terephthalate, silicone resins, nylons, acrylamide resins, and silylated polypeptide resins. A polymer powder comprising from 0.1 to 30 wt.% (mass%) of an organic ultraviolet light blocking agent is preferable, and a polymer powder comprising 4-tert-butyl-4'-methoxydibenzoylmethane, which is a UV-A absorber, is particularly preferable.

The ultraviolet light blocking agent that can be preferably used is at least one selected from the group consisting of fine particulate titanium oxide, fine particle zinc oxide, paramethoxy cinnamic acid 2-ethylhexyl, 4-tert-butyl-4'-methoxydibenzoylmethane, a benzotriazole-based UV absorber, and a triazine-based UV absorber. These ultraviolet light blocking agents are generally used, are easy to acquire, and have high ultraviolet light blocking effects and, thus can be beneficially used.

Examples of salts include inorganic salts, organic salts, amine salts, and amino acid salts. Examples of inorganic salts include sodium salts, potassium salts, magnesium salts, calcium salts, aluminum salts, zirconium salts, and zinc salts of inorganic acids such as hydrochloric acid, sulfuric acid, carbonic acid, and nitric acid. Examples of organic acid salts include salts of organic acids such as acetic acid, dehydroacetic acid, citric acid, malic acid, succinic acid, ascorbic acid, and stearic acid. Examples of amine salts and amino acid salts include salts of amines such as triethanolamine and salts of amino acids such as glutamic acid. In addition, salts of hyaluronic acid, chondroitin sulfuric acid, and the like, aluminum zirconium glycine complexes and the like, and acid-alkali neutralization salts and the like used in cosmetic product formulations can be used.

Examples of moisturizing agents include polyhydric alcohols such as glycerin, sorbitol, propylene glycol, dipropylene glycol, 1,3-butylene glycol, glucose, xylitol, maltitol, and polyethyleneglycol; hyaluronic acid, chondroitin sulfuric acid, pyrrolidone carboxylic acid salts, polyoxyethylene methylglucoside, polyoxypropylene methylglucoside, PEG/PPG dimethylether, polyols, glycols, glycol esters, and the like.

Examples of preservatives include alkyl paraoxybenzoates, benzoic acid, sodium benzoate, sorbic acid, potassium sorbate, phenoxyethanol, and the like. Examples of antimicrobial agents include benzoic acid, salicylic acid, carbolic acid, sorbic acid, alkyl paraoxybenzoates, parachloromethacresol, hexachlorophene, benzalkonium chloride, chlorhexidine chloride, trichlorocarbanilide, triclosan, photosensitizers, phenoxy ethanol and the like. However, in cases where the cosmetic composition is a rouge, it is preferable that these are not included.

Examples of the antioxidants include tocopherol, butylhydroxyanisole, dibutylhydroxytoluene, phytic acid, and the like.

Examples of pH adjusting agents include lactic acid, citric acid, glycolic acid, succinic acid, tartaric acid, dl-malic acid, potassium carbonate, sodium hydrogen carbonate, ammonium hydrogen carbonate, and the like.

Examples of the chelating agent include alanine, sodium salt of edetic acid, sodium polyphosphate, sodium metaphosphate, phosphoric acid, and the like.

Examples of the refreshing agents include L-menthol and camphor, and examples of the anti-inflammatory agents include allantoin, glycyrrhetic acid, glycyrrhizinic acid, tranexamic acid, and azulene.

Moreover, examples of skin beautifying components include skin-lightening agents such as placenta extracts, arbutin, glutathione, saxifrageous extracts, and the like; cell activating agents such as royal jelly and the like; agents for ameliorating skin roughness; circulation promoters such as nonylic acid vanillylamide, benzyl nicotinate, beta-butoxyethyl nicotinate, capsaicin, zingerone, cantharide tincture, ichthammol, caffeine, tannic acid, α-borneol, tocopherol nicotinate, inositol hexanicotinate, cyclandelate, cinnarizine, tolazoline, acetylcholine, verapamil, cepharanthine, γ-oryzanol, and the like; astringents such as zinc oxide, tannic acid, and the like; antiseborrheic agents such as sulfur, thianthol, and the like; and the like. Examples of vitamins include vitamin As such as vitamin A oil, retinol, retinol acetate, retinol palmitate, and the like; vitamin Bs such as vitamin B2s such as riboflavin, riboflavin butyrate, flavin adenine dinucleotide, and the like; vitamin B6s such as pyridoxine hydrochloride, pyridoxine dioctanoate, pyridoxine tripalmitate, and the like; vitamin B12 and derivatives thereof; vitamin B15 and derivatives thereof, and the like; vitamin Cs such as L-ascorbic acid, L-ascorbyl dipalmitic acid esters, sodium L-ascorbyl 2-sulfate, dipotassium L-ascorbyl phosphoric acid diester, and the like; vitamin Ds such as ergocalciferol, cholecalciferol, and the like; vitamin Es such as α-tocopherol, β-tocopherol, γ-tocopherol, dl-α-tocopherol acetate, dl-α-tocopherol nicotinate, dl-α-tocopherol succinate, and the like; vitamin H; vitamin P; nicotinic acids such as nicotinic acid, benzyl nicotinate, and the like; pantothenic acids such as calcium pantothenate, D-pantothenyl alcohol, pantothenyl ethyl ether, acetyl pantothenyl ethyl ether, and the like; and the like.

Examples of amino acids include amino acids such as glycine, valine, leucine, isoleucine, serine, threonine, phenylalanine, arginine, lysine, aspartic acid, glutamic acid, cysteine, cysteine, methionine, tryptophan, and the like, and/or salts thereof.

Examples of nucleic acids include deoxyribonucleic acid, and examples of hormones include estradiol and ethenyl estradiol.

The bioactive component is a substance that imparts some sort of bioactivity to the skin or the hair when applied to the skin or the hair, and examples thereof are lipophilic substances. Examples thereof include anti-inflammatory agents, anti-aging agents, tightening agents, hair regrowth agents, hair growth promoters, moisturizing agents, circulation promoters, drying agents, warming agents, vitamins, wound healing accelerators, irritation mitigation agents, analgesics, cell activating agents, enzyme components, and the like. Similarly, natural vegetable extract components, seaweed extract components and herbal medicine components can be preferably blended.

The medicament active component is a substance having a disease-treating effect, and examples thereof include proteins, peptides, and low molecular weight compounds.

The perfume is not particularly limited as long as it is a lipophilic perfume, and examples thereof include perfumes that are extracted from a variety of plant flowers, seeds, leaves, roots, and the like, perfumes extracted from seaweed, perfumes extracted from a variety of animal parts and secretions (for example, musk or sperm oil), and artificially synthesized perfumes (for example, menthol, musk, acetic acid esters, and vanilla). The perfume is compounded for the purpose of imparting a fragrance or scent to the external use preparation or the cosmetic composition. Examples of the pigment include oil-soluble dyes, water-soluble dyes, extender pigments, inorganic pigments, organic pigments, and lipophilic optical brighteners.

[Combinations with other silicone-based cosmetic raw materials]

Depending on the dosage form and formulation thereof, the external use preparation or the cosmetic composition according to the present invention may further contain a solid silicone resin or crosslinking organopolysiloxane (excluding the organopolysiloxane elastomer of the present invention), an acryl silicone dendrimer copolymer, a silicone raw rubber (silicone gum), a polyamide-modified silicone, an alkyl-modified silicone wax, or an alkyl-modified silicone resin wax. The main chain of the organopolysiloxane elastomer of the present invention comprises a polysiloxane chain, and the organopolysiloxane elastomer has a hydrophilic sugar alcohol modified group as a modifying group. This is advantageous in that it is possible to design a cosmetic composition that has excellent compounding stability with these silicone-based compounds and that takes advantage of the tactile sensation that is characteristic to these silicone-based cosmetic raw materials.

Solid silicone resin or crosslinking organopolysiloxane

The external use preparation or cosmetic composition of the present invention may further contain a solid silicone resin or crosslinking organopolysiloxane. The solid silicone resin or crosslinking organopolysiloxane preferably is hydrophobic, having absolutely no solubility in water at room temperature, or a solubility of less than 1 wt.% (mass%) per 100 g of water.

The solid silicone resin is an organopolysiloxane having a highly branched structure, a net-like structure, or a cage structure, and is solid at room temperature. Any type of product may be used, provided that it is a silicone resin that is commonly used in cosmetic compositions and does not oppose the object of the present invention. The solid silicone resin may be a spherical powder, a flaky powder, a needle like powder, a plate-like flaky powder (including plate-like powders having appearances and particle aspect ratios commonly identified with plate-like forms), or a similar particulate. Particularly, a silicone resin powder having monoorganosiloxy units (T units) and/or siloxy units (Q units), described below, can be preferably used.

Compounding the organopolysiloxane elastomer of the present invention along with the solid silicone resin is useful because the compatibility with the oil agent and uniform dispersibility are improved, improvement effects in sensation during use can be obtained, namely uniform adhesion to the applied area due to the compounding of the solid silicone resin, and improvement effects in cosmetic retainability such as moisture resistance and sebum resistance can be obtained. Note that solid silicone resins are often used as film-forming polymers that can be compounded in an oil-based system.

Examples of the solid silicone resin include MQ resins, MDQ resins, MTQ resins, MDTQ resins, TD resins, TQ resins, and TDQ resins formed from arbitrary combinations of triorganosiloxy units (M units) (where the organo groups are only methyl groups, or are methyl groups and vinyl groups or phenyl groups), diorganosiloxy units (D units) (where the organo groups are only methyl groups, or are methyl groups and vinyl groups or phenyl groups), monoorganosiloxy units (T units) (where the organo groups are methyl groups, vinyl groups, or phenyl groups), and siloxy units (Q units). Furthermore, other examples include trimethylsiloxysilicate, polyalkylsiloxysilicate, dimethylsiloxy unit-containing trimethylsiloxysilicate, and alkyl(perfluoroalkyl)siloxysilicate. These silicone resins are preferably oil soluble and can be dissolved in volatile silicone.

Particularly, a phenyl silicone resin having a high refractive index and a high content of phenyl groups (e.g. 217 Flake resin and the like, manufactured by Dow Corning Toray Co., Ltd.) can be easily used as a flaky silicone resin powder and, when compounded in a cosmetic composition, in particular, can impart a radiant feeling of sheerness to the skin or hair.

Types of crosslinking organopolysiloxanes (excluding the organopolysiloxane elastomer of the present invention) include non-emulsifiable crosslinking organopolysiloxanes and emulsifiable crosslinking organopolysiloxanes, but the organopolysiloxane chain preferably has a structure which is three-dimensionally crosslinked by a reaction between a crosslinking component consisting of a polyether unit, an alkylene unit having from 4 to 20 carbon atoms, or an organopolysiloxane unit and other optional modifying agents or the like.

Specifically, the crosslinking organopolysiloxane (excluding the organopolysiloxane elastomer of the present invention) can be obtained by performing an addition reaction on an organohydrogenpolysiloxane having a silicon-bonded hydrogen atom, a polyether compound or glycerin derivative having reactive unsaturated groups at both terminals of the molecular chain, an unsaturated hydrocarbon having more than one double bond in the molecule, and an organopolysiloxane having more than one double bond in the molecule. Here, the crosslinking organopolysiloxane may have or may be free of unreacted silicon-bonded hydrogen atoms, phenyl groups, and similar aromatic hydrocarbon groups; octyl groups and similar long chain alkyl groups having from 6 to 30 carbon atoms; polyether groups, carboxyl groups, and similar modifying functional groups. In other words, any crosslinking organopolysiloxane can be used without limitations to physical modes or preparation methods such as dilution, properties, and the like.

As an example, the crosslinking organopolysiloxane can be obtained via an addition reaction of crosslinking components selected from an organohydrogenpolysiloxane formed from a structural unit selected from the group consisting of an SiO₂ unit, an HSiO_{1.5} unit, a R^{b}SiO_{1.5} unit, a R^{b}HSiO unit, a R^{b}₂SiO unit, a R^{b}₃SiO_{0.5} unit, and a R^{b}₂HSiO_{0.5} unit (where R^{b} is a substituted or unsubstituted monovalent hydrocarbon group having from 1 to 30 carbon atoms, with the exception of aliphatic unsaturated groups; and a portion of R^{b} is a monovalent hydrocarbon group having from 8 to 30 carbon atoms), the organohydrogenpolysiloxane having an average of 1.5 or more silicon-bonded hydrogen atoms in the molecule; a polyoxyalkylene compound having unsaturated hydrocarbon groups at both molecular terminals; a polyglycerine compound, a polyglycidylether compound, or similar polyether compound; an α,ω-diene unsaturated hydrocarbon expressed by the general formula: CH₂=CH-CᵣH₂ᵣ-CH=CH₂ (where r is an integer from 0 to 26); and an organopolysiloxane formed from a structural unit selected from the group consisting of an SiO₂ unit, a (CH₂=CH)SiO_{1.5} unit, a R^{c}SiO_{1.5} unit, a R^{c}(CH₂=CH)SiO unit, a R^{c}₂SiO unit, a R^{c}₃SiO_{0.5} unit, and a R^{c}₂(CH₂=CH)SiO_{0.5} unit (where R^{c} is a substituted or unsubstituted monovalent hydrocarbon group having from 1 to 30 carbon atoms, with the exception of aliphatic unsaturated groups), the organopolysiloxane having an average of 1.5 or more silicon-bonded vinyl groups in the molecule. Note that by addition reacting the unreacted silicon-bonded hydrogen atoms, the modifying functional groups described above can be introduced. For example, by reacting 1-hexene with a crosslinking organopolysiloxane having unreacted silicon-bonded hydrogen atoms, hexyl groups (C6 alkyl groups) are introduced.

Such a crosslinking organopolysiloxane can be used without limitations, regardless of the physical modes or preparation methods such as dilution, properties, and the like, but particularly preferable examples thereof include α,ω-diene crosslinking silicone elastomers (commercially available products include DC 9040 Silicone Elastomer Blend, DC 9041 Silicone Elastomer Blend, DC 9045 Silicone Elastomer Blend, DC 9046 Silicone Elastomer Blend, EL-9140 DM Silicone Elastomer Blend, 9546 Silicone Elastomer Blend, 9027 Silicone Elastomer Blend, FB-9586 Silicone Elastomer Blend, and EL-8040 ID Silicone Organic Blend manufactured by Dow Corning USA) described in US Patent No. 5,654,362. Additional examples of partially crosslinked organopolysiloxane polymers include a (dimethicone/vinyldimethicone) crosspolymer, a (dimethicone/phenylvinyldimethicone) crosspolymer, a (PEG-8 to 30/C6 to C30 alkyldimethicone) crosspolymer, a (vinylmethicone/C6 to C30 alkyldimethicone) crosspolymer, and a (dimethicone/polyglycerin) crosspolymer according to the INCI names (International Nomenclature Cosmetic Ingredient Labeling Names).

Other preferable examples of crosslinkable organopolysiloxanes include a silicone polyester elastomer gel disclosed in WO2007/109240 and WO2009/006091 in which compatibility with various organic components is enhanced and stable thickening effects are displayed as a result of introducing a polyoxypropylene group; commercially available Dow Corning EL-8050 ID SILICONE ORGANIC ELASTOMER BLEND, Dow Corning EL-8051 IN SILICONE ORGANIC ELASTOMER BLEND, and Dow Corning EL-7040 HYDRO ELASTOMER BLEND; and PITUITOUS SILICONE FLUIDS disclosed in WO2011/028765 and WO2011/028770.

When compounded in an external use preparation or a cosmetic composition as an emulsifiable crosslinking organopolysiloxane that is crosslinked by a polyether compound, the organopolysiloxane elastomer of the present invention functions as a dispersion stabilizer, which yields the advantage that a uniform emulsification system (dosage form) can be formed.

On the other hand, when a non-emulsifiable crosslinking organopolysiloxane (also called a non-emulsifiable silicone elastomer) which is crosslinked by an unsaturated group-containing organopolysiloxane or an unsaturated hydrocarbon group such as a diene, a polyoxypropylene having reactive unsaturated groups at both molecular terminals of the molecular chain, or the like is compounded as a component in an external use preparation or a cosmetic composition, a thick, smooth tactile sensation can be imparted to the skin or the hair, and a matte finish and effects of concealing wrinkles, pigmented spots, and the like can be obtained. Further, this configuration is advantageous in that the feel of adhesion to the skin of the cosmetic composition is improved and cosmetic retainability is enhanced because the effects of retaining various oil agents and increasing the viscosity are high.

One type or two or more types of the solid silicone resin or crosslinking organopolysiloxane (excluding the organopolysiloxane elastomer of the present invention) can be compounded in accordance with the purpose thereof and are preferably compounded in a range from 0.05 to 25 wt.% (mass%) and more preferably in a range from 0.1 to 15 wt.% (mass%) of the entire cosmetic composition or the external use preparation, depending on the purpose and the intention of compounding.

### Acryl silicone dendrimer copolymer

The external use preparation or the cosmetic composition of the present invention may further contain an acryl silicone dendrimer copolymer. The acryl silicone dendrimer copolymer is a vinyl polymer having a carbosiloxane dendrimer structure on the sidechain, and particularly preferable examples thereof include the vinyl polymer described in Japanese Patent No. 4009382 (Japanese Unexamined Patent Application Publication No. 2000-063225). Examples of commercially available products include FA4001 CM Silicone Acrylate, FA4002 ID Silicone Acrylate, and the like manufactured by Dow Corning Toray Co., Ltd. and acryl silicone dendrimer copolymers having a long chain alkyl group on the sidechain or the like having from 8 to 30 carbon atoms and preferably from 14 to 22 carbon atoms. When compounding the acryl silicone dendrimer copolymer alone, superior film formability can be obtained. Therefore, by compounding the acryl silicone dendrimer copolymer in the external use preparation or cosmetic composition according to the present invention, a strong coating film can be formed on the applied part, and cosmetic durability such as sebum resistance, rubbing resistance, and the like can be significantly improved.

Using the organopolysiloxane elastomer of the present invention in combination with the acryl silicone dendrimer copolymer yields advantages in that the surface protective properties such as sebum resistance can be improved due to strong water repellency provided by the carbosiloxane dendrimer structure, and irregularities such as pores can also be effectively concealed. In addition, the organopolysiloxane elastomer of the present invention causes the acryl silicone dendrimer copolymer to blend well with the other oil agents and has excellent compatibility with the skin and surface of the hair, so the hardness of the acryl silicone dendrimer copolymer can be reduced, and a film with an excellent adhesive sensation and a lasting feel of moisture can be achieved. In addition, cosmetic retainability is improved, which has the advantage that degradation of the skin surface or hair can be suppressed for an extended period of time.

A compounded amount of the acryl silicone dendrimer copolymer can be suitably selected based on the purpose and compounding intent thereof, but is preferably in a range from 1 to 99 wt.% (mass%) and more preferably in a range from 30 to 70 wt.% (mass%) of the entire external use preparation or cosmetic composition.

### [Silicone raw rubber (silicone gum)]

In the external use preparation or the cosmetic composition of the present invention, an ultra-high viscous yet fluid component having a viscosity at room temperature of 1,000,000 mm²/s or higher, which is referred to as a silicone raw rubber (silicone gum), can be suitably used as the silicone oil. Silcone rubber is a straight-chained diorganopolysiloxane with an ultra-high degree of polymerization and is also called silicone raw rubber or organopolysiloxane rubber. Silicone rubber is differentiated from the oily silicones described above because the degree of polymerization of silicone rubber is high and, as a result, it has a degree of plasticity that is measurable. These silicone gums can be compounded directly in the external use preparation or the cosmetic composition of the present invention - in a hair cosmetic composition for the purpose of imparting a tactile sensation - or can be compounded as liquid gum dispersions in which an oil-like silicone is dispersed (oil dispersions of silicone gums).

Examples of such a silicone raw rubber include substituted or unsubstituted organopolysiloxanes having a dialkylsiloxy unit (D unit) such as a dimethylpolysiloxane, a methylphenylpolysiloxane, an aminopolysiloxane, or a methylfluoroalkylpolysiloxane, or products having micro-crosslinked structures thereof, and typical examples thereof include products expressed by the general formula: R¹⁰(CH₃)₂SiO[(CH₃)₂SiO]ₛ[(CH₃)R¹²SiO]ₜSi(CH₃)₂R¹⁰ (wherein R¹² moieties are each independently a group selected from vinyl groups, phenyl groups, alkyl groups having from 6 to 20 carbon atoms, aminoalkyl groups having from 3 to 15 carbon atoms, perfluoroalkyl groups having from 3 to 15 carbon atoms, and quaternary ammonium salt group-containing alkyl groups having from 3 to 15 carbon atoms; the terminal group R¹⁰ moieties are each independently a group selected from alkyl groups having from 1 to 8 carbon atoms, phenyl groups, vinyl groups, aminoalkyl groups having from 3 to 15 carbon atoms, hydroxyl groups, and alkoxy groups having from 1 to 8 carbon atoms; s=2,000 to 6,000; t=0 to 1,000; and s+t=2,000 to 6,000). Of these, a dimethylpolysiloxane raw rubber having a degree of polymerization of 3,000 to 20,000 is preferable. Additionally, an amino-modified methylpolysiloxane raw rubber having a 3-aminopropyl group, an N-(2-aminoethyl)3-aminopropyl group, or the like on the molecular sidechain or terminal is preferable. Moreover, in the present invention, one or two or more types of silicone gums can be used as necessary.

Silicone gum has an ultra-high degree of polymerization and, therefore forms a protective film with superior breathability and retention on hair. Therefore, the silicone gum is a component which can particularly provide glossiness and luster to hair and can impart a texture of firmness and body to the entire hair during use and after use. A silicone gum with a high degree of polymerization can also be compounded in the external use preparation or the cosmetic composition in a form that is diluted with a silicone oil to reduce the viscosity. Further, the use of the organopolysiloxane elastomer of the present invention imparts a smooth, sliding feeling to wet hair at the time of rinsing or the like, and the substance is effectively absorbed by the surface of the hair, which yields a sustained moisturizing effect to the hair after drying and a protective effect to the hair surface and makes it possible to suppress flyaway.

A compounded amount of the silicone gum is, for example, from 0.05 to 30 wt.% (mass%) and preferably from 1 to 15 wt.% (mass%) of the entire external use preparation or cosmetic composition. When the silicone gum is used as an emulsion composition prepared via a process of pre-emulsifying (including emulsion polymerization), the silicone gum can easily be compounded, and can be stably compounded in the hair cosmetic composition of the present invention. An effect of imparting a specific feeling to touch or glossiness of the hair may be insufficient if the compounded amount of the silicone gum is less than the lower limit described above.

### [Polyamide-modified Silicone]

Examples of polyamide-modified silicones that can be preferably compounded in the external use preparation or the cosmetic composition of the present invention include siloxane-based polyamide compounds described in US Patent No. 5,981,680 (Japanese Unexamined Patent Application Publication No. 2000-038450) and Japanese Unexamined Patent Application Publication (Translation of PCT Application) No. 2001-512164, and examples of commercially available products include 2-8178 Gellant, 2-8179 Gellant and the like (manufactured by Dow Corning USA). This polyamide-modified silicone also functions as an oil-based raw material, specifically as a thickening/gelling agent of a silicone oil.

When the polyamide-modified silicone is used in combination with the organopolysiloxane elastomer of the present invention, the external use preparation or the cosmetic composition of the present invention has good spreading and setting as well as an excellent sense of stability and adhesion when applied to the skin, the hair, or the like. Additionally, there are advantages from a quality standpoint such that a glossy feeling of sheerness and superior luster can be provided, the viscosity or hardness (softness) of the entire cosmetic composition containing the oil-based raw material can be appropriately adjusted, and an oily sensation (oily and sticky feeling to touch) can be totally suppressed. Furthermore, by using the organopolysiloxane elastomer, the dispersion stability of perfumes, powders, and the like will be improved. Therefore, the obtained cosmetic composition will be characterized by being able to maintain a uniform and fine cosmetic sensation for an extended period of time.

### [Silicone wax]

Silicone waxes that can be preferably compounded in the external use preparation or the cosmetic composition of the present invention are higher alkyl-modified silicones and alkyl-modified silicone resins. The higher alkyl-modified silicone is a wax at room temperature and is a component that is useful as a portion of the base material of a solid cosmetic composition (for example, an oil-based solid skin cosmetic composition or a solid hair cosmetic composition). Therefore, the higher alkyl-modified silicone can be preferably used in the external use preparation or the cosmetic composition of the present invention. Examples of the higher alkyl-modified silicone wax include methyl (long chain alkyl) polysiloxanes having both molecular terminals capped with trimethylsiloxy groups, copolymers of a dimethylpolysiloxane having both molecular terminals capped with trimethylsiloxy groups and a methyl (long chain alkyl) siloxane, dimethylpolysiloxane modified with long chain alkyls at both molecular terminals, and the like. Examples of commercially available products include AMS-C30 Cosmetic Wax, 2503 Cosmetic Wax, and the like (manufactured by Dow Corning Corporation, in the USA).

The organopolysiloxane elastomer of the present invention has good affinity with the higher alkyl-modified silicone wax and has excellent dispersibility properties in wax, so an external use preparation or a cosmetic composition having excellent storage stability over time can be obtained. In addition, the moldability of the external use preparation or the cosmetic composition, and particularly of a solid cosmetic composition, is excellent. In particular, in a system comprising a powder, an effect of uniformly and stably dispersing the powder in the base material containing the higher alkyl-modified silicone wax is obtained, and the hardness of the base material after molding can be moderately mitigated, which makes it possible to provide an external use preparation or a cosmetic composition that spreads smoothly and uniformly when applied.

In the external use preparation or cosmetic composition of the present invention, the higher alkyl-modified silicone wax preferably has a melting point of not lower than 60°C because such will lead to cosmetic retainability effects and stability at high temperatures.

The alkyl-modified silicone resin is a type of film-forming polymer that is compoundable in an oil phase and is a component that imparts sebum durability, moisturizing properties, and a fine tactile sensation of the skin to the external use preparation or the cosmetic composition. An alkyl-modified silicone resin that is in the form of a wax at room temperature can be suitably used. Preferred examples thereof include the silsesquioxane resin wax described in Japanese Unexamined Patent Application Publication (Translation of PCT Application) No. 2007-532754. Examples of commercially available products include SW-8005 C30 RESIN WAX (manufactured by Dow Corning Corporation, in the USA), and the like.

As in the case of a higher alkyl-modified silicone wax, the organopolysiloxane elastomer of the present invention has good affinity with the alkyl-modified silicone resin wax and has excellent dispersion properties in wax, so an external use preparation or a cosmetic composition having superior storage stability over time can be obtained. Further, an aqueous phase can be stably emulsified along with other optional surfactants in the oil phase containing the alkyl-modified silicone resin wax, which makes it possible to impart a luxurious tactile sensation, moisturizing effects, and effects of improving cosmetic retainability exemplified by moisture resistance and sebum resistance to the skin or the hair.

### [Antiperspiration active component and deodorant agent]

Additionally, in cases where the external use preparation or the cosmetic composition according to the present invention is an anti-perspirant, or depending on the purpose thereof, the external use preparation or the cosmetic composition can contain an anti-perspiration active component and/or a deodorant agent.

Examples of the anti-perspiration active component include astringent salts such as aluminum chlorohydrate, aluminum-zirconium tetrachlorohydrex glycine (ZAG), and the like; but aluminum, hafnium, zinc, and zirconium salts (e.g. aluminum halide, aluminum hydroxy halide, zirconium halide, zirconium oxyhalide, zirconium hydroxy halide, zirconyl hydroxide halide, aluminum chloride zirconium, zirconium lactate-aluminum, and basic aluminum halide) can be used. Examples thereof include Al₂(OH)₅Cl, aluminum bromide, buffer aluminum sulphate, alum, dried alum, various aqueous, alcohol, or glycine complexes thereof (e.g. a complex of an aluminum-zirconium chlorohydrate and glycine comprising aluminum, zirconium, and glycine (a ZAG complex), and the like. A single anti-perspiration active component may be used or a combination of two or more may be used. In cases where the anti-perspirant composition according to the present invention is a water-in-oil emulsion-type anti-perspirant composition, these anti-perspiration active components are an aqueous phase component. On the other hand, soybean extracts and isoflavones are known for their anti-perspirant effects; and, because they have low water solubility, are preferably used by dissolving them in the oil phase.

In the present invention, a compounded amount of the anti-perspiration active component is an amount sufficient to reduce perspiration, and suppressing the compounded amount to a small amount can be beneficial in personal care compositions. Specifically, from the standpoints of anti-perspirant effects and feeling to touch, the compounded amount of the anti-perspiration active component in an anti-perspirant composition is preferably from 5 to 25 wt.% (mass%) of the entire cosmetic composition. When using a water soluble anti-perspiration active component, from the standpoint of cost effectiveness, it is preferable to increase the proportion of water in the composition to a maximum limit, while maintaining anti-perspirant effects, but the anti-perspiration active component can also be added to the aqueous phase at amount near the saturation amount.

A deodorant agent can be compounded together with or instead of the anti-perspiration active component in the external use preparation or the cosmetic composition of the present invention, and particularly an antiperspiration composition. Examples of the deodorant agent include deodorizers, perfumes, and substances that prevent or remove odors caused by perspiration. Such deodorant agents are antimicrobial agents (germicides or fungicides), bacteriostatic agents, odor absorbing substances, deodorizers, perfumes, or the like, and are compounded for the purpose of preventing underarm odor, odor from perspiration, foot odor, and other bodily odors. Note that these deodorant agents are useful in cosmetic compositions or external use preparations other than anti-perspirants and it goes without saying that they can be beneficially compounded in the external use preparation or cosmetic composition of the present invention.

Examples of antimicrobial agents include alkyltrimethylammonium bromide, cetylpyridinium chloride, benzethonium chloride, benzalkonium chloride, chlorhexidine hydrochloride, chlorhexidine gluconate, [[(diisobutylphenoxy)ethoxy]ethyl] dimethylbenzylammonium chloride, N-lauroyl sarcosine sodium, N-palmitoyl sarcosine sodium, N-myristoyl glycine, N-lauroyl sarcosine potassium, trimethyl ammonium chloride, aluminum chlorohydroxy sodium lactate, triethyl citrate, tricetyl methyl ammonium chloride, 1,5-pentanediol, 1,6-hexanediol, 2,4,4'-trichloro-2'-hydroxy diphenylether (triclosan), and 3,4,4'-trichlorocarbanilide(triclocarban); L-lysine hexadecylamide and similar diaminoalkylamidos; citric acid, salicylic acid, piroctose, and other heavy metal salts, preferably zinc salts and acids thereof; pyrithione heavy metal salts, preferably pyrithione zinc, phenol zinc sulfate, ethylparaben, butylparaben, hinokitiol, farnesol, phenoxyethanol, isopropyl methylphenol, propolis, lysozyme, lysozyme chloride, combinations of lysozyme and vitamin E or derivatives thereof, combinations of organic acids such as lysozyme and α-hydroxyacid and the like; and the like.

Examples of bacteriostatic agents include 1-heptyl glyceryl ether, 1-(2-ethylhexyl)glyceryl ether, 1-octyl glyceryl ether, 1-decyl glyceryl ether, 1-dodecyl glyceryl ether, and similar glyceryl monoalkyl ethers.

The odor absorbing substance is not particularly limited, provided that it absorbs odor causing substances and reduces odor, is constituted by a portion of the inorganic powders and organic polymers described above, and displays the same characteristics.

Examples of the odor absorbing substance include zinc oxide, magnesium oxide, zeolite, aluminometasilicate, silicic anhydride, colloidal silica, talc, mica, hydroxyapatite, cellulose, corn starch, silk, nylon powder, crosslinking organopolysiloxane powder, organopolysiloxane elastomer spherical powder, and the like. Likewise, carbonates such as alkali metal carbonates, alkali metal bicarbonate salts, and the like and hydrogen carbonates, ammonium salts, tetraalkylammonium salts, and the like can be used. Of these odor absorbing substances, sodium salts and potassium salts are more preferable. Additionally, organic or inorganic porous particles carrying silver, copper, zinc, cerium, or similar metal ions (e.g. silver ion-carrying zeolite, silver ion/zinc ion/ammonium ion-carrying zeolite), or aggregates of needle-like crystals including silver cancrinite can be used. Because these function as antimicrobial agents and odor absorbing substances, they can be used beneficially as the deodorant agent.

Furthermore, hydroxyalkylated cyclodextrin, sake cake extract containing rice fermenting liquid, and various extracts derived from animals, vegetables, microorganisms, fungi, and the like such as brown seaweed extract, cinnamon bark, clove, fennel, ginger, mentha, citron, gentiana lutea, apricot, eucalyptus, Sophora flavescens, mulberry, althea, sage, Anthemis nobilis, Scutellaria root, nutgall, gardenia, hamamelis, herbs, and the like can be used as the deodorant agent. A part of these components overlaps with a bioactive component described above, but selecting these extracts as the deodorant agent for the purpose of the functional effects thereof is both beneficial and preferable from the standpoint of the composition design of the cosmetic composition.

Preferably from 0.001 to 60 wt.% (mass%), more preferably from 0.01 to 30 wt.% (mass%), and yet more preferably from 0.01 to 3 wt.% (mass%) of the odor absorbing substance is included in the entire composition. Provided that the compounded amount of the odor absorbing substance is within this range, there is an advantage that deodorizing performance can be improved while not negatively affecting the strength and feeling to touch of the formulation.

Suitable perfumes include known topical use substances, topical use substances that are effective in masking malodor accompanied by perspiration, and various topical use substances that provide a composition having a desired aroma. Examples thereof include the whole of perfumes and perfume chemicals such as perfume precursors, deodorizing fragrances, and the like that are suitable for topical application to the skin and, as necessary, may be a blended perfume component.

### [TECHNICAL FIELD]

The organopolysiloxane elastomer of the present invention or the composition containing the same can be suitably used as a raw material for an external use preparation or a raw material for a cosmetic composition. In particular, the organopolysiloxane elastomer of the present invention produced via an acidizing process and the composition of the present invention containing the organopolysiloxane elastomer of the present invention and at least one type of acid have a reduced odor and can therefore be suitably compounded in an external use preparation or a cosmetic composition.

Note that the carbonyl value measurement method described above is capable of accurately and simply assaying a carbonyl compound, so the method can be preferably used for the evaluation of the odor of a product of an external use preparation or a cosmetic composition.

### Practical Examples

The present invention is described below using examples, but the present invention is not limited thereto. In the following composition formulas, an Me₃SiO group (or an Me₃Si group) is represented by "M", an Me₃SiO group is represented by "D", an Me₂HSiO group (or an Me₂HSi group) is represented by "M^{H}", an MeHSiO group is represented by "D^{H}", an Me₂(CH₂=CH)SiO group [or an Me₂(CH₂=CH)Si group] is represented by "M^{Vi}", and an Me(CH₂=CH)SiO group is represented by "D^{Vi}". An Me₂[CH₂=CH-(CH₂)₄]SiO group [or an Me₂[CH₂=CH-(CH₂)₄]Si group] is represented by "M^{Hex}", an Me[CH₂=CH-(CH₂)₄]SiO group is represented by "D^{Hex}", an MeSiO_{3/2} group is represented by "T", and an MeSiO_{4/2} group is represented by "Q". Units in which the methyl groups in M and D are modified by one of the substituents are represented by M^{R} and D^{R}.

In addition, the xylitol monoallyl ether and xylitol residue described below are a raw material and functional groups as described in this specification. More specifically, the xylitol monoallyl ether is a raw material containing a xylitol monoallyl ether expressed by the structural formula: CH₂=CH-CH₂-OCH₂[CH(OH)]₃CH₂OH and the structural formula: CH₂=CH-CH₂-OCH[CH(OH)CH₂OH]₂ at a composition ratio of approximately 9:1, and a xylitol residue represented by the corresponding formula -C₃H₆-OCH₂[CH(OH)]₃CH₂OH or -C₃H₆-OCH[CH(OH)CH₂OH]₂ is introduced to the comodified silicone of the present invention at the same composition ratio.

Note that in the practical examples and comparative examples below, "Production of Silicone Compound No. X" and the like is included for the sake of convenience, and the obtained products are in the form of mixtures containing a small amount of unreacted raw material and the like in addition to the main components.

### [Practical Example 1] <Production of Silicone Compound No. 1>

First, 59.8 g of a methylhydrogenpolysiloxane expressed by the average composition formula: MD_{42.9}D^{H}_{6.7}M and 2.2 g of a 3-methacryloxypropyl(tris(trimethylsiloxy)silyl ethyl dimethylsiloxy)silane expressed by the following average composition formula: 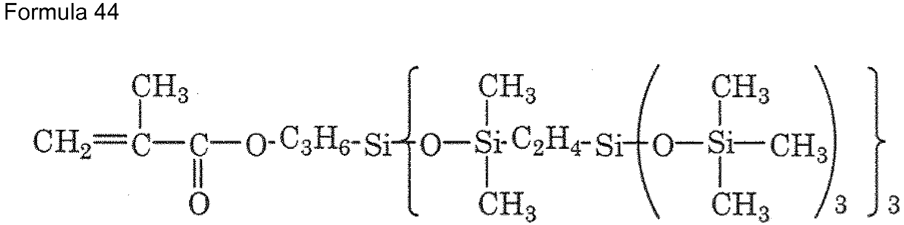 were placed in a reaction vessel and heated to 80°C while agitating under a nitrogen stream. Next, 0.12 mL of an isopropyl alcohol solution of a platinum-1,3-divinyl-1,1,3,3-tetramethyldisiloxane complex (Pt concentration: 0.45 wt.%) was added and the mixture was reacted for 2 hours at 80°C. A small amount of the reaction liquid was then sampled, and it was confirmed that the target reaction rate was reached by an alkali decomposition gas generation method (the remaining Si-H groups were decomposed using a KOH ethanol/aqueous solution, and the reaction rate was calculated from the volume of the generated hydrogen gas). Next, 6.3 g of hexadecene (α-olefin purity: 91.7%) was added to the reaction mixture, and after the mixture was reacted for 6 hours at 80°C, it was confirmed via the same method that the target reaction rate was reached. Next, 6.2 g of xylitol monoallyl ether (purity: 91.2%) and 138 g of isopropyl alcohol (IPA) were added to the reaction mixture, and 0.13 ml of the platinum catalyst described above was added. After reacting for 3 hours at 80°C, the mixture was sampled. As a result of calculating the reaction rate, it was found that a modified silicone intermediate expressed by the average composition formula: MD_{42.9}D^{R*31}_{0.1}D^{R*21}_{2.4}D^{R*11}_{1.6}D^{H}_{2.6}M had been produced. Here, R^{*11}, R^{*21}, and R^{*31} are as described below.
R^{*11}=-C₁₆H₃₃
R^{*21}=xylitol residue

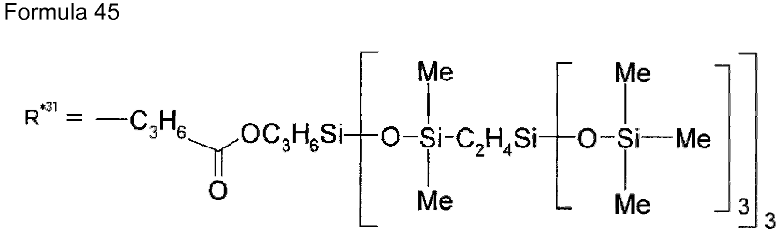

Here, 155.6 g of a methylvinylpolysiloxane expressed by the average composition formula: ^{Vi}MD₁₃₆D^{Vi}_{2.25}M^{Vi}, 0.025 g of natural vitamin E, and 0.14 ml of the platinum catalyst described above were added to the mixture. When a reaction was performed for 2.5 hours at 70 to 80°C, elastomerization occurred and the reaction mixture assumed a soft ricecake or jam-like consistency with a feeling of sheerness. In this case, the Vi/H molar ratio when crosslinking was 1.2. As a result of continuing the reaction for 2 more hours in this state in order to advance crosslinking, the hardness increased and the mixture became a dry, collapsible grease. Here, 450 g of a dimethylpolysiloxane (6 cst) serving as a diluent was added and mixed well.

Next, 3.5 g of a 0.12% phosphoric acid aqueous solution and 3.5 g of purified water were added, and after the mixture was treated for 1.5 hours at 60 to 80°C, the mixture was neutralized by adding 0.1 g of 2.5% ammonia water. A composition containing a novel organopolysiloxane elastomer modified by a xylitol group was then obtained by heating under reduced pressure to distill out low-boiling-point matter. The composition was then transferred to a Hobart mixture and subjected to kneading and grinding for 3 hours to obtain a homogenized, translucent or white, soft paste-like composition (elastomer concentration: 33%).

The average structural formula (schematic illustration) of the organopolysiloxane elastomer obtained in Practical Example 1 is illustrated below. 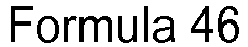 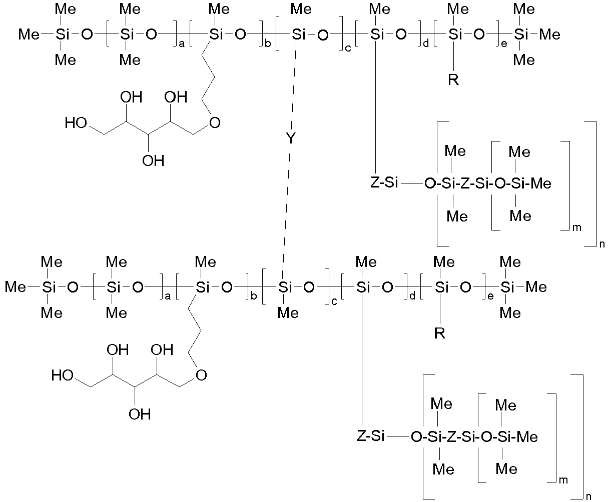 (wherein Me is a methyl group, Z in []n is -CH₂CH₂-, Z outside of []n is -C₃H₆-COO-C₃H₆-, R is -C₁₆H₃₃, Y is a linking group derived from ^{Vi}MD₁₃₆D^{Vi}_{2.25}M^{Vi}, a is 42.9, b is 2.4, c is 2.6, d is 0.1, e is 1.6, m is 3, and n is 3.)

### [Practical Example 2]<Production of Silicone Compound No. 2]>

First, 119.2 g of a methylhydrogenpolysiloxane expressed by the average composition formula: MD_{43.4}D^{H}_{7.4}M and 23.1 g of hexadecene (α-olefin purity: 91.7%) were placed in a reaction vessel, and when 0.10 ml of a hexamethyldisiloxane solution of a platinum-1,3-divinyl-1,1,3,3-tetramethyldisiloxane complex (Pt concentration: 0.45 wt. %) was added at 30°C while agitating under a nitrogen stream, heat was generated immediately, and the mixture heated to 60°C after three minutes. The temperature was then maintained at 30 to 60°C, and a small amount of the reaction solution was sampled after one hour. When the sample was confirmed using an alkali decomposition gas generation method, the reaction rate had reached the target rate. Next, 14.1 g of a xylitol monoallyl ether (purity: 85.3%), 138 g of isopropyl alcohol (IPA), and 0.03 g of natural vitamin E were added to the reaction mixture, and 0.03 ml of the platinum catalyst described above was then added. After a reaction was performed for 6 hours at 65 to 70°C, a small amount of the reaction solution was sampled. As a result of calculating the reaction rate with the same method, it was found that a modified silicone intermediate expressed by the average composition formula: MD_{43.4}D^{R*11}_{3.30}D^{R*21}_{1.98}D^{H}₂₁₂M had been produced. Here, R^{*11} and R^{*21} are as defined above.

Here, 73.9 g of a methylhexenylpolysiloxane expressed by the average composition formula: ^{Hex}MD_{56.7}D^{Hex}_{3.3} M^{Hex} was added to the mixture. When a reaction was performed at 70 to 80°C, elastomerization occurred after approximately 33 minutes, and the reaction mixture turned into a soft, greasy mass with a feeling of sheerness. In this case, the Vi/H molar ratio when crosslinking was 1.2. As a result of continuing the reaction for 2 more hours in this state in order to advance crosslinking, the hardness increased and the mixture became a dry, collapsible grease or powdered mass.

Next, 6.9 g of a 0.12% phosphoric acid aqueous solution and 3.5 g of purified water were added, and after the mixture was treated for 6 hours at 80 to 85°C, the mixture was neutralized by adding 0.20 g of 2.5% ammonia water. Next, 221 g of a novel organopolysiloxane elastomer modified by a xylitol group was obtained by heating under reduced pressure to distill out low-boiling-point matter. The aforementioned elastomer was then transferred to a Hobart mixer, and after the elastomer was subjected to kneading and grinding for one hour, the elastomer was subjected to kneading and grinding while slowly adding 409 g of a dimethylpolysiloxane (6 cst) serving as a diluent over the course of 8.5 hours to obtain a translucent or white, soft paste-like composition (elastomer concentration: 33%).

### [Practical Example 3] <Production of Silicone Compound No. 3>

First, 171.8 g of a methylhydrogenpolysiloxane expressed by the average composition formula: MD_{43.4}D^{H}_{7.4}M and 32.4 g of hexadecene (α-olefin purity: 91.7%) were placed in a reaction vessel, and when 0.10 ml of a hexamethyldisiloxane solution of a platinum-1,3-divinyl-1,1,3,3-tetramethyldisiloxane complex (Pt concentration: 0.45 wt.%) was added at 25°C while agitating under a nitrogen stream, heat was generated, and the mixture heated to 57°C after four minutes. The temperature was then maintained at 55 to 70°C, and a reaction was performed for two hours. A small amount of the reaction solution was sampled, and when the sample was confirmed using an alkali decomposition gas generation method, the reaction rate had reached the target rate. Next, 6.0 g of 3-methacryloxypropyl(tris(trimethylsiloxy)silylethyldimethylsiloxy)silane was added to the reaction mixture and reacted for 1.5 hours at 55 to 75°C, and it was confirmed by the same method that the reaction rate had reached the target rate. Next, 15.2 g of xylitol monoallyl ether (purity: 91.2%), 138 g of isopropyl alcohol (IPA), and 0.023 g of natural vitamin E were added to the reaction mixture, and after reacting for 2 hours at 60 to 75°C, a small amount of the reaction solution was sampled. As a result of calculating the reaction rate, it was found that a modified silicone intermediate expressed by the average composition formula: MD_{43.4}D^{R*31}₀₁₀D^{R*21}₁₆₀D^{R*11}₃₂₀D^{H}_{2.60}M had been produced. Here, R^{*11}, R^{*21}, and R^{*31} are as described above.

The mixture was temporarily cooled to 50°C, and 5.5 g of 5-hexadiene and 0.15 ml of the platinum catalyst described above were added to the mixture. When a reaction was performed for 2.5 hours at 50 to 60°C, elastomerization occurred, and the reaction mixture turned to a collapsible grease-like or paste-like substance. In this case, the Vi/H molar ratio when crosslinking was 1.2. As a result of continuing the reaction for 2 more hours in this state in order to advance crosslinking, the hardness increased and the mixture became a dry grease in large drops.

Next, 6.9 g of a 0.12% phosphoric acid aqueous solution and 3.5 g of purified water were added, and after the mixture was treated for 3.5 hours at 80°C, the low-boiling-point matter was removed by distillation while heating under reduced pressure to obtain 223 g of a novel organopolysiloxane elastomer modified by a xylitol group. The elastomer was then transferred to a Hobart mixer, and when the mixture was kneaded for 15 minutes, the mixture assumed the form of a white, light soft ricecake rolled up in the shape of a ball. The elastomer was then subjected to kneading while slowly adding 446 g of a dimethylpolysiloxane (6 cst) serving as a diluent over the course of 7 hours to obtain a translucent or transparent, homogeneous, soft ricecake-like composition (elastomer concentration: 33%).

### [Practical Example 4] <Production of Silicone Compound No. 4>

First, 168.9 g of a methylhydrogenpolysiloxane represented by the average composition formula: MD_{42.9}D^{H}_{6.7}M and 6.1 g of 3-methacryloxypropyl(tris(trimethylsiloxy)silylethyldimethylsiloxy)silane were placed in a reaction vessel, and 0.12 ml of an isopropyl alcohol solution of a platinum divinyl-1,1,3,3-tetramethyldisiloxane complex (Pt concentration: 0.45 wt.%) was added while agitating under a nitrogen stream. After reacting for 2 hours at 80°C, a small amount of the reaction liquid was sampled, and it was confirmed by an alkali decomposition gas generation method that the target reaction rate was reached. Next, 30.9 g of hexadecene (α-olefin purity: 91.7%) was added to the reaction mixture, and after the mixture was reacted for 3 hours at 90°C, it was confirmed via the same method that the target reaction rate was reached. Next, 9.5 g of a xylitol monoallyl ether (purity: 91.2%), 138 g of isopropyl alcohol (IPA), and 0.025 g of natural vitamin E were added to the reaction mixture, and 0.13 ml of the platinum catalyst described above was then added. After reacting for 3 hours at 80°C, the mixture was sampled. As a result of calculating the reaction rate, it was found that a modified silicone intermediate expressed by the average composition formula: MD_{42.9}D^{R*31}_{0.1}D^{R*21}_{1.2}D^{R*11}_{2.8}D^{H}_{2.6}M had been produced. Here, R^{*11}, R^{*21}, and R^{*31} are as described above.

Here, 14.6 g of a vinylcyclohexene monooxide expressed by the following structural formula: 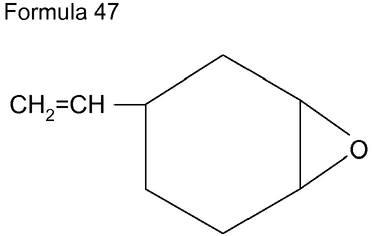 and 0.20 ml of the platinum catalyst described above were added to the mixture. When a reaction was performed for 3 hours at 80°C, elastomerization occurred, and the reaction mixture assumed a soft ricecake or jam-like consistency with a feeling of sheerness. In this case, the Vi/H molar ratio when crosslinking was 1.0. As a result of continuing the reaction for 2 more hours in this state in order to advance crosslinking, the hardness increased and the mixture became a dry, collapsible grease.

Next, 6.9 g of a 0.12% phosphoric acid aqueous solution and 3.5 g of purified water were added, and after the mixture was treated for 1 hour at 60 to 80°C, the low-boiling-point matter was removed by distillation while heating under reduced pressure to obtain a novel organopolysiloxane elastomer modified by a xylitol group. The composition was then transferred to a Hobart mixture and subjected to kneading and grinding for 3 hours to obtain a homogenized, white grease-like composition. The elastomer was then subjected to kneading while slowly adding 450 g of a dimethylpolysiloxane (6 cst) serving as a diluent over the course of 3 hours to obtain a translucent or white, soft paste-like composition (elastomer concentration: 33%).

### [Practical Example 5] <Production of Silicone Compound No. 5>

First, 140.4 g of a methylhydrogenpolysiloxane represented by the average composition formula: MD_{43.4}D^{H}_{7.4}M and 5.0 g of 3-methacryloxypropyl(tris(trimethylsiloxy)silylethyldimethylsiloxy)silane were placed in a reaction vessel, and 0.10 ml of a hexamethyldisiloxane solution of a platinum-1,3-divinyl-1,1,3,3-tetramethyldisiloxane complex (Pt concentration: 0.45 wt.%) was added while agitating under a nitrogen stream. After reacting for 2 hours at 60 to 65°C, a small amount of the reaction liquid was sampled, and it was confirmed by an alkali decomposition gas generation method that the target reaction rate was reached. Next, when 32.8 g of hexadecene (α-olefin purity: 91.7%) was added to the reaction mixture, heat was generated immediately, and the mixture heated to 95°C after two minutes. The mixture was then maintained at 60 to 80°C, and when a reaction was performed for 8.5 hours (0.10 ml of the aforementioned catalyst was added at an intermediate stage), it was found that the reaction rate had reached the target rate and that an intermediate expressed by the average composition formula: MD_{43.4}D^{R*31}_{0.1}D^{R*11}_{4.0}D^{H}_{2.98}D^{OR}_{0.32}M had been produced. Next, 2.3 g of xylitol monoallyl ether (purity: 91.2%), 138 g of isopropyl alcohol (IPA), and 0.03 g of natural vitamin E were added to the reaction mixture, and after a reaction was performed for 2 hours at 70°C, the mixture was sampled. As a result of calculating the reaction rate, it was found that a modified silicone intermediate expressed by the average composition formula: MD_{43.4}D^{R*31}_{0.1}D^{R*11}_{4.0}D^{R*21}_{0.31}D^{H}_{2.67}D^{OR}_{0.32}M had been produced. Here, R^{*11}, R^{*21}, and R^{*31} are as described above. In addition, D^{OR} is a structural unit produced by the dehydrogenation of D^{H} and an alcoholic hydroxyl group, the unit being an Me(OR)SiO group containing a Si-O-C bond or a Si-O-H bond. In this example, OR=O-CH(CH₃)₂ or OH.

Here, 44.7 g of a bisallylpolyether expressed by the average composition formula: CH₂=CH-CH₂-(OCH₂CH₂)₁₅-O-CH₂-CH=CH₂ and 0.10 ml of the platinum catalyst described above were added to the mixture. When a reaction was performed for 32 minutes at 75 to 80°C, elastomerization occurred, and the reaction mixture turned to a soft, grease-like composition that could be easily cut into thin strips. In this case, the Vi/H molar ratio when crosslinking was 1.2. As a result of continuing the reaction for 2 more hours in this state in order to advance crosslinking, the hardness increased slightly, and the composition assumed the form of a paste with a powdery appearance (and a tactile sensation similar to that of grains of boiled rice). Next, 6.9 g of a 0.12% phosphoric acid aqueous solution and 3.5 g of purified water were added, and after the mixture was treated for 3 hours at 80 to 85°C, the low-boiling-point matter was removed by distillation while heating under reduced pressure to obtain 219 g of a novel organopolysiloxane elastomer modified by a xylitol group. The elastomer was then transferred to a Hobart mixer, and when the mixture was kneaded for 20 minutes, the mixture assumed the form of a white, light soft ricecake rolled up in the shape of a ball. The elastomer was then subjected to kneading while slowly adding 438 g of a dimethylpolysiloxane (6 cst) serving as a diluent over the course of 8 hours to obtain a white, homogeneous, soft paste-like composition (elastomer concentration: 33%).

### [Practical Example 6] <Production of Silicone Compound No. 6>

First, 13.1 g of a methylhydrogenpolysiloxane expressed by the average composition formula: MD_{42.9}D^{H}_{6.7}M, 5.4 g of a methylhydrogenpolysiloxane expressed by the average composition formula: MD₇D^{H}₁₂T₁M, and 1.2 g of a methylhydrogenpolysiloxane expressed by the average composition formula: M^{H}₄Q were placed in a reaction vessel (the average composition formula of the mixture was M_{1.33}M^{H}_{1.33}D_{16.63}D^{H}_{6.23}T_{0.33}Q_{0.33}M). Next, 1.4 g of 3-methacryloxypropyl(tris(trimethylsiloxy)silylethyldimethylsiloxy)silane was added, and 0.12 g of an isopropyl alcohol solution of a platinum-1-3-divinyl-1,1,3,3-tetramethyldisiloxane complex (Pt concentration: 0.45 wt.%) was added while agitating under a nitrogen stream. After reacting for 2 hours at 90°C, a small amount of the reaction liquid was sampled, and it was confirmed by an alkali decomposition gas generation method that the target reaction rate was reached. Next, 6.8 g of hexadecene (α-olefin purity: 91.7%) was added to the reaction mixture, and after the mixture was reacted for 2 hours at 90°C, it was confirmed via the same method that the target reaction rate was reached. Next, 4.4 g of a xylitol monoallyl ether (purity: 91.2%), 138 g of isopropyl alcohol (IPA), and 0.025 g of natural vitamin E were added to the reaction mixture, and 0.13 g of the platinum catalyst described above was then added. After reacting for 1 hour at 80°C, the mixture was sampled, and the reaction rate was calculated. As a result, it was found that of the 1.33 M^{H} units and the 6.23 D^{H} units in the average molecular composition of methylhydrogenpolysiloxane, 0.1 equivalents were subjected to the addition of 3-methacryloxypropyl(tris(trimethylsiloxy)silylethyldimethylsiloxy)silane, 2.66 equivalents were subjected to the addition of 1-hexadecene, 2.0 equivalents were subjected to the addition of xylitol monoallyl ether, and the remaining M^{H} units and D^{H} units were equivalent to a total of 2.8 units.

Here, 197.7 g of a methylvinylpolysiloxane expressed by the average composition formula: ^{Vi}MD₁₄₉M^{Vi} and 0.14 g of the platinum catalyst described above were added to the mixture. When a reaction was performed for 2 hours at 80°C, elastomerization occurred, and the reaction mixture assumed a soft ricecake or jam-like consistency with a feeling of sheerness. In this case, the Vi/H molar ratio when crosslinking was 1.2. As a result of continuing the reaction for 2 more hours in this state in order to advance crosslinking, the hardness increased and the mixture became a dry, collapsible grease.

Next, 6.9 g of a 0.12% phosphoric acid aqueous solution and 3.5 g of purified water were added, and after the mixture was treated for 1 hour at 80°C, the low-boiling-point matter was removed by distillation while heating under reduced pressure to obtain a novel organopolysiloxane elastomer modified by a xylitol group. The composition was then transferred to a Hobart mixture and subjected to kneading and grinding for 3 hours to obtain a homogenized, white grease-like composition. The elastomer was then subjected to kneading while slowly adding 450 g of a dimethylpolysiloxane (6 cst) serving as a diluent over the course of 3 hours to obtain a translucent or white, soft paste-like composition (elastomer concentration: 33%).

### [Comparative Example 1] <Production of Silicone Compound No. RE-1>

First, 110.1 g of a methylhydrogenpolysiloxane represented by the average composition formula: MD₄₀D^{H}₁₅M and 12.1 g of 1-dodecene (equivalent to 1/4 the total amount of 1-dodecene added) were placed in a reaction vessel and heated to 50.0°C while agitating under a nitrogen air flow, and 0.10 g of an ethanol solution of chloroplatinic acid (Pt concentration: 3 wt.%) was then added. The temperature increased to 80°C due to the reaction heat, and after it was confirmed that the temperature dropped naturally thereafter, a second batch of 12.1 g of 1-dodecene was added and reacted. Third and fourth batches of 1-dodecene were further added and reacted with the same method. After reacting for 1 hour at 90 to 100°C, a small amount of the reaction liquid was sampled, and it was confirmed by an alkali decomposition gas generation method that the target reaction rate was reached. Next, low-boiling-point matter such as unreacted dodecene was removed by heating under reduced pressure.

Next, 25.4 g of a vinylmethylpolysiloxane expressed by the average composition formula: ^{vi}MD₆M and 0.10 g of the platinum catalyst described above were added to the reaction mixture, and after a reaction was performed for 2 hours at 90 to 100°C, it was confirmed that the reaction rate had reached the target rate with the same method. As a result of calculating the reaction rate, it was found that a modified silicone intermediate expressed by the average composition formula: MD₄₀D^{R*41}_{1.5}D^{R*12}₁₀D^{H}_{3.5}M had been produced. Here, R*12 and R*41 are as described below.
R^{*12}=-C₁₂H₂₅
R^{*41}= -C₂H₄SiMe₂(OSiMe₂)₆OSiMe₃

Here, 46.4 g of bisallylpolyether expressed by the average composition formula: CH₂=CH-CH₂-(OCH₂CH₂)₁₅-O-CH₂-CH=CH₂, 0.025 g of natural vitamin E, 138 g of toluene, and 0.10 g of the platinum catalyst described above were added to the mixture. When a reaction was performed for 2 hours at 90°C, elastomerization occurred, and the reaction mixture assumed a translucent, soft ricecake or jam-like consistency. In this case, the Vi/H molar ratio when crosslinking was 1.2. As a result of continuing the reaction for 2 more hours in this state in order to advance crosslinking, the hardness increased and the mixture became a dry, collapsible grease. Next, toluene was removed by heating under reduced pressure.

Next, 25.3 g of a 2% citric acid aqueous solution was added, and after the mixture was treated for 2 hours at 80°C, 2% baking soda water was added to neutralize the mixture for 1 hour. By distilling out low-boiling-point matter while heating under reduced pressure, an organopolysiloxane elastomer modified by polyether group, a C12 alkyl group, and a group having a linear polysiloxane structure was obtained. The composition was then transferred to a Hobart mixture and subjected to kneading and grinding for 3 hours to obtain a homogenized, white grease-like composition. The elastomer was then subjected to kneading while slowly adding 450 g of a dimethylpolysiloxane (6 cst) serving as a diluent over the course of 3 hours to obtain a translucent or white, soft paste-like composition (elastomer concentration: 33%).

### [Comparative Example 2]<Production of Silicone Compound No. RE-2>

First, 125.3 g of a methylhydrogenpolysiloxane represented by the average composition formula: MD₄₀D^{H}₁₅M and 13.7 g of 1-dodecene (equivalent to 1/4 the total amount of 1-dodecene added) were placed in a reaction vessel and heated to 50.0°C while agitating under a nitrogen air flow, and 0.10 g of an ethanol solution of chloroplatinic acid (Pt concentration: 3 wt.%) was then added. The temperature increased to 85°C due to the reaction heat, and after it was confirmed that the temperature dropped naturally thereafter, a second batch of 13.7 g of 1-dodecene was added and reacted. Third and fourth batches of 1-dodecene were further added and reacted with the same method. After reacting for 1 hour at 90 to 100°C, a small amount of the reaction liquid was sampled, and it was confirmed by an alkali decomposition gas generation method that the target reaction rate was reached. Next, low-boiling-point matter such as unreacted dodecene was removed by heating under reduced pressure.

Next, 28.9 g of a vinylmethylpolysiloxane expressed by the average composition formula: ^{Vi}MD₆M and 0.10 g of the platinum catalyst described above were added to the reaction mixture, and after a reaction was performed for 2 hours at 90 to 100°C, it was confirmed that the reaction rate had reached the target rate with the same method. As a result of calculating the reaction rate, it was found that a modified silicone intermediate expressed by the average composition formula: MD₄₀D^{R*41}_{1.5}D^{R*12}₁₀D^{H}_{3.5}M had been produced. Here, R^{*12} and R^{*41} are as defined above.

Here, 20.9 g of bisallyltriglycerine (or triglycerine allyl ether) expressed by the average composition formula: CH₂=CH-CH₂O-(C₃H₆O₂)₃-CH₂-CH=CH₂, 0.025 g of natural vitamin E, 138 g of toluene, and 0.10 g of the platinum catalyst described above were added to the mixture. When a reaction was performed for 2 hours at 80°C, elastomerization occurred, and the reaction mixture assumed a translucent, soft ricecake or jam-like consistency. In this case, the Vi/H molar ratio when crosslinking was 1.2. As a result of continuing the reaction for 2 more hours in this state in order to advance crosslinking, the hardness increased and the mixture became a dry, collapsible grease. Next, toluene was removed by heating under reduced pressure.

Next, 25.3 g of a 2% citric acid aqueous solution was added, and after the mixture was treated for 2 hours at 80°C, 2% baking soda water was added to neutralize the mixture for 1 hour. By distilling out low-boiling-point matter while heating under reduced pressure, an organopolysiloxane elastomer modified by triglycerine group, a C12 alkyl group, and a group having a linear polysiloxane structure was obtained. The composition was then transferred to a Hobart mixture and subjected to kneading and grinding for 3 hours to obtain a homogenized, white grease-like composition. The elastomer was then subjected to kneading while slowly adding 450 g of a dimethylpolysiloxane (6 cst) serving as a diluent over the course of 3 hours to obtain a translucent or white, soft paste-like composition (elastomer concentration: 33%).

### Practical Examples 7 to 12 and Comparative Examples 3 and 4

The sensation during use was evaluated for the paste-like compositions obtained in Practical Examples 1 to 6 and Comparative Examples 1 and 2 (elastomer concentration: 33%) in accordance with the following procedure and evaluation criteria. The results are shown in Table 2. In the table, "parts" indicates "parts by weight (mass)".

### [Method of functional evaluation (sensation during use)]

1. 0.20 g of the paste-like composition was placed on a finger and spread over the back of the hand.
2. At this time, the tactile sensation at the time of application and during application and the sensation on the skin after application were evaluated.

### [Functional evaluation criteria]

"Tactile sensation: at the time of application and during application"
⊚: The composition has excellent elastomeric, soft elasticity and spreads smoothly. The composition is characterized by a smoothness with a velvet-like thickness.
○: The composition has elastomeric, soft elasticity, spreads smoothly, and has a unique thick feel.
Δ: The spreadability is good and the sensation during use is smooth and natural when first applied, but there is a conspicuous stickiness and gooeyness while applied to the skin. The thick feel unique to elastomers is also minimal.
×: There is no problem with spreadability, but there is a strong, oily stickiness and gooeyness from when the composition is first applied until the end of the test. The thick feel unique to elastomers is also minimal.

### "Sensation on the skin: after application"

⊚: Skin compatibility is good, and a favorable moisturizing feel is also achieved. In addition, the oiliness is suppressed, so an extremely natural sensation on the skin with no discomfort in terms of appearance can be obtained.
○: Skin compatibility is good, and a favorable moisturizing feel is also achieved. In addition, the oiliness is suppressed, and there is practically no discomfort.
Δ: The moisturizing feel is reduced, and there is a slightly stronger oily tactile sensation.
×: Oily gooeyness is strong, and there is a feeling of warmth, which is disagreeable.

Table 2

**Table 2: Formulations and evaluation results of the mixtures (compositions) with oil agents (Practical Examples 7 to 12 and Comparative Examples 3 and 4)**

| Name of raw material | Practical Examples | | | | | | Comparative Examples | |
|---|---|---|---|---|---|---|---|---|
| | 7 | 8 | 9 | 10 | 11 | 12 | 3 | 4 |
| Paste-like composition of Practical Example 1 | 100 | - | - | - | - | - | - | - |
| Paste-like composition of Practical Example 2 | - | 100 | - | - | - | - | - | - |
| Paste-like composition of Practical Example 3 | - | - | 100 | - | - | - | - | - |
| Paste-like composition of Practical Example 4 | - | - | - | 100 | - | - | - | - |
| Paste-like composition of Practical Example 5 | - | - | - | - | 100 | - | - | - |
| Paste-like composition of Practical Example 6 | - | - | - | - | - | 100 | - | - |
| Paste-like composition of Comparative Example 1 | - | - | - | - | - | - | 100 | - |
| Paste-like composition of Comparative Example 2 | - | - | - | - | - | - | - | 100 |
| Tactile sensation (at the time of application and during application) | ⊚ | ⊚ | ⊚ | ○ | ⊚ | ○ | × | Δ |
| Sensation on the skin (after application) | ⊚ | ⊚ | ⊚ | ○ | ○ | ○ | × | × |

### Practical Examples 13 to 18 and Comparative Examples 5 and 6

In accordance with the following procedure, the paste-like compositions and mixtures with oil agents obtained in Practical Examples 1 to 6 and Comparative Examples 1 and 2 were prepared as prescribed by the formulations shown in Table 3 (elastomer concentration: 10%) and Table 4 (elastomer concentration: 5%). In addition, the viscosities of the resulting compositions were measured, and the thickening/gelling effect of the oil agent of the organopolysiloxane elastomer having a sugar alcohol-modified group of the present invention was confirmed. The results are shown in Tables 3 and 4. In the table, "parts" indicates "parts by weight (mass)".

### [Preparation method for mixture with oil agent]

1. The paste-like composition was placed in a Hobart mixer, and an oil agent was dripped into the composition while mixing over the course of 5 minutes to form a uniform composition.
2. The viscosity of the mixture was measured under conditions at 25°C.

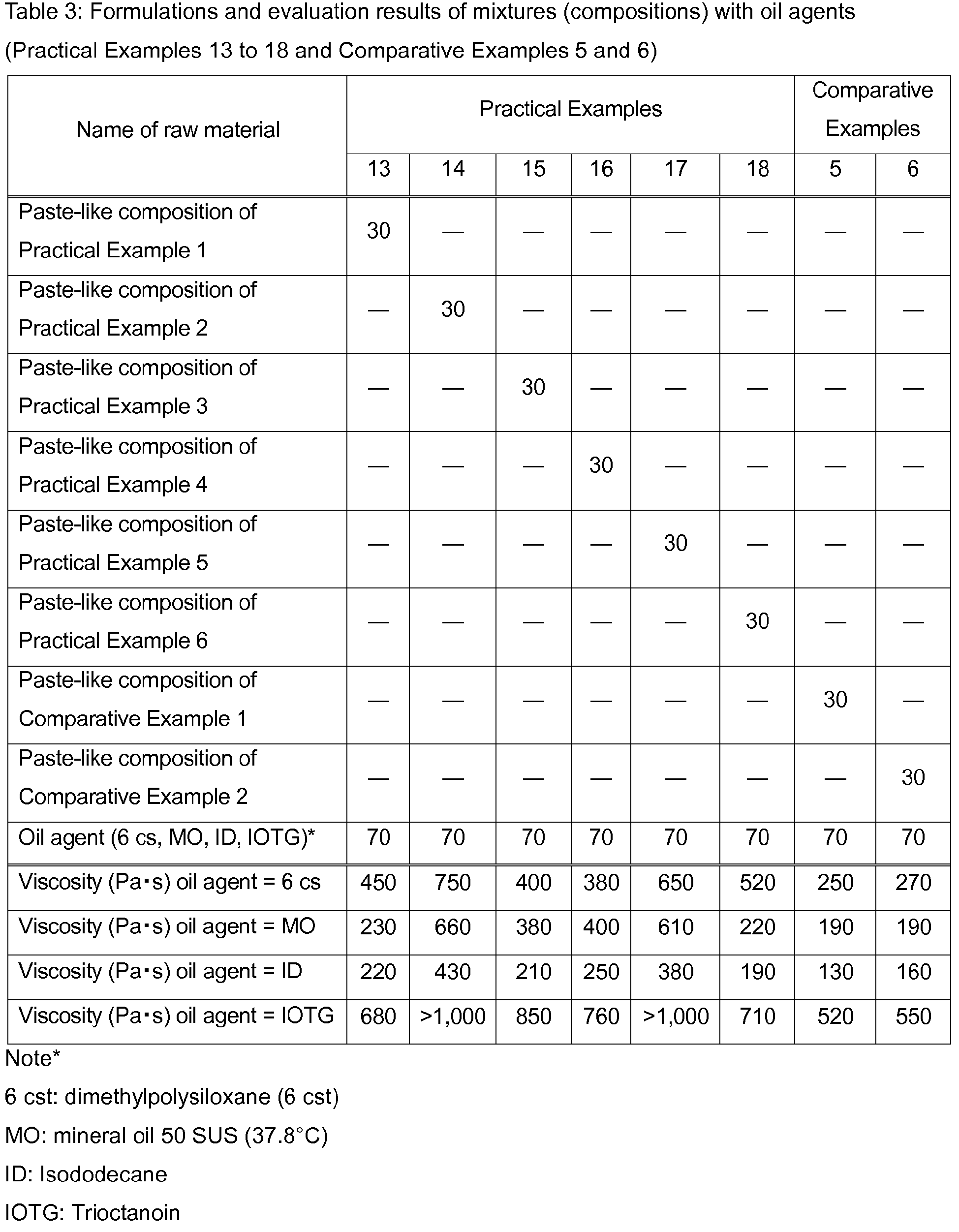

Table 4

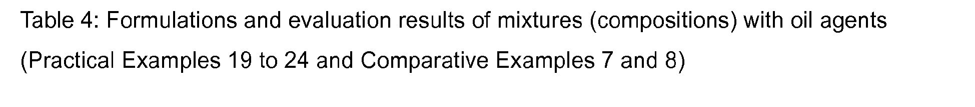

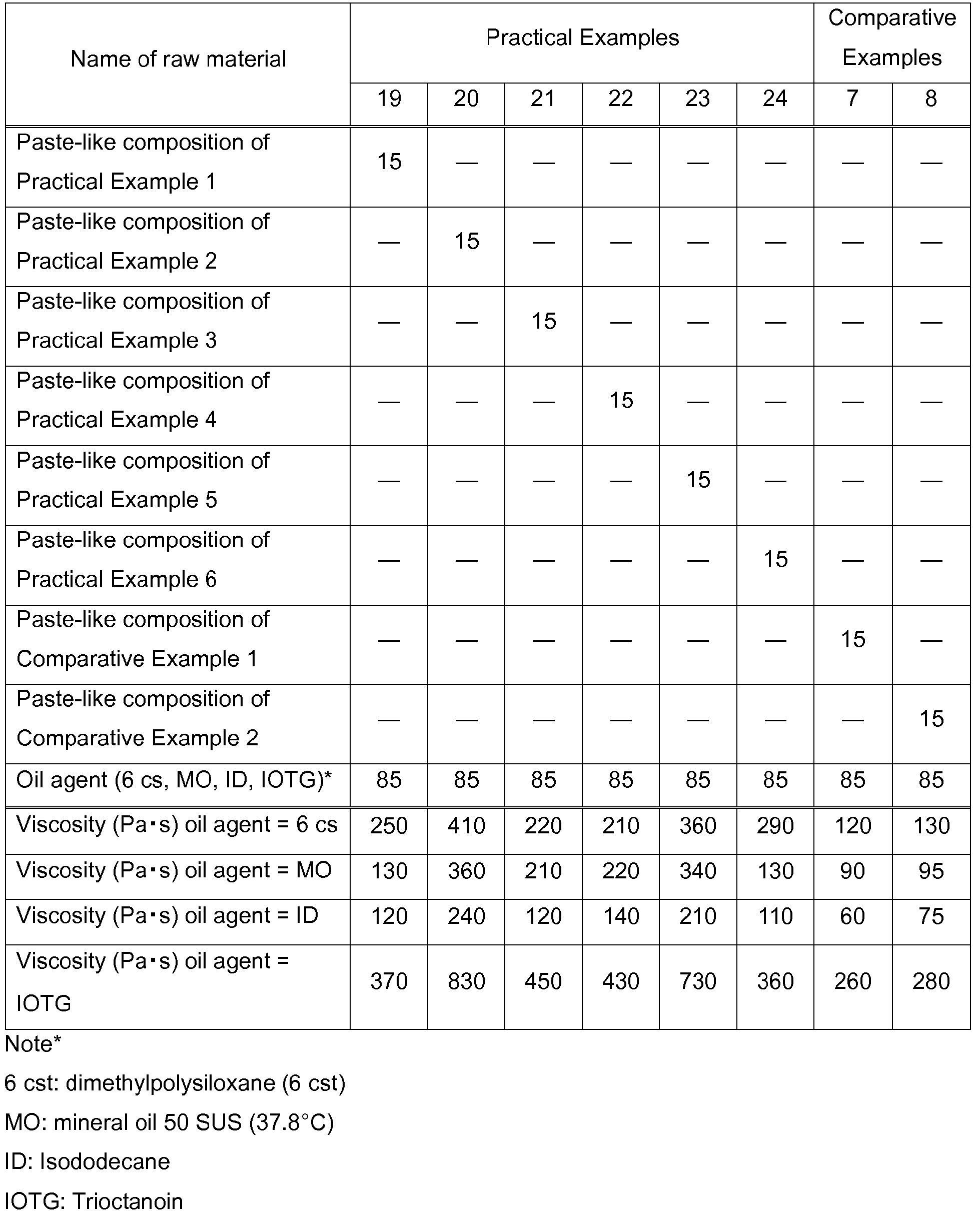

It was found from the above results that the organopolysiloxane elastomer having a sugar alcohol-modified group of the present invention has excellent affinity with various oil agents and that the elastomer can efficiently and stably thicken or gelify various oil agents. In contrast, although also depending on the type of oil agent used, the conventional hydrophilic organopolysiloxane elastomers used in the comparative examples required that a large amount of the elastomer be compounded in order to demonstrate clear effects as an oil thickening agent, and there was a problem from the perspective of the efficiency of thickening or gelification.

### Practical Examples 25 to 60 and Comparative Examples 9 to 20

Using the paste-like compositions obtained in Practical Examples 1 to 6 and Comparative Examples 1 and 2, water-in-oil emulsion compositions having the formulations shown in Tables 5 to 9 were prepared in accordance with the following procedure. These compositions were evaluated in terms of the functionality (tactile sensation and sensation during use), aesthetic appearance and form stability, emulsified particle size stability, and odor over time according to the evaluation criteria below. The results are shown in Tables 5 to 9. In the table, "parts" indicates "parts by weight (mass)".

### Preparation method for water-in-oil emulsion composition

1. The paste-like composition was placed in the pot of a Hobart mixer.
2. An oil agent was dropped into the mixture while agitating over the course of 5 minutes to obtain a uniform mixture (oil phase A).
3. Table salt and ion exchanged water were placed in a separate container. The salt was dissolved by mixing using a spatula. Furthermore, 1,3-butylene glycol was mixed and dissolved therein (aqueous phase B).
4. An aqueous phase B was dropped into the oil phase A over the course of 10 minutes while agitating the oil phase A with the Hobart mixer.
5. Agitation was halted after agitating for two more minutes. The oily component and water droplets adhering to the inner wall of the container were then scraped off using a spatula and mixed with the produced emulsion.
6. The mixture was further agitated for 3 minutes with the Hobart mixer.

### Functionality evaluation (tactile sensation and sensation during use)

The sensation during use at the time of application, during application, and after application when using each water-in-oil emulsion composition as a cosmetic composition was evaluated. Here, all of the samples were at a level that was not problematic in terms of spreadability and pure smoothness, so evaluations were performed with focus on the following points. Also, relative comparisons were carried out within groups that used a common oil agent. Specifically:
1. 0.20 g of the water-in-oil emulsion composition was placed on a finger and spread on the back of the hand.
2. At this time, the watery tactile sensation and the thick smoothness unique to elastomers at the time of application and during application were evaluated, and the imparting of a moisturizing feeling and the suppression of oiliness after application were evaluated in accordance with the following criteria.

"Watery tactile sensation and thick smoothness unique to elastomers: at the time of application and during application"
⊚: The composition has an extremely watery tactile sensation, and the unique thick and smooth tactile sensation is ongoing.
○: A watery tactile sensation is achieved, and the unique thick and smooth tactile sensation is ongoing.
Δ: A watery tactile sensation is achieved, but smoothness and thickness are not perceived strongly. ×: A slightly thick smoothness is perceived, but the composition otherwise has the feel of a common cream when applied, and practically no watery tactile sensation is perceived. (Cases where the emulsifying itself was not possible are also indicated as "×")

"Imparting of a moisturizing feel and suppression of oiliness after application: sensation on the skin after application"
⊚: Skin compatibility is good, and a favorable moisturizing feel is also achieved. In addition, the oiliness is suppressed, so an extremely natural sensation during use with no discomfort in terms of appearance can be obtained.
○: Skin compatibility is good, and a natural sensation on the skin is achieved due to a favorable moisturizing feel, but there is a slight degree of oily shininess.
Δ: Although the composition has a moisturizing feeling, the oiliness and shininess are troubling, and there is some discomfort with regard to the sensation on the skin.
×: The composition has no moisturizing feeling and has strong oily gooeyness and shininess. (Cases where the emulsifying itself was not possible are also indicated as "×")

[Evaluation of emulsion appearance and form] The appearance and form were observed and evaluated in accordance with the following criteria on the day that each water-in-oil emulsion composition was prepared and after the emulsion composition (28 g allocated into a 35 ml glass vial and hermetically sealed) was left to stand for 2 weeks at 50°C.
⊚: The entire mixture is uniform and has a cream or gel-like form with a matte texture.
○: Most of the mixture is uniform and has a cream or gel-like form with a matte texture. Slight deposits may be present.
Δ: The surface roughness of the emulsion can be easily seen, and there is a somewhat large amount of unevenness and deposits.
×: Emulsification itself is not complete, and phase separation is observed or the surface is clearly uneven.

Measurement of emulsified particle size and evaluation of stability One day after preparing the water-in-oil emulsion compositions, and after allowing the emulsion compositions (the capped 35 mL glass bottles containing 28 g of each water-in-oil emulsion composition, as described above) to sit at rest at 50°C for two weeks, the compositions were observed (1.000X) and photographed using an optical microscope, and the distribution range of the particle sizes was visually determined. Thereby, stability was evaluated by examining the initial emulsified particle size and the emulsified particle size over time.
Note that notes were made in the tables when particle coalescence was observed.
⊚: Change in emulsified particle size was small, and signs of coalescence were absent.
○: The emulsified particle size potentially increased slightly but definite coalescence was not observed. Alternatively, the emulsified particle size increased, but the overall particle size was small and the emulsion system was maintained.
Δ: It is thought that partial coalescence of the particles occurred. Definite increase in the emulsified particle size.
×: Many particles were coalesced and emulsion was in the state of breaking down. (Cases where the emulsifying itself was not possible are also indicated as "×")

### [Evaluation of emulsion odor over time]

After allowing the emulsion compositions (the capped 35 mL glass bottles containing 28 g of each water-in-oil emulsion composition, as described above) to sit at rest at 50°C for two weeks, the compositions were removed and returned to room temperature. The bottles were opened on the next day, and the odor produced was evaluated in accordance with the following criteria. Note that relative comparisons were carried out within groups that used a common oil agent.
⊚: Level with practically no noticeable odor.
○: Slight odor (sweet solution-like specific odor) is perceived.
Δ: Moderate odor (sweet solution-like specific odor) is perceived.
×: Strong odor (sweet solution-like specific odor) is perceived.

Table 5

**Table 5: Formulations and evaluation results of the water-in-oil emulsion compositions (Practical Examples 25 to 30 and Comparative Examples 9 and 10)**

| Name of raw material | Practical Examples | | | | | | Comparative Examples | |
|---|---|---|---|---|---|---|---|---|
| | 25 | 26 | 27 | 28 | 29 | 30 | 9 | 10 |
| Paste-like composition of Practical Example 1 | 6.7 | - | - | - | - | - | - | - |
| Paste-like composition of Practical Example 2 | - | 6.7 | - | - | - | - | - | - |
| Paste-like composition of Practical Example 3 | - | - | 6.7 | - | - | - | - | - |
| Paste-like composition of Practical Example 4 | - | - | - | 6.7 | - | - | - | - |
| Paste-like composition of Practical Example 5 | - | - | - | - | 6.7 | - | - | - |
| Paste-like composition of Practical Example 6 | - | - | - | - | - | 6.7 | - | - |
| Paste-like composition of Comparative Example 1 | - | - | - | - | - | - | 6.7 | - |
| Paste-like composition of Comparative Example 2 | - | - | - | - | - | - | - | 6.7 |
| Dimethylpolysiloxane (6 cst) | 18.3 | 18.3 | 18.3 | 18.3 | 18.3 | 18.3 | 18.3 | 18.3 |
| Sodium chloride | 0.5 | 0.5 | 0.5 | 0.5 | 0.5 | 0.5 | 0.5 | 0.5 |
| Purified water | 68.5 | 68.5 | 68.5 | 68.5 | 68.5 | 68.5 | 68.5 | 68.5 |
| 1,3-butylene glycol | 6 | 6 | 6 | 6 | 6 | 6 | 6 | 6 |
| Sensation during use (at the time of application until after application) | ⊚ | ⊚ | ⊚ | ⊚ | ⊚ | ⊚ | × | Δ |
| Sensation during use (senation on the skin) | ⊚ | ⊚ | ⊚ | ⊚ | ○ | ⊚ | × | ○ |
| Appearance and form (initial) | ⊚ | ⊚ | ⊚ | ⊚ | ⊚ | ○ | ⊚ | ⊚ |
| Appearance and form (over time) | ⊚ | ⊚ | ⊚ | ⊚ | ⊚ | ○ | ⊚ | ○ |
| Initial particle size distribution (µm) | 3-11 | 3-15 | 3-12 | 3-10 | 1-7 | 3-10 | 1-5.2 | 2-11 Pass |
| Particle size distribution over time (µm) | 3-11 | 3-14 | 3-12 | 3-11 | 1-8 | 3-11 | 1-8 | 2-20 Pass |
| Stability of emulsified particles | ⊚ | ⊚ | ⊚ | ⊚ | ⊚ | ⊚ | ○ | Δ |
| Odor of emulsion over time | ○ | ○ | ○ | ○ | ○ | ○ | ○ | ○ |

Table 6

**Table 6: Formulations and evaluation results of the water-in-oil emulsion compositions (Practical Examples 31 to 36 and Comparative Examples 11 and 12)**

| | Practical Examples | | | | | | Comparative Examples | |
|---|---|---|---|---|---|---|---|---|
| Name of raw material | 31 | 32 | 33 | 34 | 35 | 36 | 11 | 12 |
| Paste-like composition of Practical Example 1 | 6.7 | - | - | - | - | - | - | - |
| Paste-like composition of Practical Example 2 | - | 6.7 | - | - | - | - | - | - |
| Paste-like composition of Practical Example 3 | - | - | 6.7 | - | - | - | - | - |
| Paste-like composition of Practical Example 4 | - | - | - | 6.7 | - | - | - | - |
| Paste-like composition of Practical Example 5 | - | - | - | - | 6.7 | - | - | - |
| Paste-like composition of Practical Example 6 | - | - | - | - | - | 6.7 | - | - |
| Paste-like composition of Comparative Example 1 | - | - | - | - | - | - | 6.7 | - |
| Paste-like composition of Comparative Example 2 | - | - | - | - | - | - | - | 6.7 |
| Mineral oil 50SUS (37.8°C) | 18.3 | 18.3 | 18.3 | 18.3 | 18.3 | 18.3 | 18.3 | 18.3 |
| Sodium chloride | 0.5 | 0.5 | 0.5 | 0.5 | 0.5 | 0.5 | 0.5 | 0.5 |
| Purified water | 68.5 | 68.5 | 68.5 | 68.5 | 68.5 | 68.5 | 68.5 | 68.5 |
| 1,3-butylene glycol | 6 | 6 | 6 | 6 | 6 | 6 | 6 | 6 |
| Sensation during use (at the time of application until after application) | ⊚ | ⊚ | ⊚ | ⊚ | ⊚ | ⊚ | × | Δ |
| Sensation during use (senation on the skin) | ⊚ | ⊚ | ⊚ | ⊚ | ○ | ⊚ | × | ○ |
| Appearance and form (initial) | ⊚ | ⊚ | ⊚ | ⊚ | ⊚ | ○ | ⊚ | ⊚ |
| Appearance and form (over time) | ⊚ | ⊚ | ⊚ | ⊚ | ⊚ | ○ | ⊚ | ○ |
| Initial particle size distribution (µm) | 2-8 | 2-11 | 2-10 | 2-10 | 1-6 | 2-9 | 2-7 | 2-30 Pass |
| Particle size distribution over time (µm) | 2-10 | 2-10 | 2-11 | 2-10 | 1-6 | 2-11 | 2-10 | 4-30 Pass |
| Stability of emulsified particles | ○ | ⊚ | ⊚ | ⊚ | ⊚ | ○ | ○ | Δ |
| Odor of emulsion over time | ○ | ○ | ○ | ○ | ○ | ○ | ○ | ○ |

Table 7

**Table 7: Formulations and evaluation results of the water-in-oil emulsion compositions (Practical Examples 37 to 42 and Comparative Examples 13 and 14)**

| Name of raw material | Practical Examples | | | | | | Comparative Examples | |
|---|---|---|---|---|---|---|---|---|
| | 37 | 38 | 39 | 40 | 41 | 42 | 13 | 14 |
| Paste-like composition of Practical Example 1 | 6.7 | - | - | - | - | - | - | - |
| Paste-like composition of Practical Example 2 | - | 6.7 | - | - | - | - | - | - |
| Paste-like composition of Practical Example 3 | - | - | 6.7 | - | - | - | - | - |
| Paste-like composition of Practical Example 4 | - | - | - | 6.7 | - | - | - | - |
| Paste-like composition of Practical Example 5 | - | - | - | - | 6.7 | - | - | - |
| Paste-like composition of Practical Example 6 | - | - | - | - | - | 6.7 | - | - |
| Paste-like composition of Comparative Example 1 | - | - | - | - | - | - | 6.7 | - |
| Paste-like composition of Comparative Example 2 | - | - | - | - | - | - | - | 6.7 |
| Isododecane | 18.3 | 18.3 | 18.3 | 18.3 | 18.3 | 18.3 | 18.3 | 18.3 |
| Sodium chloride | 0.5 | 0.5 | 0.5 | 0.5 | 0.5 | 0.5 | 0.5 | 0.5 |
| Purified water | 68.5 | 68.5 | 68.5 | 68.5 | 68.5 | 68.5 | 68.5 | 68.5 |
| 1,3-butylene glycol | 6 | 6 | 6 | 6 | 6 | 6 | 6 | 6 |
| Sensation during use (at the time of application until after application) | ⊚ | ⊚ | ⊚ | ⊚ | ⊚ | ⊚ | × | × |
| Sensation during use (senation on the skin) | ⊚ | ⊚ | ⊚ | ⊚ | ⊚ | ⊚ | × | × |
| Appearance and form (initial) | ⊚ | ⊚ | ⊚ | ⊚ | ⊚ | ○ | ⊚ | × |
| Appearance and form (over time) | ⊚ | ⊚ | ⊚ | ⊚ | ⊚ | ○ | ⊚ | × |
| Initial particle size distribution (µm) | 2-10 | 3-13 | 3-13 | 3-11 | 2-9 | 3-12 | 2-9 | - |
| Particle size distribution over time (µm) | 2-10 | 3-12 | 3-13 | 3-11 | 2-7 | 3-13 | 2-10 | - |
| Stability of emulsified particles | ⊚ | ⊚ | ⊚ | ⊚ | ⊚ | ○ | ○ | × |
| Odor of emulsion over time | ⊚ | ⊚ | ⊚ | ⊚ | ⊚ | ⊚ | ⊚ | - |

Table 8

**Table 8: Formulations and evaluation results of the water-in-oil emulsion compositions (Practical Examples 43 to 48 and Comparative Examples 15 and 16)**

| Name of raw material | Practical Examples | | | | | | Comparative Examples | |
|---|---|---|---|---|---|---|---|---|
| | 43 | 44 | 45 | 46 | 47 | 48 | 15 | 16 |
| | | | | | | | | |
| Paste-like composition of Practical Example 1 | 6.7 | - | - | - | - | - | - | - |
| Paste-like composition of Practical Example 2 | - | 6.7 | - | - | - | - | - | - |
| Paste-like composition of Practical Example 3 | - | - | 6.7 | - | - | - | - | - |
| Paste-like composition of Practical Example 4 | - | - | - | 6.7 | - | - | - | - |
| Paste-like composition of Practical Example 5 | - | - | - | - | 6.7 | - | - | - |
| Paste-like composition of Practical Example 6 | - | - | - | - | - | 6.7 | - | - |
| Paste-like composition of Comparative Example 1 | - | - | - | - | - | - | 6.7 | - |
| Paste-like composition of Comparative Example 2 | - | - | - | - | - | - | - | 6.7 |
| Trioctanoin | 18.3 | 18.3 | 18.3 | 18.3 | 18.3 | 18.3 | 18.3 | 18.3 |
| Sodium chloride | 0.5 | 0.5 | 0.5 | 0.5 | 0.5 | 0.5 | 0.5 | 0.5 |
| Purified water | 68.5 | 68.5 | 68.5 | 68.5 | 68.5 | 68.5 | 68.5 | 68.5 |
| 1,3-butylene glycol | 6 | 6 | 6 | 6 | 6 | 6 | 6 | 6 |
| Sensation during use (at the time of application until after application) | ⊚ | ⊚ | ⊚ | ⊚ | ⊚ | ⊚ | × | × |
| Sensation during use (senation on the skin) | ⊚ | ⊚ | ⊚ | ⊚ | ⊚ | ⊚ | × | × |
| Appearance and form (initial) | O | ⊚ | ⊚ | ⊚ | ⊚ | O | ⊚ | ⊚ |
| Appearance and form (over time) | O | ⊚ | ⊚ | ⊚ | ⊚ | O | ⊚ | O |
| Initial particle size distribution (µm) | 1-6 | 1-5 | 1-4 | 1-4 | 1-4 | 1-6 | 1-5 | 3-30 Pass |
| Particle size distribution over time (µm) | 1-8 | 1-5 | 1-4 | 1-4 | 1-4 | 1-8 | 1-7 | 3-30 Pass |
| Stability of emulsified particles | ⊚ | ⊚ | ⊚ | ⊚ | ⊚ | O | O | Δ |
| Odor of emulsion over time | O | O | O | O | O | O | O | O |

Table 9

**Table 9: Formulations and evaluation results of the water-in-oil emulsion compositions (Practical Examples 49 to 52 and Comparative Examples 17 and 18)**

| Name of raw material | Practical Examples | | | | Comparative Examples | |
|---|---|---|---|---|---|---|
| | 49 | 50 | 51 | 52 | 17 | 18 |
| Paste-like composition of Practical Example 2 | 6.7 | - | - | - | - | - |
| Paste-like composition of Practical Example 3 | - | 6.7 | - | - | - | - |
| Paste-like composition of Practical Example 4 | - | - | 6.7 | - | - | - |
| Paste-like composition of Practical Example 5 | - | - | - | 6.7 | - | - |
| Paste-like composition of Comparative Example 1 | - | - | - | - | 6.7 | - |
| Paste-like composition of Comparative Example 2 | - | - | - | - | - | 6.7 |
| Sunflower oil | 9.15 | 9.15 | 9.15 | 9.15 | 9.15 | 9.15 |
| Mineral oil 50SUS (37.8°C) | 9.15 | 9.15 | 9.15 | 9.15 | 9.15 | 9.15 |
| Sodium chloride | 0.5 | 0.5 | 0.5 | 0.5 | 0.5 | 0.5 |
| Purified water | 68.5 | 68.5 | 68.5 | 68.5 | 68.5 | 68.5 |
| 1,3-butylene glycol | 6 | 6 | 6 | 6 | 6 | 6 |
| Sensation during use (at the time of application until after application) | ⊚ | ⊚ | ⊚ | ⊚ | × | × |
| Sensation during use (senation on the skin) | ⊚ | ⊚ | ⊚ | ⊚ | × | × |
| Appearance and form (initial) | ⊚ | ⊚ | ⊚ | ⊚ | ⊚ | × |
| Appearance and form (over time) | ⊚ | ⊚ | ⊚ | ⊚ | O | × |
| Initial particle size distribution (µm) | 1-5 | 1-5 | 1-5 | 1-6 | 1-6 | - |
| Particle size distribution over time (µm) | 1-5 | 1-5 | 1-5 | 1-5 | 2-9 | - |
| Stability of emulsified particles | ⊚ | ⊚ | ⊚ | ⊚ | Δ | × |
| Odor of emulsion over time | O | O | O | O | O | - |

Based on the above results, it was found that by using ground particles of the sugar alcohol-modified organopolysiloxane elastomer of the present invention as an emulsifier for a water-in-oil emulsion composition, it is possible to stably emulsify/disperse an aqueous phase in various oil agents having various characteristics such as polar ester oils (trioctanoin, sunflower oil), mineral oils with relatively high molecular weights, and non-polar isododecane having a low molecular weight in addition to silicone oils, and that an emulsion having the contradictory characteristics of an extremely soft structure and tactile sensation while having high viscosity capable of guaranteeing stability can be obtained.

In particular, since conventional emulsifiers have a limited effect of improving the tactile sensation of oil agents, only hard emulsions with a heavy tactile sensation were obtained with formulations containing large amounts of ester oils such as triglycerides. However, by using the organopolysiloxane elastomer of the present invention, an extremely watery tactile sensation and a smooth, velvet-like sensation during use with a unique thick feeling can be imparted even with formulations containing large amounts of such heavy oil agents, and the emulsification stability can be secured. That is, the ground partiesl of the sugar alcohol-modified organopolysiloxane elastomer of the present invention can be used with much more versatility than the conventional hydrophilic silicone elastomers used in the comparative examples, and it was confirmed that the elastomer dramatically increases the overall value of the external use preparation or cosmetic composition.

Further, the emulsion obtained here has the excellent feature that there is very little odor generation due to the passage of time or temperature, which verifies that the emulsion is extremely useful as a raw material for an external use preparation or a cosmetic composition.

### Practical Example 53

The polyol (non-water)-in oil emulsion composition with the formulation shown in Table 10 was prepared using the paste-like composition obtained in Practical Example 1. In the table, "parts" indicates "parts by weight (mass)".

Table 10

**Table 10: Formulation of non-water-in-oil emulsion composition (Practical Example 53)**

| Name of raw material | Practical Example 53 |
|---|---|
| Paste-like composition of Practical Example 1 | 6.7 |
| Oil agent: dimethylpolysiloxane (2 cst) | 13.3 |
| Sodium chloride | 1.0 |
| Ascorbic acid | 1.0 |
| Propylene glycol | 78.0 |

### [Preparation method for non-water-in-oil emulsion composition]

1. The paste-like composition was placed in the pot of a Hobart mixer.
2. An oil agent was dropped into the mixture while agitating over the course of 5 minutes to obtain a uniform mixture (oil phase A).
3. Table salt, ascorbic acid, and propylene glycol were placed in a separate container, mixed well with a spatula, and dissolved (polyol phase B).
4. The polyol phase B was dropped into the oil phase A over the course of 5 minutes while agitating the oil phase A with the Hobart mixer.
5. Agitation was halted after agitating for two more minutes. The oily component and polyol phase adhering to the inner wall of the container were then scraped off using a spatula and mixed with the produced emulsion.
6. The mixture was further agitated for 3 minutes with the Hobart mixer.

The cream or gel-like emulsion composition obtained here was stable without any noticeable changes in appearance or form even after being stored for 2 weeks at 50°C.

Practical Example 54

Using the paste-like composition obtained in Practical Example 1, a water-in-oil emulsion clear gel-type antiperspirant composition with the formulation shown in Table 11 was prepared in accordance with the following procedure. In the table, "parts" indicates "parts by weight (mass)".

Preparation procedure
1. Components 1 to 3 were mixed and dissolved uniformly (oil agent).
2. Component 4 was placed in the pot of the Hobart mixer.
3. An oil agent was dropped into the mixture while agitating with the Hobart mixer over the course of 5 minutes to obtain a uniform mixture (oil phase A).
4. Specified amounts of the aqueous phase components (excluding the ion exchange water No. 10 shown last) were placed in a separate container and mixed and dissolved using a spatula (aqueous phase B).
5. Two or three drops of each of these two phases were sampled, and refractive indices (RI) of each at 25°C were measured.
6. The up to 1.0 parts of ion exchange water (No. 10) shown last was added in small portions so that the RI value of the aqueous phase was within 0.0001 units and matched the RI of the oil phase. This process was repeated until the desired matching of the RI values was achieved.
7. The aqueous phase B was poured into the oil phase A little by little over the course of approximately 10 minutes while agitating the oil phase A with the Hobart mixer.
8. Agitation was halted after agitating for two more minutes, oil component and aqueous component adhered to the inner wall of the container was scraped off by using a spatula and mixed with the produced W/O transparent emulsion composition.
9. The mixture was further agitated for 3 minutes with the Hobart mixer.

Table 11

**Table 11: Formulation of a water-in-oil transparent gel-like antiperspirant composition (Practical Example 54)**

| No. | Component | Practical Example 54 |
|---|---|---|
| | Portion A: Oil phase | - |
| 1 | Dimethylpolysiloxane (2 cst) | 13.0 |
| 2 | Dimethylpolysiloxane (20 cst) | 3.3 |
| 3 | Isopropyl palmitate | 1.0 |
| 4 | Paste-like composition of Practical Example 1 | 6.7 |
| | Portion B: Aqueous phase | - |
| 5 | Ion exchange water | 14.5 |
| 6 | Propylene glycol | 10.0 |
| 7 | POE (20) sorbitan monooleate | 0.66 |
| 8 | 50% aluminum chlorohydrate aqueous solution | 40.0 |
| 9 | 70% sorbitol aqueous solution | 13.6 |
| 10 | Ion exchange water (for balancing with the RI of Portion A) | 0.05 |
| Total | | ∼103 |
| Measurement | RI of portion A (at 25°C) | 1.4025 |
| | RI of portion B (at 25°C) | 1.4025 |
| | Final RI (at 25°C) | 1.4027 |

The water-in-oil emulsion transparent gel-like antiperspirant composition obtained here was stable with no noticeable changes in appearance or form even after being stored for 2 weeks at 50°C.

[Practical Example 55 and Comparative Examples 21 and 22] In accordance with the following procedure, oil-based foundations containing the paste-like compositions obtained in Practical Example 1 and Comparative Examples 1 and 2 were prepared as prescribed by the formulations in Table 12. In addition, an artificial leather surface was coated with each obtained foundation using a Meyer bar, and the cosmetic retention was evaluated in accordance with the following evaluation criteria for films obtained after the foundations dried. The storage stability of the obtained foundations was also evaluated. The results are shown in Table 12. In the table, "parts" indicates "parts by weight (mass)".

### [Oil-based foundation preparation method]

1. Components other than an inorganic powder were dissolved or uniformly dispersed while agitating at 70 to 80°C.
2. An inorganic powder (silicone-treated kaolin, silicone-treated titanium dioxide, and silicone-treated red iron oxide) was added to this, mixed well, and uniformly dispersed.
3. After being degassed, a container was filled with the mixture, and the mixture was returned to room temperature.

### [Cosmetic retention test method]

1. After an artificial leather surface was coated with an oil-based foundation using a Meyer bar, the foundation was left to stand overnight and dried in a constant-temperature bath at 40°C. Three of these samples were prepared for each foundation.
2. A filter paper soaked with water, a filter paper soaked with squalane, and a filter paper not soaked with anything were prepared.
3. The artificial leather surfaces coated with the foundation in 1 were pressed into the filter papers of 2 and moved forward and backward ten times.
4. The amount of transfer of the sample from the artificial leather to the filter paper after the completion of the forward and backward movements was determined visually based on the darkness of the color.

### [Cosmetic retention evaluation criteria]

⊚: No transfer whatsoever.
○: Very little transfer.
Δ: Some transfer.
×: Severe transfer.

### [Storage stability test method]

1. First, 28 g of each oil-based foundation was measured in a 35 mL glass bottle. The bottles were capped and allowed to sit at rest in a 50°C constant temperature bath for two weeks.
2. The bottles were removed and allowed to return to room temperature, and the presence or absence of changes in appearance relative to the initial appearance was confirmed.

### [Storage stability evaluation criteria]

⊚: No changes in appearance are observed.
○: Practically no abnormalities in appearance are observed.
Δ: Slight powder precipitation or slight separation of the oil phase is observed on the surface.
×: Powder precipitation or phase separation are clearly observed.

Table 12

**Table 12: Formulations and evaluation results of the oil-based foundations (Practical Example 55 and Comparative Examples 21 and 22)**

| Name of raw material | Practical Examples | Comparative Examples | |
|---|---|---|---|
| | 55 | 21 | 22 |
| Paste-like composition of Practical Example 1 | 8 | - | - |
| Paste-like composition of Comparative Example 1 | - | 8 | - |
| Paste-like composition of Comparative Example 2 | - | - | 8 |
| Microcrystalline wax | 4 | 4 | 4 |
| Mineral oil | 3 | 3 | 3 |
| Sorbitan sesquioleate | 1 | 1 | 1 |
| Decamethyl cyclopentasiloxane | 39 | 39 | 39 |
| Isopropyl myristate | 2 | 2 | 2 |
| Silicone-treated kaolin | 25 | 25 | 25 |
| Silicone-treated titanium dioxide | 15 | 15 | 15 |
| Silicone-treated red Iron oxide | 3 | 3 | 3 |
| Cosmetic retention (not applied) | ⊚ | ○ | ⊚ |
| Cosmetic retention (water application) | ⊚ | ○ | ⊚ |
| Cosmetic retention (squalane application) | ⊚ | Δ | ○ |
| Storage stability | ⊚ | ⊚ | Δ |

As described above, it was confirmed that when the sugar alcohol-modified organopolysiloxane elastomer of the present invention is used, the moisture resistance and sebum resistance of a cosmetic composition containing the powder improve in comparison to cases in which the conventional hydrophilic group-containing organopolysiloxane elastomers used in the comparative examples are used, and there is little secondary adhesion. In addition, the oil-based foundations using the organopolysiloxane elastomer of the present invention demonstrated good storage stability. These results indicate that the organopolysiloxane elastomer of the present invention is an excellent powder dispersion stabilizer.

Further, the organopolysiloxane elastomer of the present invention can be used in formulations 1 to 47 of the specific cosmetic compositions shown in the table below. [Table 13]

**Table 13**

| | |
|---|---|
| Formulation example 1: W/O cream foundation | Formulation example 25: Anhydrous roll-on antiperspirant |
| Formulation example 2: W/O Liquid foundation | Formulation example 26: Nonaqueous anti-perspirant deodorant stick composition |
| Formulation example 3: W/O compact foundation | Formulation example 27: W/O solid anti-perspirant stick composition |
| Formulation example 4: O/W/O liquid foundation | Formulation example 28: W/O emulsion type anti-perspirant cream composition |
| Formulation example 5: W/O makeup foundation | Formulation example 29: Water-in-oil emulsion transparent antiperspirant composition |
| Formulation example 6: Rouge | Formulation example 30: Non-aqueous stick-type antiperspirant composition |
| Formulation example 7: Powder foundation | Formulation example 31: Hair conditioner |
| Formulation example 8: W/O compact foundation | Formulation example 32: Shampoo |
| Formulation example 10: Eye shadow | Formulation example 33: Hair cream (set type) |
| Formulation example 10: W/O UV cutting cream | Formulation example 34: Shampoo |
| Formulation example 11: W/O UV cutting | Formulation example 35: Hair conditioner |
| emulsion | |
| Formulation example 12: O/W UV cutting cream | Formulation example 36: Hair treatment rinse type |
| Formulation example 13: W/O/W cream | Formulation example 37: Hair treatment leave-on type |
| Formulation example 14: O/W/O emulsion | Formulation example 38: Hair mist |
| Formulation example 15: Skin care emulsion | Formulation example 39: Hair foam |
| Formulation example 16: Skin care cream | Formulation example 40: Hair spray |
| Formulation example 17: Foundation | Formulation example 41: Hair wax |
| Formulation example 18: O/W sunscreen | Formulation example 42: Hair cream |
| Formulation example 19: Powder eye shadow | Formulation example 43: Hair lotion |
| Formulation example 20: Rouge | Formulation example 44: Hair oil |
| Formulation example 21: Mascara | Formulation example 45: Hair color oxidation type |
| Formulation example 22: O/W cream | Formulation example 46: Hair manicure |
| Formulation example 23: O/W cream | Formulation example 47: Perm |
| Formulation example 24: Stick-type antiperspirant | |

The specific content of these formulation examples has been disclosed in detail and in entirety in the patent application corresponding to this application (Japanese Patent Application involving a priority claim based on Japanese Patent Application No. 2011-121097) and the Japanese patent application serving as a basis for the priority claim (Japanese Patent Application No. 2011-121097) filed in Japan by the present patent applicant on the same day as this application, and the content thereof is incorporated herein by reference.

## Claims

1. An organopolysiloxane elastomer having a silicon-bonded sugar alcohol-modified group and having a crosslinked three-dimensional network structure comprising a carbon-silicon bond in the crosslinking portion.

2. The organopolysiloxane elastomer according to claim 1, wherein the sugar alcohol-modified group is expressed by the following general formula (4-1): 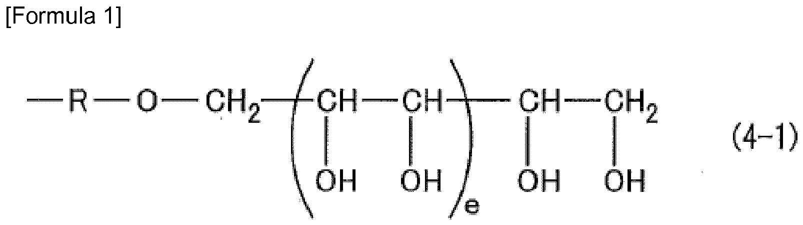 (wherein
R is a divalent organic group, and
e is 1 or 2) or the following general formula (4-2): 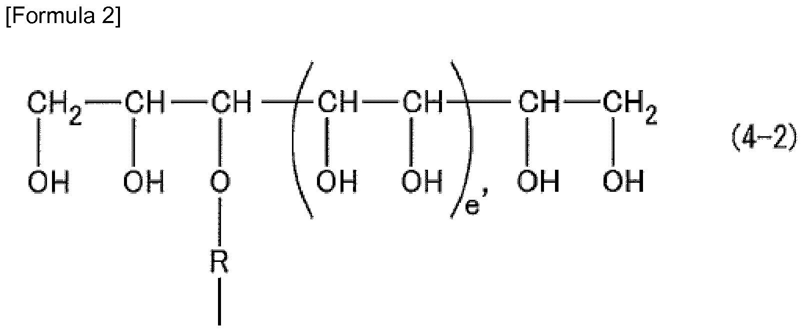 (wherein
R is as defined above, and
e' is 0 or 1).

3. The organopolysiloxane elastomer according to claim 1 or 2, wherein in the general formula (4-1) or (4-2), the divalent organic group represented by R is a substituted or unsubstituted, straight-chain or branched divalent hydrocarbon group having from 3 to 5 carbon atoms.

4. The organopolysiloxane elastomer according to any one of claims 1 to 3 obtained by reacting:
(A) an organohydrogenpolysiloxane;
(B) a sugar alcohol group-containing organic compound having a reactive unsaturated group; and
(C) at least one type of organic compound selected from the group consisting of (C1) an organic compound having an average number of reactive unsaturated groups in the molecule that is greater than 1 and (C2) an organic compound having at least one reactive unsaturated group and at least one epoxy group in the molecule.

5. The organopolysiloxane elastomer according to claim 4, wherein an average value of a number of silicon-bonded hydrogen atoms per molecule of the component (A) reacting with the reactive unsaturated groups of the component (C) constituting the crosslinking portion is at least 1.5.

6. The organopolysiloxane elastomer according to claim 4 or 5, wherein the component (A) is expressed by the average composition formula (1):
[Formula 3]
R¹ₐH_{b}SiO_{(4-a-b)/2} (1)
(wherein R¹ moieties are each independently monovalent organic groups, 1.0≤a≤3.0, and 0.001≤b≤1.5).

7. The organopolysiloxane elastomer according to any one of claims 4 to 6, wherein the component (C) is at least one type of organic compound selected from the following formulas (C1-1) to (C1-5) and (C2-1) to (C2-2):
(C1-1) an α,ω-diene expressed by the general formula (2-1):
[Formula 4]
CH₂=CH(CH₂)ₓCH=CH₂ (2-1)
(wherein 1≤x≤20);
(C1-2) an α,ω-diyne expressed by the general formula (2-2):
[Formula 5]
CH=C(CH₂)ₓC=CH (2-2)
(wherein 1≤x≤20);
(C1-3) an α,ω-ene-yne expressed by the general formula (2-3):
[Formula 6]
CH₂=CH(CH₂)ₓC≡CH (2-3)
(wherein 1≤x≤20);
(C1-4) a bisalkenyl polyether compound expressed by the general formula (2-4):
[Formula 7]
CₘH₂ₘ₋₁O(CₙH₂ₙO)_{y}CₘH₂ₘ₋₁ (2-4)
(wherein 2≤m≤20, 2≤n≤4, y is the total value of the number of repetitions of the oxyethylene unit, the oxypropylene unit, and the oxybutylene unit, and 1≤y≤180);
(C1-5) an unsaturated group-containing silicone-compound expressed by the average composition formula (2-5):
[Formula 8]
R²ₚR³_{q}SiO_{(4-p-q)/2} (2-5)
(wherein R² moieties may each be independent from one another but are monovalent organic groups that are different from R³;
R³ moieties are each independently monovalent unsaturated aliphatic hydrocarbon groups having from 2 to 30 carbon atoms, 1.0≤p≤2.5, and 0.001≤q≤1.5);
(C2-1) an unsaturated epoxy compound expressed by the general formula (2-6): 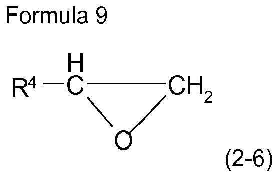 (wherein R⁴ is a substituted or unsubstituted, straight-chain or branched monovalent hydrocarbon group having one reactive unsaturated group and from 2 to 20 carbon atoms); and (C2-2) an unsaturated group-containing cycloaliphatic epoxy compound expressed by the general formula (2-7):
(C2-2) an unsaturated group-containing cycloaliphatic epoxy compound expressed by the general formula (2-7): 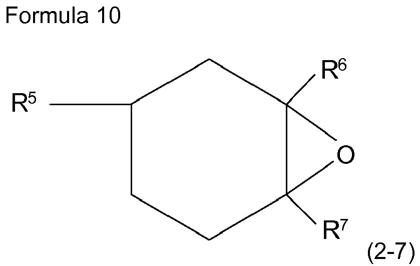 (wherein R⁵ is a substituted or unsubstituted, straight-chain or branched monovalent hydrocarbon group having one reactive unsaturated group and from 2 to 20 carbon atoms;
R⁶ is a hydrogen atom or a methyl group; and
R⁷ is a hydrogen atom or a methyl group)

8. The organopolysiloxane elastomer according to claim 6 or 7, wherein in the average composition formula (1), the monovalent organic group represented by R¹ is selected from the following (D1) to (D10);
(D1) a substituted or unsubstituted, straight-chain or branched monovalent hydrocarbon group having from 1 to 60 carbon atoms;
(D2) a polyoxyalkylene group expressed by -R⁸O(AO)_{z}R⁹ (wherein AO is an oxyalkylene group having from 2 to 4 carbon atoms; R⁸ is a substituted or unsubstituted, straight-chain or branched divalent hydrocarbon group having from 3 to 5 carbon atoms; R⁹ is a hydrogen atom, a substituted or unsubstituted, straight-chain or branched monovalent hydrocarbon group having from 1 to 24 carbon atoms, or a substituted or unsubstituted, straight-chain or branched acyl group having from 2 to 24 carbon atoms; and z=1 to 100);
(D3) a substituted or unsubstituted, straight-chain or branched alkoxy group having from 1 to 30 carbon atoms;
(D4) a hydroxyl group;
(D5) an ester group expressed by -R¹⁰-COOR¹¹ (wherein R¹⁰ is a substituted or unsubstituted, straight-chain or branched divalent hydrocarbon group having from 2 to 20 carbon atoms, and R¹¹ is a substituted or unsubstituted, straight-chain or branched monovalent hydrocarbon group having from 1 to 30 carbon atoms);
(D6) an ester group expressed by -R¹⁷-OCOR¹⁸ (wherein R¹⁷ is a substituted or unsubstituted, straight-chain or branched divalent hydrocarbon group having from 2 to 20 carbon atoms, and R¹⁸ is a substituted or unsubstituted, straight-chain or branched monovalent hydrocarbon group having from 1 to 30 carbon atoms);
(D7) L¹
here, L¹ is a silylalkyl group having a siloxane dendron structure and, when i=1, is expressed by the following general formula (3): 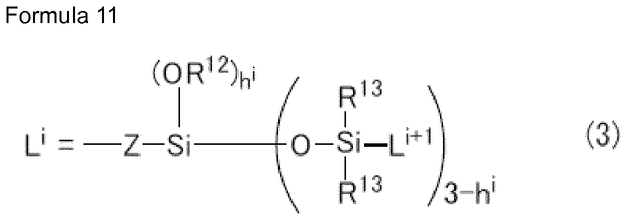 (wherein
R¹² is a substituted or unsubstituted, straight-chain or branched monovalent hydrocarbon group having from 1 to 30 carbon atoms;
R¹³ moieties each independently represents an alkyl group or a phenyl group having from 1 to 6 carbon atoms;
Z is a divalent organic group;
i represents a generation of the silylalkyl group represented by Lⁱ and is an integer of 1 to k when k is the number of generations, which is the number of repetitions of the silylalkyl group; the number of generations k is an integer from 1 to 10; Lⁱ⁺¹ is the silylalkyl group when i is less than k, and R¹³ when i=k; and hⁱ is a number in a range from 0 to 3);
(D8) an alkyl group substituted by a chain polysiloxane structure expressed by the following general formula (4): 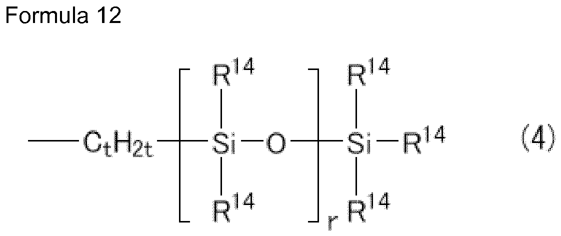 (wherein R¹⁴ moieties are each independently substituted or unsubstituted, straight-chain or branched monovalent hydrocarbon groups having from 1 to 30 carbon atoms, hydroxyl groups, or hydrogen atoms, and at least one of the R¹⁴ moieties is the monovalent hydrocarbon group; t is a number in a range from 2 to 10; and r is a number in a range from 1 to 100);
(D9) an epoxy group expressed by the following general formula (5): 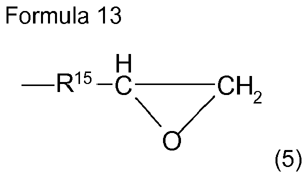 (wherein R¹⁵ is a substituted or unsubstituted, straight-chain or branched divalent hydrocarbon group having from 2 to 20 carbon atoms); and
(D10) a cycloaliphatic epoxy group expressed by the following general formula (6): 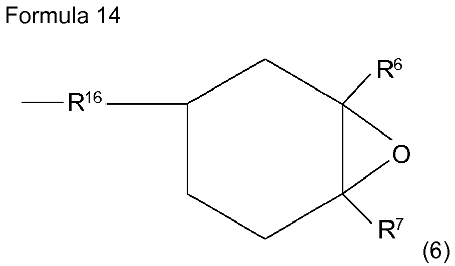 (wherein R¹⁶ is a substituted or unsubstituted, straight-chain or branched divalent hydrocarbon group having from 2 to 20 carbon atoms, and R⁶ and R⁷ are synonymous with those described above).

9. The organopolysiloxane elastomer according to any one of claims 1 to 8 in a particulate form.

10. The organopolysiloxane elastomer according to claim 9 in a particulate form having a volume average particle size in a range from 20 to 1000 µm.

11. The organopolysiloxane elastomer according to any one of claims 1 to 10, wherein the elastomer contains an oil agent in an amount greater than or equal to the weight of the elastomer itself and is swellable.

12. A composition containing the organopolysiloxane elastomer described in any one of claims 1 to 11 and at least one type of oil agent.

13. The composition according to claim 12 in a paste form.

14. The composition according to claim 12 or 13 obtained by mixing at least one type of oil agent after grinding the organopolysiloxane elastomer described in any one of claims 1 to 11 using a mechanical force or grinding a mixture of the organopolysiloxane elastomer and at least one type of oil agent using a mechanical force.

15. An organopolysiloxane elastomer or composition thereof formed by treating the organopolysiloxane elastomer described in any one of claims 1 to 11 or the composition described in any one of claims 12 or 14 with at least one type of acidic substance and removing volatile components by heating or decompression.

16. The composition according to any one of claims 12 to 15, the composition being an emulsion composition.

17. A raw material for an external use preparation or a cosmetic composition containing the organopolysiloxane elastomer described in any one of claims 11 to 15 or the composition described in any one of claims 12 to 16.

18. The raw material for an external use preparation or a cosmetic composition according to claim 17, wherein the raw material is a thickening agent, a gelling agent, a tactile sensation improver, a surfactant, an emulsifier, or a powder dispersion stabilizer.

19. An external use preparation or a cosmetic composition containing the organopolysiloxane elastomer described in any one of claims 11 to 15 or the composition described in any one of claims 12 to 16.

20. A production method for an organopolysiloxane elastomer, comprising reacting:
(A) an organohydrogenpolysiloxane;
(B) a sugar alcohol group-containing organic compound having a reactive unsaturated group; and
(C) at least one type of organic compound selected from the group consisting of (C1) an organic compound having an average number of reactive unsaturated groups in the molecule that is greater than 1 and (C2) an organic compound having at least one reactive unsaturated group and at least one epoxy group in the molecule.

21. The production method for an organopolysiloxane elastomer according to claim 20, wherein part or all of the reaction requiring the component (A), the component (B) and the component (C) as essential components is performed in the presence of at least one solvent selected from a group indicated by (P-1) to (P-2) below:
(P-1): organic compound; and
(P-2): compound containing silicon atoms.

22. The production method for an organopolysiloxane elastomer according to claim 20 or 21, wherein a crosslinking reaction is performed by adding the component (C) after first reacting the component (A) and the component (B); and an optional component (Q) that is a compound having one unsaturated group in the molecule (excluding the compound of (C2)) may be reacted with the component (A) prior to the reaction between the component (A) and the component (B), further reacted after the reaction between the component (A) and the component (B), or further reacted after crosslinking by the component (C).

23. The production method for an organopolysiloxane elastomer according to claim 20 or 21, wherein a reaction between the component (A) and the component (C) is first performed to derive a crosslinked portion, and the component (B) is then added and reacted thereafter; and an optional component (Q) that is a compound having one unsaturated group in the molecule (excluding the compound of (C2)) may be reacted with the component (A) prior to the reaction between the component (A) and the component (C), further reacted after the reaction between the component (A) and the component (C), or further reacted after the reaction with the component (B).

24. A production method for an organopolysiloxane elastomer or a composition thereof, wherein after an organopolysiloxane elastomer obtained by a reaction according to any one of claims 20 to 23 or a composition containing the same is treated with at least one type of an acidic substance, volatile components are removed by heating or decompression.

25. The production method for an organopolysiloxane elastomer or a composition thereof according to claim 24, wherein after the elastomer is treated with the acidic substance, the elastomer is neutralized by adding at least one type of basic substance.
